# EUROPEAN PATENT APPLICATION

(11) **EP 3 885 358 A1**
(43) Date of publication of application: **29.09.2021**
(21) Application number: 19887613.8
(22) Date of filing: 18.11.2019
(51) Int. Cl.: C07K 14/71, C07K 14/47, A61K 38/00, A61P 37/02, A61P 37/00

(54) **COMPOSITION AND METHOD FOR REGULATING MIGRATION OF IMMUNE CELLS**

(30) Priority: 19.11.2018 CN 201811378994
(71) Applicant: Center for Excellence in Molecular Cell Science, Chinese Academy of Sciences, Shanghai 200031 (CN)
(72) Inventor: CHEN, Jianfeng, Shanghai 200031 (CN); LIN, Changdong, Shanghai 200031 (CN)
(74) Representative: Potter Clarkson
(86) International application number: PCT/CN2019/119171
(87) International publication number: WO 2020/103789

(57) **Abstract**

A composition and method for regulating the migration of immune cells. The method for regulating the migration of immune cells comprises the step of strengthening or weakening the interaction between Hsp90 and α4 integrin in immune cells. Also provided is an immune cell capable of strengthening or weakening the interaction between Hsp90 and α4 integrin and a pharmaceutical composition thereof. The present method and pharmaceutical composition may be used to treat pathogen infections and autoimmune diseases or to kill tumor cells.

## Description

### FIELD OF DISCLOSURE

This disclosure relates to composition and method for regulating immune cell migration.

### BACKGROUND OF DISCLOSURE

The migration of immune cells is a key point of body immunity and host defense. It plays an important role in the effective immune surveillance and the homeostasis of the internal environment. It is a research hotspot and frontier subject in the field of life sciences. Immune cells migrate directionally from the blood to lymphatic organs and tissues through high endothelial venules (HEVs), including the homing of immune cells to peripheral lymphoid organs, or the directional migration to inflammation sites and pathological tissues. The homing of immune cells involves the coordination of multiple adhesion molecules on the surface of immune cells and vascular endothelial cells, consisting of a series of highly ordered adhesion events, namely, localization and rolling of immune cells on blood vessel walls, chemokines-dependent cell activation, stable adhesion, and finally transvascular endothelial migration and diapedesis. Among the many molecules that mediate the homing of immune cells, integrin is the most important homing receptor for immune cells, which facilitates precise regulation of immune cell adhesion and migration by interacting with corresponding integrin ligands expressed on the surface of blood vessel endothelium.

As an important cell adhesion molecule (CAM) expressed on the surface of cell membranes, integrin is a heterodimer composed of two subunits of α and β connected by non-covalent bonds. Integrins are widely distributed in organisms. At present, 18 different α subunits and 8 different β subunits have been found in vertebrates, which combine to form at least 24 integrins. Among them, α4 and β2 integrins, as the two main receptors that mediate the homing of immune cells, play a very important role in the adhesion, migration and diapedesis of immune cells on the vascular endothelium to secondary lymphoid organs, inflammation or tumor sites. The adhesion and migration of immune cells mediated by integrins is achieved by dynamic regulation of its ligand affinity. When the cell is not stimulated, the integrins on the cell surface are in a low-active conformation with a low ligand affinity. When the cell is stimulated, the relevant signaling pathways inside the cell are activated, and the regulatory protein inside the cell causes a series of conformational changes of integrins (from a low-activity folded conformation to a high-activity extended conformation) through inside-out signaling, leading to the activation of integrins. The activated integrins can bind to ligands with high affinity and mediate stable cell adhesion. On the other hand, the binding of extracellular ligands and the extracellular domains of integrins can also cause the overall conformational changes of integrins and the clustering of integrins on the cell surface, thereby recruiting intracellular signaling molecules and activating corresponding intracellular signaling pathways (outside-in signaling) to promote cell spreading and migration.

Fever is an evolutionarily highly conserved defense mechanism against injury. When the body is stimulated by pathogens, the temperature of the local inflammation site or the systemic temperature will increase significantly, which can effectively improve the survival of the organism under infection or inflammation. Specifically, fever is a complex physiological response of the body to external infections and stimuli (such as bacterial endotoxin, inflammation, and injury). Fever is generally induced by locally released pyrogenic cytokines, including tumor necrosis factor-a (TNF-α), interleukin-1β (IL-1β) and interleukin-6 (IL-6), etc. In warm-blooded animals, these pyrogenic cytokines can affect the hypothalamus to increase the threshold of temperature regulation, and initiate a series of biochemical, physiological and behavioral responses, which eventually lead to an increase in body temperature. A large number of research reports have confirmed that fever-range thermal stress is closely related to the enhancement of the innate immunity and adaptive immunity under stress conditions. One of the important functions is to promote the migration of immune cells to secondary lymphoid organs or inflammatory sites, thereby promoting immune response and tissue repair. Scholars generally believe that the fever-range hyperthermia changes the dynamics of blood flow by dilating blood vessels to promote the recruitment of immune cells to tissues such as inflammation sites. However, recent studies have found that fever-range thermal stress plays a more active role in directing migration of immune cells to secondary lymphoid organs or inflammation sites. The main research results are: fever-range thermal stress promotes the expression of intercellular adhesion molecules ICAM-1 and chemokine CCL21 of the cells on the surface of capillary hyperendothelial veins, thereby enhancing the integrin LFA-1-dependent diapedesis of immune cells across capillary hyperendothelial veins. During this process, the IL-6/sIL6-Rα signaling pathway plays an important role in the increased expression of ICAM-1 induced by fever. In summary, current understanding of the regulation of immune cell migration by fever is limited to the vascular system, but little is known about the regulation of fever on immune cells themselves and its mechanism.

Interestingly, studies have found that fever-range thermal stress can directly act on immune cells, selectively promoting α4 integrin-mediated adhesion and migration, but does not affect the function of β2 integrin. However, these studies are limited to phenotype, but not related to the molecular mechanism.

### SUMMARY OF DISCLOSURE

The present disclosure provides a method of regulating the migration of immune cells, comprising the step of enhancing or weakening the interaction between Hsp90 and α4 integrin in immune cells.

In one or more embodiments, the method includes enhancing the interaction between Hsp90 and α4 integrin in immune cells by any one or more of the following means: (1) overexpressing Hsp90 protein and/or Hsp90 mutein in the immune cells, wherein, compared with the wild-type Hsp90 protein, the Hsp90 mutein is only mutated in its middle domain, or the Hsp90 mutein has a mutation causing its inability to self-dimerize; (2) overexpressing α4 integrin in the immune cells.

In one or more embodiments, the method includes weakening the interaction between Hsp90 and α4 integrin in immune cells by any one or more of the following means: (1) knocking out Hsp90 protein or knocking down its expression in immune cells; (2) knocking out α4 integrin or knocking down its expression in immune cells; (3) expressing Hsp90 mutein that has weakened or no interaction with α4 integrin in wild-type immune cells or immune cells with Hsp90 protein knocked out; (4) expressing α4 integrin mutein that has weakened or no interaction with Hsp90 protein in wild-type immune cells or immune cells with α4 integrin knocked out.

In one or more embodiments, treating immune cells with an expression vector and/or an integration vector of Hsp90 protein and/or Hsp90 mutein, and/or treating immune cells with an expression vector of α4 integrin and/or an integration vector, and/or treating immune cells with reagents that increase the transcription levels of Hsp90 and/or α4 integrin naturally occurred in immune cells, and/or placing immune cells at a fever-range hyperthermia, thereby enhancing the interaction between Hsp90 and α4 integrin in immune cells.

In one or more embodiments, the interaction between Hsp90 and α4 integrin in immune cells is weakened or destroyed through: knocking out Hsp90 protein or α4 integrin from immune cells by transferring a gene-knockout vector into the immune cells, and/or knocking out Hsp90 protein or α4 integrin from immune cells by ZFN, TALEN or CRISPR/Cas9 and the like, and/or knocking down the expression of Hsp90 protein and/or α4 integrin by interfering-RNA-mediated gene silencing, and/or integrating into the genome of immune cells an expression cassette expressing the Hsp90 mutein that has weakened or no interaction with α4 integrin and/or an expression cassette expressing the α4 integrin mutein that has weakened or no interaction with Hsp90 protein by transferring a gene-insertion vector into immune cells while knocking out the coding sequence of wild-type Hsp90 and/or α4 integrin.

In one or more embodiments, the Hsp90 mutein that has weakened or no interaction with α4 integrin lacks its N-terminal and/or C-terminal domain compared with the wild-type Hsp90 protein.

The present disclosure also provides a genetically engineered immune cell, wherein, compared with the wild-type immune cells, the genetically engineered immune cell has an enhanced or weakened interaction between Hsp90 and α4 integrin.

In one or more embodiments, the genetically engineered immune cell: (1) overexpresses Hsp90 protein and/or Hsp90 mutein, wherein, compared with wild-type Hsp90 protein, the Hsp90 mutein is only mutated in its middle domain, or the Hsp90 mutein has a mutation causing its inability to self-dimerize; (2) overexpresses α4 integrin.

In one or more embodiments, the genetically engineered immune cell: (1) does not express Hsp90 or has reduced expression level of Hsp90, or expresses Hsp90 having reduced activity, or expresses Hsp90 mutants, as compared with wild-type immune cells; and/or (2) has reduced expression level of α4 integrin, or expresses α4 integrin mutants, as compared with wild-type immune cells, thereby weakening or eliminating the interaction between Hsp90 and α4 integrin in the genetically engineered immune cell. In one or more embodiments, the Hsp90 mutant lacks its N-terminal and/or C-terminal domain compared to the wild-type Hsp90 protein. In one or more embodiments, compared with the wild-type α4 integrin, the α4 integrin mutant has one or more amino acid residues mutated in its intracellular segment other than amino acid residues 968-974, causing its interaction with Hsp90 protein weakened or eliminated; preferably, there is a mutation at at least one of R985, W989 and Y991; preferably, the mutation is a substitution mutation; more preferably, the substituted amino acid residue is alanine.

The present disclosure also provides a pharmaceutical composition containing the genetically engineered immune cell described herein.

The present disclosure also provides a mutant Hsp90 protein selected from the group consisting of:
(1) a mutant Hsp90 protein, lacking the N-terminal domain and/or the C-terminal domain of the wild-type Hsp90 protein, or having a mutation in C-terminal domain causing its inability to mediate the self-dimerization of Hsp90 protein, as compared with the wild-type Hsp90 protein; and
(2) a mutant Hsp90 protein, only having a mutation in the middle domain as compared with the wild-type Hsp90 protein while maintaining the ability to bind to α4 integrin as compared with the wild-type; and
(3) a mutant Hsp90 protein, having a mutation causing its inability to self-dimerize as compared with the wild-type Hsp90 protein; preferably, the mutation occurs in the C-terminal domain of the Hsp90 protein; more preferably, the mutation is the deletion of the last 49 amino acids of C-terminus.

The present disclosure also provides a mutant α4 integrin, the mutant α4 integrin has one or more amino acid residues mutated in its intracellular segment other than amino acid residues 968-974, causing its interaction with Hsp90 protein weakened or eliminated; preferably, there is a mutation at at least one of R985, W989 and Y991; preferably, the mutation is a substitution mutation; more preferably, the substituted amino acid residue is alanine.

The present disclosure also provides a coding sequence of the mutant Hsp90 protein and the mutant α4 integrin described herein, or complementary sequence thereof, a nucleic acid construct containing the coding sequence or complementary sequence, and a host cell containing the nucleic acid construct.

The present disclosure also provides uses selected from the group consisting of:
(1) use of a reagent that enhances the interaction between Hsp90 and α4 integrin in immune cells in the manufacture of immune cells for immunotherapy; preferably, the reagent is selected from the group consisting of: an expression vector or an integration vector for the wild-type Hsp90 protein, or the mutant Hsp90 protein being only mutated in its middle domain, or the mutant Hsp90 protein having a mutation causing its inability to self-dimerize; and an expression vector and/or integration vector for α4 integrin;
(2) use of immune cells with enhanced interaction between Hsp90 protein and α4 integrin in the manufacture of a medicament for the treatment of pathogen infections or tumors;
(3) use of a reagent that weakens the interaction between Hsp90 and α4 integrin in immune cells in the manufacture of immune cells for the treatment of sepsis, hematological tumors, chronic inflammations or autoimmune diseases; preferably, the reagent is selected from the group consisting of: a Hsp90 mutant lacking the N-terminal and/or C-terminal domain or having a mutation in the N-terminal and/or C-terminal domain causing its interaction with α4 integrin weakened or eliminated as compared with the wide-type Hsp90, and/or a targeting vector of the Hsp90 mutant; a α4 integrin mutant having a mutation in its intracellular segment which causes its interaction with Hsp90 weakened or eliminated, and/or a targeting vector of the α4 integrin mutant; and ZFN, TALEN and/or CRISPR/Cas9 reagents and/or small interfering RNA used to knock out Hsp90 and/or α4 integrin or to knock down its/their expression; and
(4) use of immune cells with weakened interaction between Hsp90 and α4 integrin in the manufacture of a medicament for the treatment of sepsis, hematological tumors, chronic inflammations or autoimmune diseases.

### BRIEF DESCRIPTION OF DRAWINGS

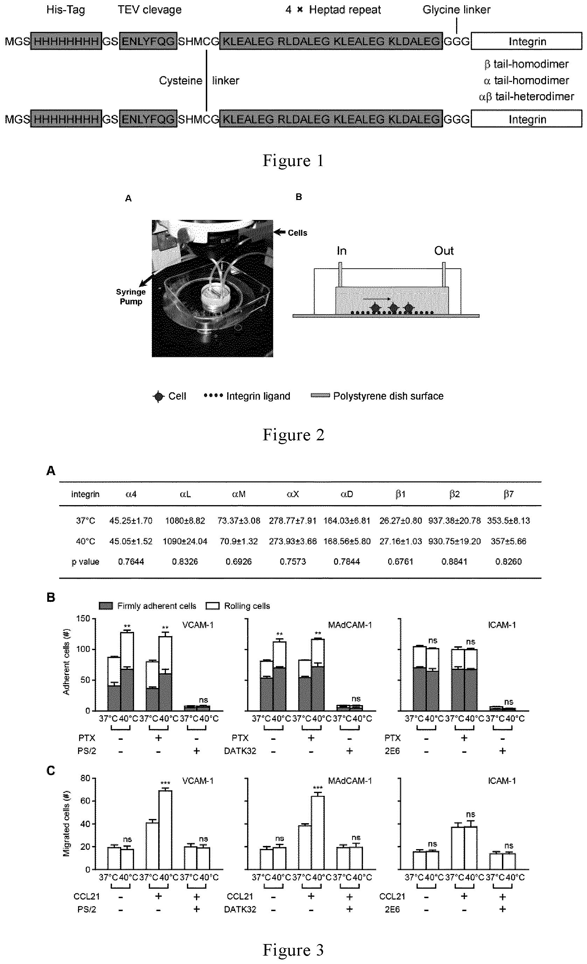

Figure 1: Integrin cytoplasmic domain model proteins.
Figure 2: Flow chamber system.
Figure 3: Fever-range thermal stress promotes α4 integrin-mediated cell adhesion and transmigration. T cells from C57BL/6J mouse spleen were pre-treated at 37 °C or 40 °C in culture medium with or without 100 ng/ml PTX for 12 hr. α4β7-VCAM-1 binding was disrupted by pre-treating the cells with 10 µg/ml α4β7 blocking antibody DATK32 when examining α4β1-mediated cell adhesion and migration on VCAM-1 substrate. (A) Cell surface expression of α4 and β2 integrins was determined by flow cytometry. Numbers within the table showed the specific mean fluorescence intensities and p values. (B) Adhesion of T cells to the immobilized VCAM-1-Fc (5 µg/ml), MAdCAM-1-Fc (5 µg/ml) or ICAM-1-Fc (5 µg/ml) substrate at a wall shear stress of 1 dyn/cm². Cells pre-treated with α4 blocking antibody PS/2 (10 µg/ml), α4β7 blocking antibody DATK32 (10 µg/ml) or β2 blocking antibody 2E6 (10 µg/ml) were used as controls. (C) Transmigration of T cells across VCAM-1-Fc (5 µg/ml), MAdCAM-1-Fc (5 µg/ml) or ICAM-1-Fc (5 µg/ml) coated membrane in the absence and presence of CCL21 (500 ng/ml) in the lower chamber. Data represent the mean ± SEM (n ≥ 3). ** p < 0.01, *** p < 0.001, ns: not significant (Student's t test); asterisk in (B) indicates the changes of total adherent cells.

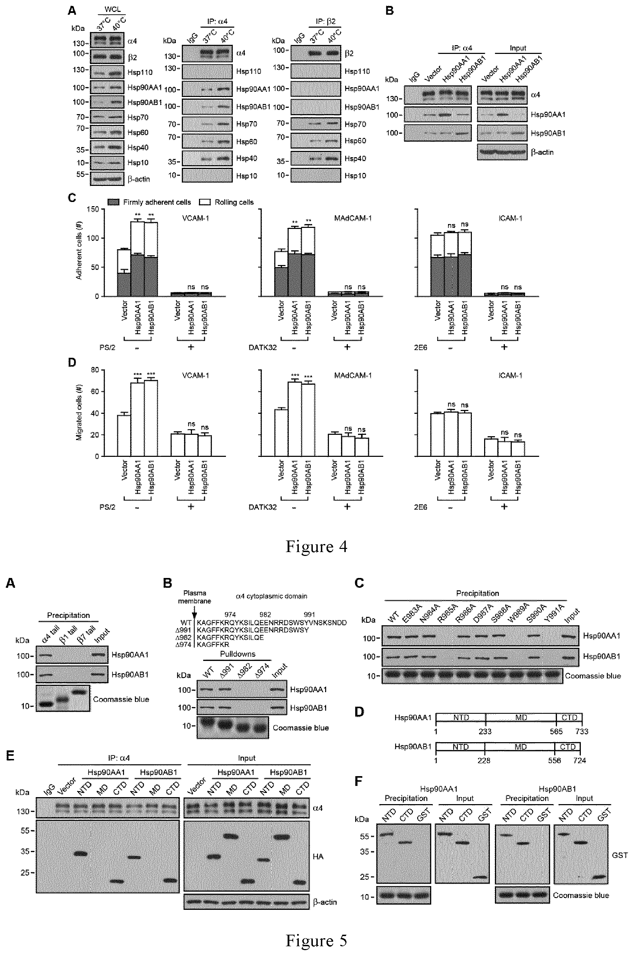

Figure 4: Hsp90 specifically binds to α4 integrin and promotes cell adhesion and transmigration. (A) Immunoblot analysis of integrin α4, β2 and Hsps in whole cell lysate (WCL) of T cells pre-treated at 37 °C or 40 °C and co-immunoprecipitation of Hsps with integrin α4 or β2 in the cell membrane fractions. (B-D) T cells were transiently transfected with vector, Hsp90AA1 or Hsp90AB1, respectively. α4β7-VCAM-1 binding was disrupted by pre-treating the cells with 10 µg/ml α4β7 blocking antibody DATK32 when examining α4β1-mediated cell adhesion and migration on VCAM-1 substrate in (C and D). Co-immunoprecipitation of Hsp90AA1 or Hsp90AB1 with integrin α4 in the membrane fractions of T cells (B). Adhesion of T cells to immobilized VCAM-1-Fc (5 µg/ml), MAdCAM-1-Fc (5 µg/ml) or ICAM-1-Fc (5 µg/ml) substrate at a wall shear stress of 1 dyn/cm² (C). Transmigration of T cells across VCAM-1-Fc (5 µg/ml), MAdCAM-1-Fc (5 µg/ml) or ICAM-1-Fc (5 µg/ml) coated membrane in the presence of CCL21 (500 ng/ml) in the lower chamber (D). Data represent the mean ± SEM (n ≥ 3) in (C and D). ** p < 0.01, *** p < 0.001, ns: not significant (one-way ANOVA with Dunnett post-tests); asterisk in (C) indicates the changes of total adherent cells.
Figure 5: Both the N-terminus and C-terminus of Hsp90 can bind to the cytoplasmic domain of α4 integrin. (A-C) Precipitation of Hsp90AA1 and Hsp90AB1 from T cell lysate by Ni²⁺-charged resins loaded with indicated integrin tail model proteins. Coomassie blue staining of gels assessed the loading of each integrin tail model protein. WT α4, β1, β7 tails were used in (A); α4 tail truncations were tested in (B) and schematic diagram showed WT and truncated α4 tail constructs; single-point mutants of α4 tail were tested in (C). (D) Schematic diagram of Hsp90 structures. N-terminal domain (NTD), middle domain (MD) and C-terminal domain (CTD). (E) HA-tagged NTD, MD or CTD of Hsp90AA1 or Hsp90AB1 was overexpressed in T cells and then coimmunoprecipitated with integrin α4 in the cell membrane fractions. (F) Precipitation of recombinant GST-tagged NTD and CTD proteins of Hsp90AA1 or Hsp90AB1 by Ni²⁺-charged resins loaded with α4 tail model protein.

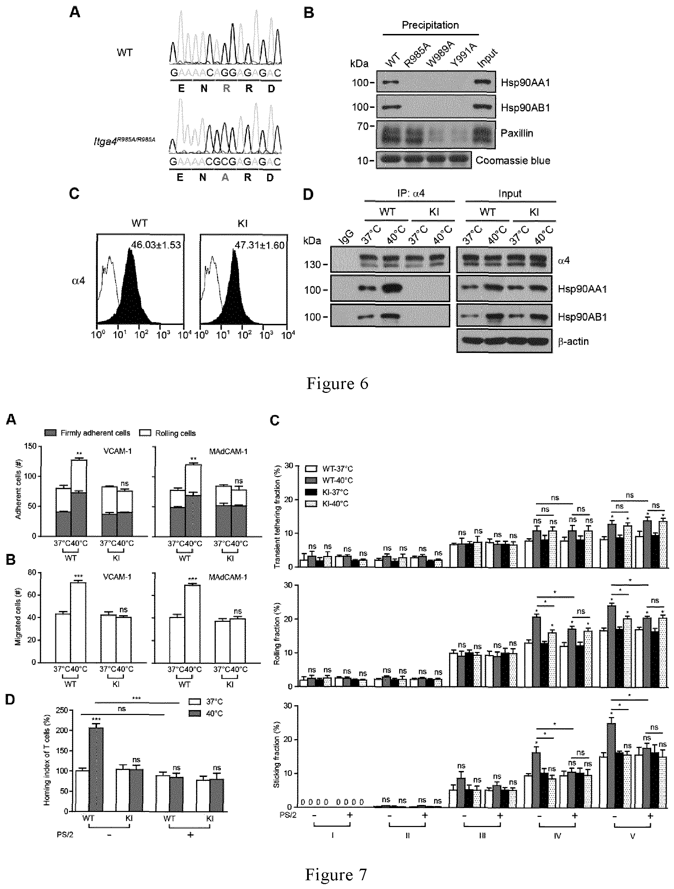

Figure 6: Construction of integrin *Itga4*^{R985A/R985A} knock-in (KI) mice. (A) *Itga4*^{R985A/R985A} (KI) C57BL/6J mice was generated by using the CRISPR/Cas9 system. Sequencing analysis of WT and KI mice. DNA sequencing confirmed an Arg985 to Ala substitution in mouse α4 integrin gene in KI mice. (B) Precipitation of Hsp90AA1, Hsp90AB1 and paxillin from T cell lysate by Ni²⁺-charged resins loaded with WT or mutants of α4 tail model proteins. Coomassie blue staining of gels assessed the loading of each integrin tail model protein. (C) Integrin α4 expression on T cells was determined by flow cytometry. T cells were isolated form mouse spleen. Numbers within the panel showed the specific mean fluorescence intensities. Opened histogram: mock control. (D) WT T cells or *Itga4*^{R985A/R985A} (KI) T cells were pre-treated at 37 °C or 40 °C in culture medium for 12 hr. Hsp90AA1 and Hsp90AB1 were coimmunoprecipitated with integrin α4 in the cell membrane fractions. Data represent the mean ± SEM (n ≥ 3).
Figure 7: Disruption of Hsp90-α4 integrin binding inhibits fever-induced T cell adhesion and transmigration. WT T cells or *Itga4*^{R985A/R985A} (KI) T cells were pre-treated at 37 °C or 40 °C in culture medium for 12 hr. (A) Adhesion of T cells to immobilized VCAM-1-Fc (5 µg/ml) or MAdCAM-1-Fc (5 µg/ml) substrate at wall shear stress of 1 dyn/cm². (B) Transmigration of T cells across VCAM-1-Fc (5 µg/ml) or MAdCAM-1-Fc (5 µg/ml) coated membrane in the presence of CCL21 (500 ng/ml) in the lower chamber. (C) Intravital microscopy of the interactions of calcein-labelled WT or KI T cells with the inguinal lymph node venular tree of WT recipient mice. Transient tethering, rolling and sticking fractions of WT and KI T cells were shown. Cells pre-treated with α4 blocking antibody PS/2 (10 µg/ml) were used as controls (D) *In vivo* short-term homing of calcein-labelled WT or KI T cells to inguinal lymph nodes of WT mice. Cells pre-treated with α4 blocking antibody PS/2 (10 µg/ml) were used as controls. The homing index was calculated as the percentage of the homed T cells in inguinal lymph nodes relative to WT T cells pre-treated at 37 °C without PS/2 antibody treatment. Data represent the mean ± SEM (n ≥ 3). * p < 0.05, ** p < 0.01, *** p < 0.001, ns: not significant (Student's t test); asterisk in (A) indicates the changes of total adherent cells.

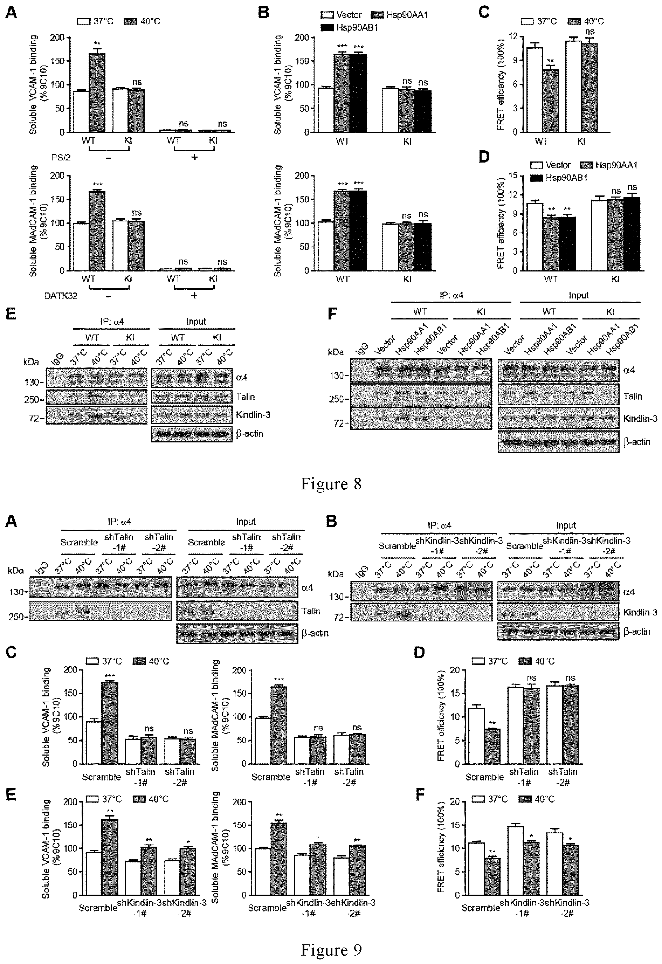

Figure 8: Hsp90-α4 integrin binding induces activation of α4 integrin. (A-B) Binding of soluble VCAM-1-Fc and MAdCAM-1-Fc to WT and *Itga4*^{R985A/R985A} (KI) T cells pre-treated at 37 °C or 40 °C (A) or to T cells transfected with vector, Hsp90AA1 or Hsp90AB1 (B) was calculated by the specific mean fluorescence intensity and quantified as a percentage of α4 expression. α4β7-VCAM-1 binding was disrupted by pre-treating the cells with 10 µg/ml α4β7 blocking antibody DATK32 when examining α4β1-mediated soluble VCAM-1 binding. Cells pre-treated with the α4 blocking antibody PS/2 (10 µg/ml) or α4β7 blocking antibody DATK32 (10 µg/ml) were used as a negative control for VCAM-1 and MAdCAM-1 binding, respectively, in (A). (C-D) Effect of fever-range thermal stress (C) or Hsp90 overexpression (D) on the conformation of α4 ectodomain in WT or KI T cells. FRET efficiency between integrin α4 β-propeller domain and the plasma membrane was calculated. (E-F) Co-immunoprecipitation of talin or kindlin-3 with α4 integrins in T cells pre-treated at 37 °C or 40 °C (E) or T cells transfected with vector, Hsp90AA1 or Hsp90AB1 (F). Data represent the mean ± SEM (n ≥ 3). * p < 0.05, ** p < 0.01, *** p < 0.001, ns: not significant (Student's t test or one-way ANOVA with Dunnett post-tests).
Figure 9: Knockdown of Talin and Kindlin-3 inhibits the activation of α4 integrin. T cells with talin or kindlin-3 silencing were pre-treated at 37 °C or 40 °C in culture medium for 12 hr. (A-B) Co-immunoprecipitation of talin (A) or kindlin-3 (B) with α4 integrins in T cells. (C-D) Binding of soluble VCAM-1-Fc and MAdCAM-1-Fc to T cells with talin (C) or kindlin-3 (D) silencing was calculated by the specific mean fluorescence intensity and quantified as a percentage of α4 expression. α4β7-VCAM-1 binding was disrupted by pre-treating the cells with 10 µg/ml α4β7 blocking antibody DATK32 when examining α4β1-mediated soluble VCAM-1 binding. (E-F) The conformation of α4 ectodomain in T cells with talin (E) or kindlin-3 (F) silencing. FRET efficiency between integrin α4 β-propeller domain and the plasma membrane was calculated. Data represent the mean ± SEM (n ≥ 3). * p < 0.05, ** p < 0.01, *** p < 0.001, ns: not significant (Student's t test).

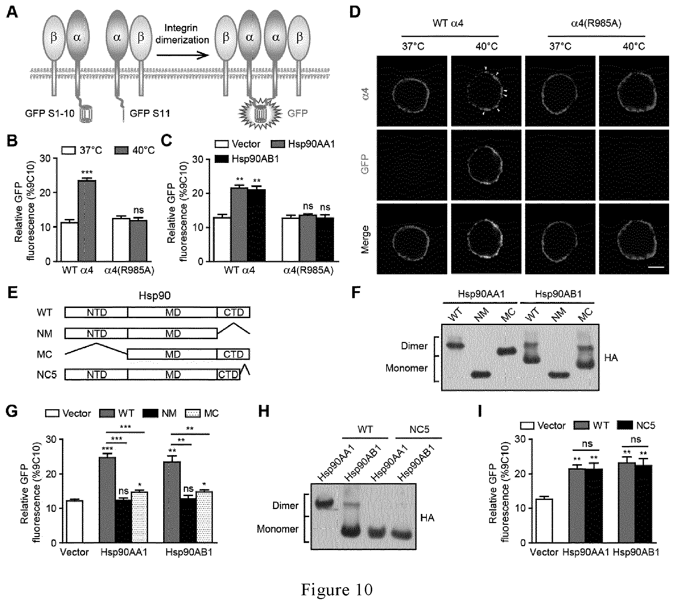

Figure 10: Hsp90-α4 integrin binding induces the dimerization and clustering of α4 integrin on the plasma membrane. (A) Design of a reporter of integrin α4 dimerization on the plasma membrane using BiFC-Spit GFP system. (B-C) Relative GFP fluorescence of T cells expressing WT α4 integrin-Split GFP or α4(R985A) integrin-Split GFP pre-treated at 37 °C or 40 °C (B) or transfected with vector, Hsp90AA1 or Hsp90AB1 (C) was calculated by the mean fluorescence intensity of GFP and quantified as a percentage of α4 integrin expression. (D) Confocal microscopy visualization of the integrin clustering on the plasma membrane. White arrowheads indicate the representative integrin clusters. Scale bar, 3 µm. (E) Schematic diagram of WT and mutant Hsp90. NM: CTD truncation; MC: NTD truncation; NC5: deletion of C-terminal 49 amino acids in CTD to disrupt Hsp90 homodimerization. (F) Cell lysates of T cells transiently expressing HA-tagged Hsp90 WT, NM or MC mutant were loaded onto a native-PAGE, and then HA-tagged proteins were detected by immunoblot. (G) Relative GFP fluorescence of T cells expressing WT α4 integrin-Split GFP and transfected with vector, Hsp90 WT, NM or MC mutant was calculated by the mean fluorescence intensity of GFP and quantified as a percentage of α4 expression. (H) Cell lysates of T cells transiently expressing HA-tagged Hsp90 WT or NC5 mutant were loaded onto a native-PAGE, and then HA-tagged proteins were detected by immunoblot. (I) Relative GFP fluorescence of T cells expressing WT α4 integrin-Split GFP and transfected with vector, Hsp90 WT or NC5 mutant was calculated by the mean fluorescence intensity of GFP and quantified as a percentage of α4 integrin expression. Data represent the mean ± SEM (n = 3). * p < 0.05, ** p < 0.01, *** p < 0.001, ns: not significant (Student's t test or one-way ANOVA with Dunnett post-tests).

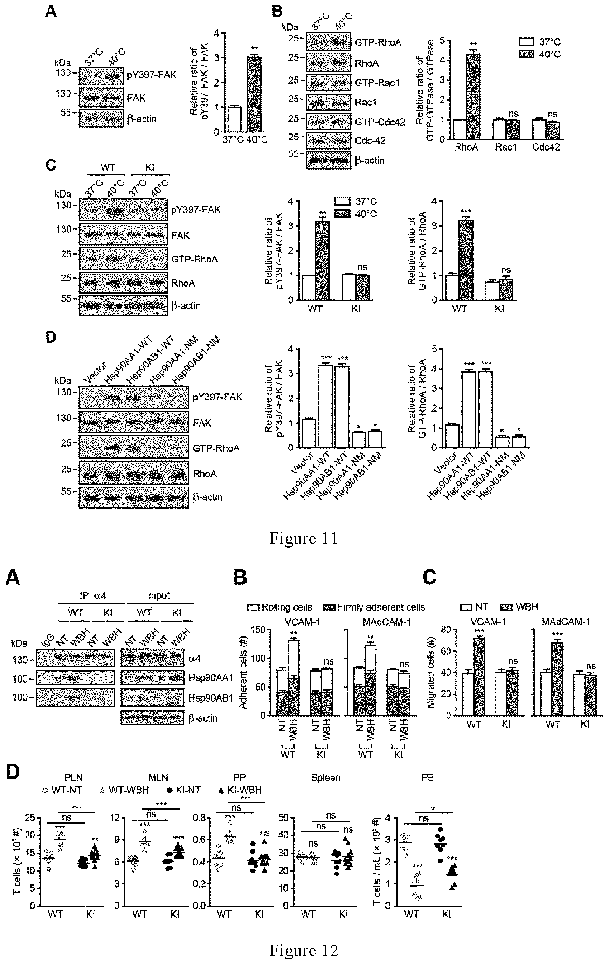

Figure 11: The binding of Hsp90-α4 integrin activates FAK-RhoA signaling pathway. (A) Immunoblot analysis of FAK phosphorylation (pY397) in T cells pre-treated at 37 °C or 40 °C. The relative ratio of pY397-FAK/FAK was normalized to the value of cells pre-treated at 37 °C. (B) Effect of fever-range thermal stress on Rho GTPases activation. GTP-bound RhoA, Rac1 and Cdc42 were detected by binding to recombinant GST-RBD or GST-PBD in T cells pre-treated at 37 °C or 40 °C by GST precipitation assays. The relative ratio of GTP-GTPase/GTPase was normalized to the value of cells pre-treated at 37 °C. (C) Immunoblot analysis of FAK phosphorylation (pY397) and RhoA activation in WT or *Itga4*^{R985A/R915A} (KI) T cells pre-treated at 37 °C or 40 °C. The relative ratios of pY397-FAK/FAK and GTP-RhoA/RhoA were normalized to the values of WT T cells pre-treated at 37 °C. (D) Immunoblot analysis of FAK phosphorylation (pY397) and RhoA activation in T cells transfected with vector, Hsp90 WT or NM mutants. The relative ratios of pY397-FAK/FAK and GTP-RhoA/RhoA were normalized to the values of cells transfected with vector. Data represent the mean ± SEM (n = 3). * p < 0.05, ** p < 0.01, *** p < 0.001, ns: not significant (Student's t test or one-way ANOVA with Dunnett post-tests).
Figure 12: Disruption of Hsp90-α4 interaction inhibits fever-induced T cell trafficking *in vivo.* WT and *Itga4*^{R915A/R915A} (KI) C57BL/6J mice were treated with normothermia (NT, core temperature 36.8 ± 0.2 °C) or fever-range whole-body hyperthermia (WBH, core temperature 39.5 ± 0.5 °C) for 6 hr (n = 7-10 mice per group), and then were sacrificed. T cells were isolated from spleen. α4β7-VCAM-1 binding was disrupted by pre-treating the cells with 10 µg/ml α4β7 blocking antibody DATK32 when examining α4β1-mediated cell adhesion and migration on VCAM-1 substrate in (B and C). (A) Co-immunoprecipitation of Hsp90AA1 and Hsp90AB1 with integrin α4 in the cell membrane fractions. (B) Adhesion of T cells to immobilized VCAM-1-Fc (5 µg/ml) or MAdCAM-1-Fc (5 µg/ml) substrate at a wall shear stress of 1 dyn/cm². (C) Transmigration of T cells across VCAM-1-Fc (5 µg/ml) or MAdCAM-1-Fc (5 µg/ml) coated membrane in the presence of CCL21 (500 ng/ml) in the lower chamber (D) The total numbers of T cells in PLNs, MLNs, PPs, spleen and PB were quantified. Data represent the mean ± SEM (n ≥ 3). * p < 0.05, ** p < 0.01, *** p < 0.001, ns: not significant (Student's t test); asterisk in (B) indicates the changes of total adherent cells.

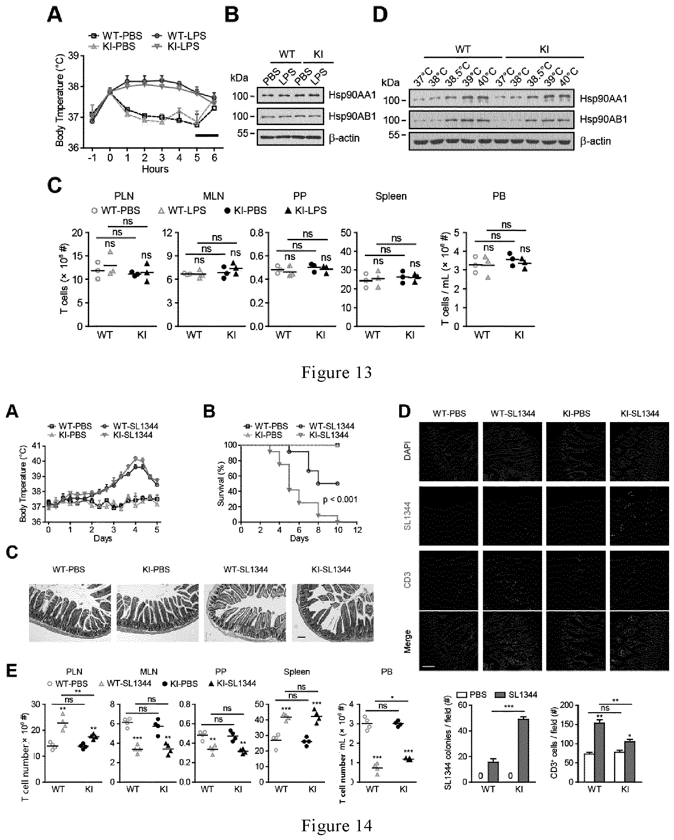

Figure 13: LPS-induced mild fever does not affect the homing of T cells in mice. WT and KI mice were injected with LPS (10 µg/kg) or PBS at time zero (n = 3 mice per group). (A) Body temperature was monitored for every hour. Bar showed dark period. (B) Immunoblot analysis of Hsp90AA1 and Hsp90AB1 in T cells isolated from PLNs in mice. (C) The total numbers of T cells in PLNs, MLNs, PPs, spleen and PB were quantified. (D) Effect of different temperatures on the expression of Hsp90. T cells were isolated from PLNs in WT mice and then treated at 37 °C, 38 °C, 38.5 °C, 39 °C or 40 °C for 12 hr. Immunoblot analysis of Hsp90AA1 and Hsp90AB1 in T cells. Data represent the mean ± SEM (n ≥ 3). ns: not significant (Student's t test).
Figure 14: Disruption of Hsp90-α4 interaction impairs the clearance of bacterial infection. WT and KI mice were orally injected with PBS or *S. typhimurium* strain SL1344 (10⁸ CFU per mouse, n = 4-12 mice per group). (A) Body temperature was monitored for 5 days. (B) The survival rates of WT and KI mice. (C) H&E staining of the small intestine at day 5 post infection. Scale bar, 100 µm. (D) Immunofluorescence analysis of the small intestine sections at day 5 post infection. DAPI (blue), S. typhimurium expressing GFP (green) and CD3 (red). Quantifications of S. typhimurium colonies and CD3⁺ cells were shown below. Scale bar, 100 µm. (E) The total numbers of T cells in PLNs, MLNs, PPs, spleen and PB were quantified at day 5 post infection. Data represent the mean ± SEM (n ≥ 3). * p < 0.05, ** p < 0.01, *** p < 0.001, ns: not significant (Student's t test).

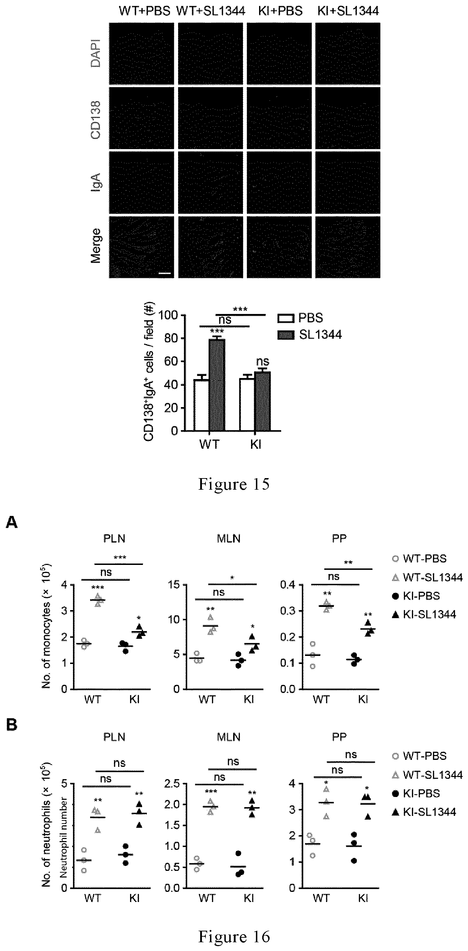

Figure 15: Disruption of Hsp90-α4 interaction inhibits the migration of B cells to the site of bacterial infection. WT and KI mice were orally injected with PBS or *S. typhimurium* strain SL1344 (10⁸ CFU per mouse, n = 4-12 mice per group). Immunofluorescence analysis of the small intestine sections at day 5 post infection. DAPI (blue), CD138 (green) and IgA (red). Quantification of CD138⁺IgA⁺ cells was shown below. Scale bar, 100 µm. Data represent the mean ± SEM (n ≥ 3). *** p < 0.001, ns: not significant (Student's t test).
Figure 16: Disruption of Hsp90-α4 interaction inhibits monocyte recruitment to draining lymph nodes in mice during *S. typhimurium* infection. WT and *Itga4*^{R985A/R985A} (KI) mice were orally injected with PBS or *S. typhimurium* strain SL1344 (10⁸ CFU per mouse, n = 3 mice per group). The total numbers of CD11b⁺Ly6C^{high}Ly6G^{low} monocytes (A) and CD11b⁺Ly6G^{high}Ly6C^{low} neutrophils (B) in PLNs, MLNs and PPs were quantified at day 3 post infection. Data represent the mean ± SEM (n = 3). * p < 0.05, ** p < 0.01, *** p < 0.001, ns: not significant (Student's t test).

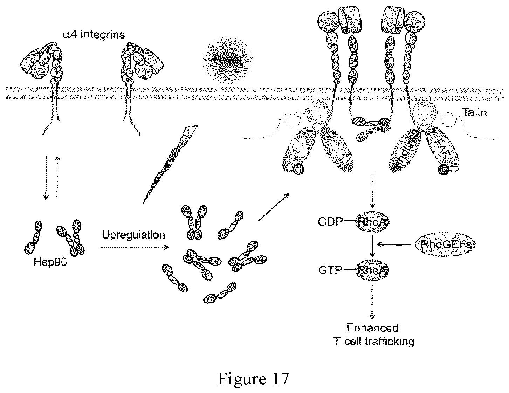

Figure 17: Schematic diagram of fever promoting T cell trafficking via a thermal sensory Hsp90-α4 integrin pathway.

### DETAILED DESCRIPTION

It should be understood that, within the scope of the present disclosure, the above technical features of the present disclosure and the technical features specifically described in the following (e.g., Examples) can be combined with each other, thereby forming preferred technical solution(s).

Fever is an evolutionarily conserved defense process for organisms to respond to infection or injury, and can effectively improve survival. However, the detailed mechanism of fever is not very clear. Through a large number of biochemical, molecular and cellular experiments, the inventors confirmed that: fever-range thermal stress can promote the directional migration of immune cells through the activation and signaling of α4 integrin mediated by Hsp90 protein. Hsp90 protein selectively binds α4 integrin, but not β2 integrin, thereby specifically enhancing α4 integrin-mediated T cell adhesion and transmigration. The specific molecular mechanism is: N-terminus and C-terminus of one Hsp90 molecule can simultaneously bind to the intracellular segments of two α4 subunits, leading to the dimerization and clustering of α4 integrin on the cell membrane and the activation of subsequent intracellular FAK-RhoA signaling pathway, and ultimately promoting the directional migration of immune cells (Figure 16). Considering the key role of α4 integrin in intestinal immune homeostasis, the thermosensitive Hsp90-α4 integrin signal axis may have important functions in inflammation, intestinal immune diseases and other α4 integrin-related diseases, such as multiple sclerosis and inflammatory bowel disease. Thermal stress or directly overexpressing Hsp90 protein in immune cells can promote the directional migration of immune cells, enhance immune response, eliminate pathogen infection or kill tumor cells. Inhibition of Hsp90 expression or destroy of Hsp90-α4 integrin interaction can reduce the immune response in chronic inflammation or autoimmune diseases, or inhibit the migration of immune cells to secondary lymphoid organs or local tissues in diseases such as sepsis or hematological tumors, thereby increasing the concentration of immune cells in the blood circulation, and promoting the elimination of blood infections or blood tumors by immune cells.

Therefore, the disclosure provides a method for regulating the migration of immune cells by regulating the interaction between Hsp90 and α4 integrin. As used herein, "regulating" or "regulation" includes up-regulation and down-regulation, wherein up-regulation includes promotion, increase and/or enhancement, and down-regulation includes inhibition, reduction, weakening, destruction and/or diminishing. As used herein, "immune cells" include cells known in the art that are involved in or associated with immune responses in the animal body, especially the human body, including but not limited to: lymphocytes, dendritic cells, monocytes, macrophages, granulocytes and mast cells, etc. As used herein, "migrating" or "migration" refers to the movement of immune cells to their destinations such as lesions (inflammation sites or tumors), including but not limited to lymphocyte homing (lymphocytes directionally migrate from the blood to the lymphatic organs through the capillary high endothelial veins), and the diapedesis of lymphocytes to the inflammation sites. "Directional" or "directionally" refers to the specific migration of immune cells to secondary lymphoid organs, inflammatory sites or tumor tissues to enhance immune surveillance, maintain immune homeostasis, or promote immune response. The term "individual", "subject" or "patient" herein refers to mammals, especially humans.

Herein, Hsp protein refers to heat shock protein, which is a heat stress protein that exists widely in mammals. When the organism is exposed to high temperature, it will be stimulated by heat to synthesize this protein to protect the organism itself. According to the size of the protein, heat shock proteins are roughly divided into six categories, namely Hsp110, Hsp90, Hsp70, Hsp60, Hsp40, and small molecule heat shock proteins (such as Hsp10). This disclosure specifically relates to Hsp90. Hsp90 protein includes Hsp90AA1 and Hsp90AB1. The amino acid sequence of murine Hsp90AA1 is shown in NP_034610.1 (SEQ ID NO:1), with its mRNA sequence in NM_010480.5 (SEQ ID NO: 2); the amino acid sequence of murine Hsp90AB1 is shown in NP_032328.2 (SEQ ID NO: 3), with its mRNA sequence in NM_008302.3 (SEQ ID NO: 4). The amino acid sequence of human Hsp90AA1 is shown in NP_005339.3 (SEQ ID NO: 5), with its mRNA sequence in NM_005348.3 (SEQ ID NO: 6); the amino acid sequence of human Hsp90AB1 is shown in NP_001258898.1 (SEQ ID NO: 7), with its mRNA sequence in NM_001271969.1 (SEQ ID NO: 8); the amino acid sequence of murine Hsp70 is shown in NP_034608.2 (SEQ ID NO: 9), with its mRNA sequence in NM_010478.2 (SEQ ID NO: 10)); the amino acid sequence of murine Hsp60 is shown in NP_001343441.1 (SEQ ID NO: 11), with its mRNA sequence in NM_001356512.1 (SEQ ID NO: 12); the amino acid sequence of murine Hsp40 is shown in NP_061278.1 (SEQ ID NO: 13), with its mRNA sequence in NM_018808.3 (SEQ ID NO: 14). It should be understood that the heat shock proteins described herein, especially Hsp90 protein, include heat shock proteins derived from various animals of interest, especially mammals.

Also included herein are Hsp90 mutants (also referred to as mutant Hsp90 protein or Hsp90 muteins). The Hsp90 protein includes an N-terminal domain, a middle domain and a C-terminal domain. The N-terminal domain of the wild-type Hsp90 protein is as shown in the amino acid residues 1-233 of the murine SEQ ID NO:1 or the amino acid residues 1-228 of the SEQ ID NO: 3, or the amino acid residues 1-233 of the human Hsp90 protein SEQ ID NO:5 or the amino acid residues 1-228 of SEQ ID NO: 7; the C-terminal domain is as shown in the amino acid residues 566-733 of the murine SEQ ID NO:1 or the amino acid residues 557-724 of the SEQ ID NO: 3, or the amino acid residues 565-732 of the human Hsp90 protein SEQ ID NO:5 or the amino acid residues 557-724 of SEQ ID NO: 7. The Hsp90 mutant herein include a mutant that retains the N-terminal domain and C-terminal domain of the wild-type Hsp90 protein, and only has a mutation in the middle domain. The mutation in the middle domain can be an insertion, a substitution or a deletion, and the middle domain can even be completely replaced by other amino acid sequences, as long as the mutation does not affect the binding of the N-terminal domain and C-terminal domain of the mutant Hsp90 protein to the α4 integrin. Such mutants retain the ability to interact with α4 integrin because they retain the N- and C-terminal domains of the wild-type Hsp90. In certain embodiments, Hsp90 mutants include mutants that have a mutation causing its inability to self-dimerize. Such a mutation may be a deletion mutation, especially a deletion mutation in the C-terminal domain, such as deletion of the last 49 amino acids of the C-terminal domain. In certain embodiments, Hsp90 mutants include mutants that lack the N-terminal domain, the C-terminal domain, or both the N-terminal domain and the C-terminal domain.

In this disclosure, α4 integrin has its well-known meaning in the art (murine α4 gene access number GeneID: 16401; human α4 gene access number GeneID: 3676). It is mainly expressed on the surface of immune cells and mediates the adhesion and migration of immune cells by binding with the vascular endothelial cell surface ligand VCAM-1 or MAdCAM-1.

It should be understood that, unless specifically mentioned herein, Hsp90 and α4 integrin generally refer to Hsp90 and α4 integrin from various sources.

The regulation of the interaction between Hsp90 protein and α4 integrin can be realized by regulating Hsp90 protein and/or regulating α4 integrin.

For example, the interaction between Hsp90 protein and α4 integrin can be improved by enhancing the expression or activity of Hsp90 protein. Methods for enhancing the expression of Hsp90 protein include, but are not limited to, high temperature treatment, overexpression of Hsp90 protein, and expression of its mutants. Specifically, in order to up-regulate the expression of Hsp90 protein, the well-known method of thermal stress can be adopted. Generally, the expression of Hsp90 in a cell can be up-regulated at a temperature of 38.5°C or above. Of course, the high temperature should not cause the treated subject, such as the cell itself, or the animal body containing the cell to feel intense discomfort or death. Therefore, the duration of thermal stress is usually within 12 hours. Gene engineering techniques well known in the art can be used to achieve overexpression of Hsp90 protein. For example, a vector expressing Hsp90 protein can be constructed and transferred into cells of interest to obtain cells overexpressing Hsp90. The vector can be an expression vector that does not integrate into the genome of the cell; or it can also be an integration vector (or an insertion vector, one kind of targeting vectors), which can integrate the polynucleotide sequence expressing Hsp90 into the genome of the cell and achieve the expression. In certain embodiments, the interaction between Hsp90 and α4 integrin can be enhanced by expressing a mutant Hsp90 protein in cells of interest. Such mutant Hsp90 protein retains the N-terminal domain and C-terminal domain of the wild-type Hsp90 protein, while the middle domain between the N-terminal domain and the C-terminal domain of the wild-type Hsp90 protein can be replaced by other amino acid sequences. For example, the middle domain can be replaced by an amino acid sequence with the same or similar length of the middle domain of the wild-type Hsp90 protein (for example, within 10 amino acid residues difference in length). Alternatively, the mutant Hsp90 protein is the Hsp90 mutant described herein that has a mutation causing its inability to mediate the self-dimerization of the Hsp90 protein. Similarly, the vector used to express the mutant Hsp90 protein may be an expression vector or an integration vector.

In some embodiments, in addition to increasing the expression of Hsp90 protein or its mutants, the expression of α4 integrin can be increased. Similarly, genetic engineering techniques can be used to overexpress α4 integrin in cells of interest. The vector used to express α4 integrin may be an expression vector or an integration vector.

Methods of down-regulating the interaction between Hsp90 protein and α4 integrin include but are not limited to, down-regulating the expression of Hsp90 protein and/or α4 integrin in cells of interest. Methods known in the art can be used to down-regulate the expression of Hsp90 protein in immune cells. For example, genetic engineering techniques can be used to knock out or knock down the expression of Hsp90 protein and/or α4 integrin in immune cells. These methods include, but are not limited to: transferring a gene knockout vector into immune cells of interest, and knocking out the coding sequence of the Hsp90 protein and/or α4 integrin in the genome with the vector, so that immune cells do not express the Hsp90 protein and/or α4 integrin; or knocking out Hsp90 and/or α4 integrin in immune cells using ZFN, TALEN or CRISPR/Cas9. In certain embodiments, gene silencing mediated by interfering RNA (such as siRNA) can be used to knock down the expression of Hsp90 protein and/or α4 integrin. Alternatively, in some embodiments, certain biological activities of Hsp90 may be necessary. Therefore, the coding sequence for expressing the mutant Hsp90 protein can be integrated into the genome of the cell of interest using a gene knock-in vector (a replacement vector, one kind of targeting vectors), replacing the coding sequence of the wild-type Hsp90 protein, so that the cell of interest expresses the mutant Hsp90 protein, which does not bind to α4 integrin or provides a reduced binding (such as lower than 50%) as compared to the non-mutated Hsp90 protein, but retains the biological activities of Hsp90 protein. Exemplary mutant Hsp90 protein include, but are not limited to, Hsp90 mutants lacking the N-terminal domain and/or C-terminal domain of the Hsp90 protein, or Hsp90 mutants having a mutation in the N-terminal and/or C-terminal domain causing the binding of N-terminal and/or C-terminal domain to α4 integrin weakened or eliminated as compared with the wide-type.

In certain embodiments, the interaction between Hsp90 protein and α4 integrin can be down-regulated by mutating α4 integrin, thereby down-regulating the interaction. Since α4 integrin binds to Hsp90 protein through its intracellular segment, corresponding mutation(s) are made in the intracellular segment of α4 integrin (amino acid residues 968-999 of human and murine α4 integrin, wherein only residues 992 are different, with residue 992 of the human α4 integrin being isoleucine (I) and that of the murine α4 integrin being valine (V)). Mutations can be insertions, deletions or substitutions. The number of mutated amino acids is not limited. However, it is necessary to retain the KAGFFKR sequence in the intracellular segment of α4 integrin to maintain the salt bridge between α4 integrin and β integrin, to avoid destroying the salt bridge and causing abnormal activation of integrin. Exemplary mutations include, but are not limited to, mutation(s) in at least one of R985, W989, and Y991. Preferably, the mutation occurs at position R985. For example, R985 can be deleted, or R985 can be replaced by other amino acid(s), especially by amino acid residue(s) that do not belong to the same category. For example, R (arginine) is a positively charged polar amino acid (basic amino acid), with L (lysine) and H (histidine) in the same category. Therefore, any amino acid(s) other than these two amino acids can be used to replace R. The amino acid residues at the other two positions can also be replaced by other amino acid(s), preferably by amino acid(s) that do not belong to the same category. Preferably, the amino acid residue used for substitution is alanine (A). In certain embodiments, for murine α4 integrin, the mutation is the deletion of 9 amino acids residues "ENRRDSWSY" or 17 amino acid residues "ENRRDSWSYVNSKSNDD" in the intracellular segment. When α4 integrin of other species are used, the above-mentioned mutations can occur at positions corresponding to the murine α4 integrin described herein. The mutant α4 integrin is also included in the scope of this disclosure. Methods known in the art can be used to introduce mutant α4 integrin into immune cells. For example, a gene knock-in vector can be used to integrate the coding sequence of the mutant α4 integrin into the genome of the cell of interest to replace the coding sequence of wild-type α4 integrin, so that the cell of interest expresses the mutant α4 integrin.

In this disclosure, amino acids can be roughly divided into: (1) non-polar amino acids (hydrophobic amino acids), including alanine (Ala), valine (Val), leucine (Leu), isoleucine (Ile), proline (Pro), phenylalanine (Phe), tryptophan (Trp) and methionine (Met); (2) polar amino acids (hydrophilic amino acids), including (a) polar uncharged amino acids: glycine (Gly), serine (Ser), threonine (Thr), cysteine (Cys), tyrosine (Tyr), asparagine (Asn) and glutamine (Gln); (b) positively charged polar amino acids (basic amino acids): lysine (Lys), arginine (Arg) and histidine (His); and (c) negative charged polar amino acids (acidic amino acids): aspartic acid (Asp) and glutamic acid (Glu).

The transcription level of Hsp90 protein is regulated by transcription factors such as HSF1. HSF1 can tightly bind to the heat shock element (HSE) of Hsp90 gene, and then Hsp90 gene can be transcribed by RNA polymerase. In addition to the transcriptional activation by HSF1, Hsp90AB1 is also regulated by the IL-6 transcription factor NF-IL6 (nuclear factor for IL-6) and STAT-3 (signal transducer and activator of transcription 3). In addition, Hsp90AB1 is also regulated by IFN-γ (interferon-y) activated STAT-1 transcription. By adding certain agonists or inhibitors of related signaling pathways, the expression of these transcription factors can be promoted or inhibited to dynamically regulate the expression of Hsp90 protein.

The interaction between Hsp90 protein and α4 integrin can be up-regulated in immune cells to promote the directional migration of immune cells, enhance immune response, eliminate pathogen infection or kill tumor cells. Therefore, this disclosure also provides a method for enhancing immune response, eliminating pathogen infection or treating solid tumors, the method comprising up-regulating the interaction between Hsp90 protein and α4 integrin in immune cells of a subject. Immune cells can be treated with an expression vector and/or an integration vector of the wild-type Hsp90 protein, the mutant Hsp90 protein being only mutated in its middle domain, and/or the mutant Hsp90 protein having a mutation causing its inability to self-dimerize; and/or immune cells can be treated with an expression vector and/or an integration vector of α4 integrin; and/or immune cells can be treated with reagents that increase the transcription levels of Hsp90 and/or α4 integrin naturally occurred in immune cells, and/or immune cells can be placed at a fever-range high temperature, thereby enhancing the interaction between Hsp90 and α4 integrin in immune cells.

In this disclosure, pathogen infection is an acute local or systemic infection caused by pathogens or conditional pathogens that invade local tissues or blood circulation, grow and reproduce in large numbers, and produce toxins and other metabolites, including urogenital and gastrointestinal infections caused by *Escherichia coli,* gram-negative bacteria, or anaerobic bacteria, and respiratory tract infections caused by pneumococcus. In certain embodiments, the pathogen infections described herein include, but are not limited to, infections caused by *Salmonella typhimurium, E. coli* and the like. Herein, a tumor refers to a physiological disorder in mammals that is usually characterized by unregulated cell growth. Tumors can be divided into benign tumors and malignant tumors, and malignant tumors are also called cancers. Tumors can be divided into solid tumors or hematological tumors. Enhancing the interaction between the Hsp90 protein and α4 integrin in the subject's immune cells can promote the migration of immune cells to solid tumors, thereby ultimately killing tumor cells and inhibiting tumor growth. Examples of tumors include, but are not limited to, squamous cell carcinoma, small cell lung cancer, non-small cell lung cancer, lung adenocarcinoma, lung squamous cell carcinoma, peritoneal cancer, hepatocellular carcinoma, gastrointestinal cancer, pancreatic cancer, glioma, cervical cancer, ovarian cancer, liver cancer, bladder cancer, liver sarcoma, breast cancer, colon cancer, colorectal cancer, endometrial cancer or uterine cancer, salivary gland cancer, kidney cancer, prostate cancer, vulvar cancer, thyroid cancer, head and neck cancer, etc.

Alternatively, by down-regulating the expression of Hsp90 protein in immune cells or reducing the interaction between Hsp90 protein and α4 integrin, the migration of immune cells to secondary lymphoid organs or local tissues can be inhibited, thereby increasing the concentration of immune cells in the blood circulation, and promoting the elimination of blood infections (such as sepsis) or blood tumors by immune cells. Hematological tumors include lymphoma, leukemia, myeloma, or lymphoid malignancies, as well as cancer of the spleen and cancer of the lymph nodes. Examples of hematological tumors include B-cell related cancers, including, for example, high-grade, intermediate-grade, and low-grade lymphomas, including B-cell lymphomas, such as mucosal-associated lymphoid tissue B-cell lymphoma and non-Hodgkin's lymphoma (NHL), mantle cell lymphoma, Burkitt's lymphoma, small lymphocytic lymphoma, marginal zone lymphoma, diffuse large cell lymphoma, follicular lymphoma, Hodgkin's lymphoma and T cell lymphoma; and leukemia, including secondary leukemia, chronic lymphocytic leukemia (CLL) such as B-cell leukemia (CD5+ B lymphocytes), myeloid leukemia such as acute myeloid leukemia, chronic myeloid leukemia, lymphoid leukemia such as acute lymphocytic leukemia (ALL) and myelodysplasia; and other hematology- and/or B cell or T cell-related cancers. Hematological tumors also include cancers of other hematopoietic cells, and the hematopoietic cells include polymorphonuclear leukocytes, such as basophils, eosinophils, neutrophils and monocytes, dendritic cells, platelets, red blood cells and natural killer cells.

On the other hand, down-regulating the interaction between Hsp90 protein and α4 integrin can be used to reduce immune response. For example, in chronic inflammation or autoimmune diseases, the interaction between Hsp90 protein and α4 integrin can be downregulated (such as inhibiting the expression of Hsp90 or destroying the interaction of Hsp90-α4 integrin) to reduce the immune response, inhibit the development of chronic inflammation or reduce autoimmune response, and maintain immune homeostasis. Chronic inflammation includes Inflammatory Bowel Disease (IBD), including Crohn's disease and ulcerative colitis, rheumatic arthritis, rheumatoid arthritis, non-specific chronic inflammation and granulomatous inflammation. Autoimmune diseases may include, but are not limited to, Systemic Lupus Erythematosus, asthma, psoriasis, multiple Sclerosis, Celiac Disease, insulin-dependent diabetes, Shaker Lien's syndrome or Sjogren's syndrome, Hashimoto's thyroiditis, Graves' disease, spontaneous thrombocytopenia purpurea and aplastic anemia, etc.

The interaction between Hsp90 and α4 integrin in immune cells can be weakened or destroyed by: knocking out Hsp90 protein or α4 integrin from immune cells by transferring a gene-knockout vector into the immune cells, and/or knocking out Hsp90 protein or α4 integrin from immune cells using gene editing techniques such as ZFN, TALEN or CRISPR/Cas9 and the like, and/or knocking down the expression of Hsp90 protein and/or α4 integrin by interfering-RNA mediated gene silencing, and/or integrating into the genome of immune cells an expression cassette expressing the Hsp90 mutant that has weakened or no interaction with α4 integrin or that lacks N-terminal and/or C-terminal domains, and/or an expression cassette expressing the α4 integrin mutant that has weakened or no interaction with Hsp90 protein by transferring a gene insertion vector into immune cells while knocking out the coding sequence of wild-type Hsp90 and/or α4 integrin. The ZFN, TALEN and CRISPR/Cas9 applicable to the present disclosure are well known in the art. Each technique knocks out target genes through the combination of DNA recognition domain and endonuclease.

The regulation method described herein can be an *in vitro* method or an *in vivo* method. For example, in certain embodiments, provided herein is a method for preparing and enhancing the directional migration ability of immune cells, the method includes increasing the interaction between Hsp90 protein and α4 integrin of the immune cells as compared to the corresponding wide-type cells (i.e. immune cells directly isolated from the subject) by one or more of the following treatments on immune cells *in vitro:* (1) treating (such as transferring) immune cells with an expression vector and/or an integration vector of the wild-type Hsp90 protein, the mutant Hsp90 protein being only mutated in its middle domain, and/or the mutant Hsp90 protein having a mutation causing its inability to self-dimerize; (2) treating (such as transferring) immune cells with an expression vector and/or an integration vector of α4 integrin; (3) treating (such as transferring or co-incubating) immune cells with reagents that increase the transcription levels of Hsp90 and/or α4 integrin naturally occurred in immune cells; (4) treating immune cells with a fever-range high temperature (such as placing the immune cells under the high temperature). The expression vectors, integration vectors, and reagents can be the reagents described in any of the embodiments herein. The enhanced (or increased) directional migration ability refers to an increase in the directional migration ability of immune cells treated by the methods described herein, for example, by at least 10%, at least 20%, at least 30%, at least 50%, or at least 100% compared with immune cells that have not been treated by the methods described herein. A method known in the art for testing the directional migration ability of immune cells can be used to evaluate the directional migration ability of the immune cells of the present disclosure. For example, the method described in section 1.2.6 below can be used for the evaluation. Specifically, chemokine induced cell migration can be used. By counting the cells that have migrated to the underneath of the wells, the ability of cells to migrate across the membrane is relatively quantified. The more cells migrated, the stronger the ability of directional migration. Thus, for example, in certain embodiments, the immune cells treated with the methods described herein migrate across the membrane by at least 1.1 times, at least 1.2 times, at least 1.3 times, at least 1.5 times or at least 2 times than immune cells not treated with the methods described herein. Thus, for example, in certain embodiments, the number of the immune cells treated with the methods described herein and migrated across the membrane is at least 1.1 folds, at least 1.2 folds, at least 1.3 folds, at least 1.5 folds or at least 2 folds of the number of the immune cells not treated with the methods described herein.

In certain embodiments, the present disclosure provides a method for down-regulating the directional migration ability of immune cells, the method comprises decreasing the interaction between Hsp90 protein and α4 integrin of the immune cells as compared to the corresponding wide-type cells by treating the immune cells with one or more of the following reagents *in vitro:* (1) reagents knocking out Hsp90 protein or knocking down its expression in immune cells; (2) reagents knocking out α4 integrin or knocking down its expression in immune cells; (3) reagents achieving the expression of the Hsp90 mutant that has weakened or no interaction with α4 integrin in immune cells; (4) reagents achieving the expression of α4 integrin mutant that has weakened or no interaction with Hsp90 protein in immune cells. The aforementioned expression vector, integration vector or ZFN, TALEN or CRISPR/Cas9 can be used as the reagent to achieve the knockout, knockdown or expression of the mutant Hsp90 protein and mutant α4 integrin. In some embodiments, the Hsp90 mutants are mutants lacking the N-terminal and/or C-terminal domain, or mutants having a mutation in the N-terminal and/or C-terminal domain and causing the interaction between Hsp90 and α4 integrin weakened or eliminated.

The weakened (or decreased) directional migration ability refers to an decrease in the directional migration ability of immune cells treated by the methods described herein, for example, by at least 10%, at least 20%, at least 30%, or at least 50% compared with immune cells that have not been treated by the methods described herein. As described above, any method known in the art for testing the directional migration ability of immune cells can be used to evaluate the directional migration ability of the immune cells of the present disclosure. For example, the method described in section 1.2.6 below can be used for the evaluation.

This disclosure also provides an immune cell, such as T lymphocytes, B lymphocytes, natural killer cells, monocytes, and dendritic cells. Compared with corresponding wild-type cells, the immune cell has increased or decreased interaction between Hsp90 and α4 integrin. In certain embodiments, the immune cell is a genetically engineered cell that: (1) overexpresses Hsp90 protein or Hsp90 mutant protein compared with wild-type cells, wherein, as compared with wild-type Hsp90 protein, said Hsp90 mutant only has a mutation in the middle domain, or the Hsp90 protein has a mutation causing its inability to self-dimerize; and/or (2) comprises an expression vector of α4 integrin; and/or (3) contains or expresses the Hsp90 transcription activator naturally occurred in immune cells. In some embodiments, the immune cell is a genetically engineered cell that, as compared with wild-type cells: (1) does not express Hsp90 or has reduced expression level of Hsp90, or expresses Hsp90 having reduced activity, or expresses Hsp90 mutants; and/or (2) has reduced expression level of α4 integrin, or expresses α4 integrin mutants, thereby weakening or eliminating the interaction between Hsp90 and α4 integrin in the genetically engineered immune cell. The immune cells described herein are living cells. In some embodiments, the immune cells are prepared by the method for regulating the directional migration ability of immune cells as described in any of the embodiments herein.

In certain embodiments, this disclosure also provides a pharmaceutical composition comprising the living immune cells described herein (promoting the interaction between Hsp90 and α4 integrin) and optionally a pharmaceutical acceptable carrier or excipient. Herein, the pharmaceutically acceptable carrier, excipient or stabilizer is non-toxic to the immune cells and recipients of the immune cells at specified dose and concentration, and may include those commonly used in immune cell therapy for the delivery of living immune cells. The immune cells are present in a therapeutically effective amount in the pharmaceutical composition. The therapeutically effective amount of the immune cells described herein are dependent on factors such as different types of immune cells, and age, sex and disease severity of the patient receiving the immune cells. The immune cells described herein can be administered by conventional methods of administration, for example, conventional immune cell therapy can be used to perform the retransfusion of immune cells.

The immune cells and pharmaceutical compositions described herein can be used to treat various diseases or symptoms that benefit from the natural biological functions of the immune cells. For example, the immune cells as described herein can be T cells, wherein genetically engineered cytotoxic T cells or pharmaceutical compositions thereof can be used to treat various diseases or symptoms (i.e., various diseases or symptoms caused by cell infection) that are benefit from the natural biological functions of cytotoxic T cells (such as destroying infected cells). More specifically, in certain embodiments, the immune cells described herein and pharmaceutical compositions thereof are particularly useful for the treatment of pathogen infections and tumors described herein.

Therefore, in certain embodiments, this disclosure also provides an immune cell therapy, especially tumor immunotherapy, comprising: obtaining immune cells from a subject to be treated; treating the immune cells *in vitro* to enhance the interaction between Hsp90 and α4 integrin, which makes the immune cells have increased migration ability compared with the corresponding wild-type cells; and retransfusing the immune cells. In certain embodiments, the immune cell therapy described herein comprises treating cells of a subject in need with high temperature stress, wherein the high temperature is 38.5°C or above, usually not more than 40°C. The period of high temperature stress can be determined according to different subjects and different diseases. Moreover, if the temperature is higher, the stress period is usually shorter; otherwise, the stress period can be relatively longer. In other embodiments, the patient having tumor can be induced to fever by different means under medical monitoring, and the inhibitory effect of fever treatment on tumor development can be observed after a certain period of time. Although scientists have known for a long time that the fever caused by infection can cause concurrent remission of tumor patients' diseases, but its systemic mechanism is still unclear. This disclosure provides a possible mechanism that fever promotes immune cells to clear solid tumor cells by promoting the Hsp90-α4 integrin signal axis. Fever treatment under medical monitoring may become a new immunotherapy for patients suffering from cancer. The inducers that may cause fever in the body include various pathogens, metabolites of pathogenic microorganisms or their toxins, or cytokines that can cause fever in the body. Pathogens include but are not limited to bacteria, viruses, fungi, mycoplasma, chlamydia, rickettsiae, spirochetes, and plasmodium. Cytokines include interleukin-6, interleukin-1β, tumor necrosis factor-a and prostaglandin E2.

In certain embodiments, this document also provides a method for treating sepsis, hematological tumors, chronic inflammation, or autoimmune diseases, the method comprising the step of reducing the interaction between Hsp90 and α4 integrin in immune cells of the subject. In some embodiments, (1) the immune cells have knocked out or knocked down expression of Hsp90 and/or α4 integrin as compared with wild-type cells; and/or (2) the immune cells contain an expression vector of mutated Hsp90 and/or α4 integrin, wherein the mutation can inhibit the binding of endogenous Hsp90 to α4 integrin. The treatment can be achieved in a conventional manner. For example, the genetically engineer immune cells from the subject can treated with conventional immune cell therapy to reduce the interaction between Hsp90 and α4 integrin, and then the genetically engineered immune cells can be retransfused to the individual or the subject; or the subject can be administrated with targeting drugs harboring small interfering RNAs (siRNAs) or reagents required for gene editing technology, such as guide RNAs (sgRNAs) and related endonuclease (such as Cas9 protein complex), thereby reducing the expression of the target protein in the immune cells of the subject.

The present invention also includes the coding sequences and complementary sequences of the various mutants described herein, and nucleic acid constructs comprising the coding sequences or complementary sequences. Herein, a nucleic acid construct is an artificially constructed nucleic acid segment that can be introduced into a target cell or tissue. The nucleic acid construct contains the coding sequence described herein or complementary sequence thereof, and one or more regulatory sequences operably linked to these sequences. The regulatory sequence can be a suitable promoter. The promoter sequence is usually operably linked to the coding sequence of the amino acid sequence to be expressed. The promoter can be any nucleotide sequence having transcriptional activity in the selected host cell, including mutant, truncated, and hybrid promoters, which can be obtained from a gene encoding an extracellular or intracellular polypeptide that is homologous or heterologous to the host cell. The regulatory sequence may also be a suitable transcription terminator, a sequence recognized by the host cell to terminate transcription. The terminator is connected to the 3' terminus of the nucleotide sequence encoding the polypeptide, and any terminator that is functional in the host cell of choice can be used herein.

In certain embodiments, the nucleic acid construct is a vector. Specifically, the coding sequences described herein can be cloned into many types of vectors, including but not limited to plasmids, phagemids, phage derivatives, animal viruses, and cosmids. The vector can be an expression vector or a cloning vector.

Generally, suitable vectors contain a replication origin that functions in at least one organism, promoter sequences, convenient restriction enzyme sites, and one or more selectable markers. Representative examples of these promoters are: the lac or trp promoter of *E.coli;* the PL promoter of phage λ; eukaryotic promoters including the CMV immediate early promoter, the HSV thymidine kinase promoter, the early and late SV40 promoters, methanol oxidase promoters of *Pichia pastoris,* and other known promoters that can control gene expression in prokaryotic or eukaryotic cells or their viruses. Marker genes can provide phenotypic traits for selection of transformed host cells, including but not limited to dihydrofolate reductase, neomycin resistance, and green fluorescent protein (GFP) for eukaryotic cell, or tetracycline or ampicillin resistance for *E.coli.* When the polynucleotides described herein are expressed in higher eukaryotic cells, transcription will be enhanced if an enhancer sequence is inserted into the vector. Enhancers are cis-acting factors of DNA, usually are about 10bp to 300bp, which act on the promoter to enhance gene transcription.

Those skilled in the art know how to select appropriate vectors, promoters, enhancers and host cells. Methods well known to those skilled in the art can be used to construct expression vectors containing the polynucleotide sequences described herein and appropriate transcription/translation control signals. These methods include *in vitro* recombinant DNA technology, DNA synthesis technology, *in vivo* recombinant technology and so on.

Also included herein is a host cell comprising the polynucleotide sequences described herein or nucleic acid constructs thereof. The host cell can be a prokaryotic cell, such as a bacterial cell; or a lower eukaryotic cell, such as a yeast cell; a filamentous fungal cell, or a higher eukaryotic cell, such as a mammalian cell. Representative examples are: bacterial cells such as cells of *Escherichia coli, Streptomyces; Salmonella typhimurium;* fungal cells such as yeast cells, filamentous fungi cells; plant cells; insect cells such as *Drosophila* S2 or Sf9 cells; animal cells such as CHO, COS, 293 cells, or Bowes melanoma cells, etc.

The vectors herein can be introduced into host cells by conventional methods, including microinjection, gene gun, electroporation, virus-mediated transformation, electron bombardment, calcium phosphate precipitation, and the like.

Further, the disclosure also includes the uses of various products described herein, including uses in manufacture of a medicament. Specifically, the disclosure includes: (1) use of a reagent that enhances the interaction between Hsp90 and α4 integrin in immune cells in the manufacture of immune cells for immunotherapy; preferably, the reagent is selected from the group consisting of: an expression vector or an integration vector for the wild-type Hsp90 protein, or the mutant Hsp90 protein being only mutated in its middle domain, or the mutant Hsp90 protein having a mutation causing its inability to self-dimerize; and an expression vector and/or integration vector for α4 integrin; (2) use of immune cells with enhanced interaction between Hsp90 protein and α4 integrin in the manufacture of a medicament for the treatment of pathogen infections or tumors; (3) use of a reagent that weakens the interaction between Hsp90 and α4 integrin in immune cells in the manufacture of immune cells for the treatment of sepsis, hematological tumors, chronic inflammations or autoimmune diseases; preferably, the reagent is selected from the group consisting of: a Hsp90 mutant lacking the N-terminal and/or C-terminal domain or having a mutation in the N-terminal and/or C-terminal domain causing the interaction between Hsp90 and α4 integrin weakened or eliminated as compared with the wide-type Hsp90, and/or a targeting vector of the Hsp90 mutant; a α4 integrin mutant having a mutation in its intracellular segment which causes the interaction between Hsp90 and α4 integrin weakened or eliminated, and/or a targeting vector of the α4 integrin mutant; and ZFN, TALEN and/or CRISPR/Cas9 reagents and/or small interfering RNA used to knock out Hsp90 and/or α4 integrin or to knock down its/their expression; and (4) use of immune cells with weakened interaction between Hsp90 and α4 integrin in the manufacture of a medicament for the treatment of sepsis, hematological tumors, chronic inflammations or autoimmune diseases. Reagents for ZFN, TALEN and/or CRISPR/Cas9 suitable for the present invention are well known in the art, and include but are not limited to DNA recognition domains (such as sgRNA) and endonucleases.

In certain embodiments, this disclosure also includes: Hsp90 or a mutant thereof for immune cell therapy, wherein, as compared with wild-type Hsp90 protein, said mutant only has a mutation in the middle domain, or the mutant has a mutation causing its inability to self-dimerize; α4 integrin for immune cell therapy; a Hsp90 mutant for treating sepsis, hematological tumors, chronic inflammations or autoimmune diseases, wherein the Hsp90 mutant lacks the N-terminal and/or C-terminal domain or having a mutation in the N-terminal and/or C-terminal domain causing its interaction with α4 integrin weakened or eliminated as compared with the wide-type; a α4 integrin mutant described herein for treating sepsis, hematological tumors, chronic inflammations or autoimmune diseases, wherein the α4 integrin mutant has a mutation in its intracellular segment; immune cells described herein for treating sepsis, hematological tumors, chronic inflammation or autoimmune diseases; and immune cells described herein for treating pathogen infections or tumors.

The present disclosure will be illustrated by way of specific examples below. It should be understood that these examples are merely illustrative and are not intended to limit the scope of the present disclosure. Unless otherwise specified, the materials and methods used in the examples are conventional materials and methods in the art.

### A. Materials and methods

### 1.1. Experimental Materials

### 1.1.1 Common buffer

TBS: 20 mM Tris-HCl (pH 7.4), 150 mM NaCl, ImM CaCl₂, ImM MgCl₂;
Cell lysis buffer: TBS, 1% Triton X-100, 0.05% Tween 20, Complete Protease Inhibitor Cocktail Tablets, PhosSTOP Phosphatase Inhibitor Cocktail Tablets;
***Immunoprecipitation buffer (IP buffer)**:* TBS, 1% Triton X-100, 0.05% Tween 20;
**2×SDS protein loading buffer**: 100 mM Tris-HCl (pH6.8), 4% SDS, 0.2% bromophenol blue, 20% glycerol, 10% β-Me;
**Tris-glycine protein running buffer**: 25 mM Tris, 250 mM glycine, 0.1% SDS;
**Protein transfer buffer**: 3 g Tris, 14.4 g glycine, 200 ml methanol, added Milli-Q H₂O to 1L;
**TBST buffe**r: 8.8 g NaCl, 6 g Tris, 0.5 ml Tween-20, pH 7.5, added Milli-Q H₂O to 1L;
**PBS**: 8 g NaCl, 0.2 g KCl, 3.63 g Na₂HPO₄•3H₂O, 0.24 g KH₂PO₄, added Milli-Q H₂O to 1L, pH 7.4, sterilizated by filtration;
**HBS**: 20 mM HEPES, 150 mM NaCl, added to 1L, pH 7.4, sterilizated by filtration;
**2**×**HBS**: 8.0 g NaCl, 0.37 g KCl, 201 mg Na₂HPO₄·7H₂O, 1.0 g glucose, 5.0 g Hepes, added Milli-Q H₂O to 500 ml, pH 7.05, sterilizated by filtration, stored at 4 °C;
**LB culture medium**: 10 g peptone, 10 g NaCl, 5 g yeast extract;
**Buffers used in the flow chamber experiment**:
   Ca²⁺&Mg²⁺-free HBSS: 137 mM NaCl, 5.4 mM KCl , 0.4 mM KH₂PO₄, 0.3 mM Na₂HPO₄, 4.2 mM NaHCO₃, 5.6 mM glucose, pH 7.4;
   Coating buffer: PBS, 10 mM NaHCO₃, pH9.0;
   Blocking buffer: 2% BSA in HBSS;
   Washing buffer: 0.2 g BSA, 40 ml HBSS, 400 µl EDTA (0.5 M, pH 8.0);
   Buffer A: 0.225 g BSA, 45 ml HBSS;
**Buffers for purification of integrin intracellular domain model proteins (His-Tag)**:
   Charge Buffer: 50 mM NiCl₂;
   Lysis buffer: 20 mM Tris-HCl (pH 7.9), 0.5 M NaCl, 5 mM imidazole, 1% Triton X-100, 1 µg/ml aprotinin;
   Binding buffer (8M urea): 20 mM Tris-HCl (pH 7.9), 0.5 M NaCl, 5 mM imidazole, 0.2% Triton X-100, 8M urea;
   Washing buffer (8M urea): 20 mM Tris-HCl (pH 7.9), 0.5 M NaCl, 60 mM imidazole, 0.2% Triton X-100, 8M urea;
   Elution buffer (8M urea): 20 mM Tris-HCl (pH 7.9), 0.5 M NaCl, 1 M imidazole, 0.2% Triton X-100, 8M urea;
   XT buffer: 50 mM NaCl, 10 mM Pipes, 150 mM sucrose, 50 mM NaF, 40 mM Na₄P₂O₇.10H₂O, 25 mM imidazole, 1 mM Na₃VO₄, 0.5% Triton X-100, pH 6.8;
**Buffers for purification of GST-Tag fusion protein**:
   Lysis buffer: PBS, 1% Triton X-100;
   Elution buffer: 20 mM reduced glutathione, 50 mM Tris-HCl, pH 8.0;
**Buffers for purification of antibodies or Fc-Tag fusion protein**:
   Binding buffer: 0.1 M Tris-HCl, pH 8.0;
   Elution buffer: 0.1 M glycine, pH 3.0;
   Neutralization buffer: 1 M Tris-HCl, pH 8.0.

### 1.1.2 Basic chemical reagents

DMEM medium, RPMI 1640 medium, penicillin and streptomycin, L-glutamine, TEMED, etc. were purchased from Invitrogen; fetal bovine serum was purchased from Gibco; sodium dodecyl sulfonate (SDS), HEPES, DMSO, aprotinin, leupeptin, paraformaldehyde (PFA), etc. were purchased from Sigma; peptone and yeast extract were purchased from OXOID LTD;

The remaining reagents are conventional reagents, which can be available from, for example, the Sibas Company of the Institute of Biochemistry, Green Bird Technology Development Co., Ltd., Cal Biochem, Shanghai Chinese Academy of Sciences SLD Company, Shanghai Zhenxing No. 1 Chemical Factory and Shanghai No. 1 Reagent Factory, etc.

**1.1.3 Antibody**

| **Primary Antibody** | Species | Experiment | Source |
|---|---|---|---|
| Integrin α4 (PS/2) | Rat | IF, F | ATCC |
| Integrin α4β7 (DATK32) | Rat | IF, F | ATCC |
| Integrin β7 (FIB504) | Rat | IF, F | ATCC |
| Integrin β2 (2E6) | Hamster | IF, F | ATCC |
| Integrin α4 (EPR1355Y) | Rabbit | W | ABCAM |
| integrin β2 (EP1286Y) | Rabbit | W | ABCAM |
| Hsp90AA1 (ab2928) | Rabbit | W | ABCAM |
| Hsp90AB1 (ab2927) | Rabbit | W | ABCAM |
| RhoA (EPR18134) | Rabbit | W | ABCAM |
| Hsp10 (A7437) | Rabbit | W | ABclonal |
| Hsp40 (A5504) | Rabbit | W | ABclonal |
| Hsp60 (A0969) | Rabbit | W | ABclonal |
| Hsp70 (A0284) | Rabbit | W | ABclonal |
| Hsp110 (A6622) | Rabbit | W | ABclonal |
| GST-Tag (AE006) | Rabbit | W | ABclonal |
| HA-Tag (HA-7) | Mouse | W | Sigma |
| Talin (8d4) | Mouse | W | Sigma |
| PE anti-α4 integrin (9C10) | Rat | F | BD Pharmingen |
| PE anti-aL integrin (2D7) | Rat | F | BD Pharmingen |
| PE anti-β2 integrin (C71/16) | Rat | F | BD Pharmingen |
| PerCP-Cy™5.5 anti-CD3e (145-2C11) | Rat | F | BD Pharmingen |
| Paxillin (349) | Mouse | W | BD Pharmingen |
| FAK (77) | Mouse | W | BD Pharmingen |
| Rac1 (102) | Mouse | W | BD Pharmingen |
| Cdc42 (44) | Mouse | W | BD Pharmingen |
| PE/Cy7 anti-aM integrin (M1/70) | Rat | F | Biolegend |
| PE anti-aX integrin (N418) | Hamster | F | Biolegend |
| pY397-FAK (EP2160Y) | Rabbit | W | Milli pore |
| Kindlin-3 (#13843) | Rabbit | W | Cell signaling |
| anti-aD integrin (NBP1-9023 7) | Rabbit | F | Novus |
| FITC anti-β1 integrin (sc-19656) | Hamster | F | Santa cruz |
| β-actin (ab008) | Mouse | W | Multi Sciences |

| **Secondary Antibody** | Dilution rate | Experiment | Source |
|---|---|---|---|
| HRP-goat anti-rabbit IgG | 1:10000 | W | Lianke |
| HRP-goat anti-mouse IgG | 1:10000 | W | Lianke |
| FITC-goat anti-mouse IgG | 1:1000 | F, IF | Invitrogen |
| FITC-goat anti-rat IgG | 1:1000 | F, IF | Invitrogen |
| Cy3-goat anti-rat IgG | 1:1000 | F, IF | Invitrogen |

| | | | |
|---|---|---|---|
| W-Western blotting; IP-immunoprecipitation; IF-immunofluorescence; F-flow cytometry | | | |

**1.1.4 Enzymes**

| **Name** | **Source** |
|---|---|
| KOD plus Neo | TOYOBO |
| PNK | Fermentas |
| Restriction enzyme | New England Biolabs and Fermentas |
| T4 ligase | Fermentas |
| Dpn I | Fermentas |

**1.1.5 Kits and others**

| **Name** | **Source** |
|---|---|
| Reverse transcription system | TOYOBO |
| ECL substrate | Pierce |
| DNA Mini Purification Kit | Tiangen |
| BCA Protein Assay Kit | Beyotime |
| PhosSTOP Phosphatase Inhibitor Cocktail Tablets | Roche |
| Complete Protease Inhibitor Cocktail Tablets | Roche |
| Puromycin | Inviogen |
| Hygromycin | Amresco |
| PMA | Sigma |
| ADP | Sigma |
| DAPI | Sigma |
| poly-L-lysine | Sigma |
| Chromatography qualitative analysis filter paper | Sunwah |
| Nitrocellulose membrane | Whatman |
| 0.22/0.45 µm Cellulose acetate sterilizated filter membrane | Pall |
| Protein A/G-sepharose | Amersham |
| | Pharmacia |
| Insulin needle | BD |

**1.1.6 Main instrument**

| **Name** | **Source** |
|---|---|
| PCR System | Bio-Rad and Eastwin |
| Flow chamber system | Harvard Apparatus |
| Electrophoresis System | Tianneng |
| Laser Scanning Confocal Microscope | Leica |
| ÄKATA | GE healthcare |
| NanoDrop 2000 | Thermo |

### 1.2. Experimental method

### 1.2.1 Plasmid construction

### I. Construction of plasmid of integrin intracellular domain model proteins

To study the intracellular regulatory protein of integrins, the most direct and effective method is to purify the full-length functional integrins. However, integrins have large extracellular domains and complex high-level structures. It is quite difficult to directly purify integrins with normal structure and function. To solve this problem, scientists have constructed a tail model protein that can well mimic the intracellular domain of integrins under physiological conditions. According to previous reports, an integrin intracellular domain model protein contains the following components from N-terminal to C-terminal: 1) polyhistidine tag (His-Tag) for coupling with Ni²⁺-NTA beads for subsequent expression and purification and pull down; 2) TEV restriction site for removal of the tag; 3) a pair of cysteine residues used to connect the expressed polypeptide chain into a dimer form; 4) a helical coiled coil consisting of four consecutive seven-amino-acid peptides, wherein the coiled-coil serves as a topological structure that fixes two model protein of integrin intracellular domains, which are arranged in parallel to simulate the transmembrane structure of integrin and mediate the dimerization of integrin in aqueous solution; 5) polyglycine linking sequence (glycine linker); 6) cDNA sequence of intracellular domains such as integrin α4, β1, β7 (Figure 1). Finally, the DNA sequence of the model protein of integrin intracellular domain was cloned into the prokaryotic expression vector pETDuet-1 (Novagen).

### II. Construction of eukaryotic expression vector of integrin

The full length of the integrin α4 subunit was amplified by PCR into the pCDH-puro vector (System Biosciences), and the R985A mutation and the shRNA-tolerant synonymous point mutation were achieved by Quick change method. The C-terminus of the full-length integrin α4 subunit was fused to Split GFP (GFP 1-10 and GFP S11) with a long linker, and was added into the pCDH-puro vector.

### III. Construction of eukaryotic expression vector of heat shock protein

First, using the cDNA of mouse T cells as a template, the mRNA sequences of Hsp90AA1, Hsp90AB1, Hsp70, Hsp60, and Hsp40 were respectively amplified by PCR (the amino acid sequence of Hsp90AA1 is shown in SEQ ID NO:1 with its mRNA sequence shown in SEQ ID NO: 2; the amino acid sequence of Hsp90AB1 is shown in SEQ ID NO: 3 with its mRNA sequence shown in SEQ ID NO: 4; the amino acid sequence of Hsp70 is shown in SEQ ID NO: 9 with its mRNA sequence shown in SEQ ID NO: 10; the amino acid sequence of Hsp60 is shown in SEQ ID NO: 11 with its mRNA sequence shown in SEQ ID NO: 12; the amino acid sequence of Hsp40 is shown in SEQ ID NO: 13 with its mRNA sequence shown in SEQ ID NO: 14), and added into pCDH-puro-mRuby2 or pHAGE-IRES-mcherry vector (Chen Jianfeng, Shanghai Institute of Biochemistry and Cell Biology, Chinese Academy of Sciences), wherein the fusion expression of mRuby2 or mcherry red fluorescent protein indicates transfection efficiency. The ATPase inactivating mutations Hsp90AA1-D93N and Hsp90AB1-D88N of Hsp90AA1 and Hsp90AB1 were achieved by Quick change method, respectively.

The three domains of each of the two subtypes of Hsp90 are:
1)Hsp90AA1-N-terminal domain, M1-D233; middle domain, E234-K565; C-terminal domain, K566-D733;
2)Hsp90AB1-N-terminal domain, M1-D228; middle domain, E229-S556; C-terminal domain, K557-D724.

Truncation or deletion mutations of Hsp90: Hsp90-NM (lacking C-terminal domain), Hsp90-MC (lacking N-terminal domain) and Hsp90-NC5 (lacking the last 49 amino acids of the C-terminal domain) were amplified by PCR into pCDNA3.1-(+)-HA-Tag. The HA tag is used for western blotting.

### IV. Construction of prokaryotic expression vector of heat shock protein

The N-terminal and C-terminal domains of Hsp90AA1 and Hsp90AB1 were amplified by PCR and added into the pGEX-6P-1 vector (GE Healthcare). The target protein was fused to glutathione S-transferase tag (Glutathione S-transferase) for prokaryotic expression and purification.

### V. Construction of prokaryotic expression vector of Rho GTPases effector protein

The effector protein of Rac1 and Cdc42 is PAK (p21 activating kinase), and the effector protein of RhoA is Rhotekin. The 67-150 amino acid sequence of PAK-PBD (PAK binding domain) was amplified from human cDNA, and the 7-89 amino acid sequence of Rhotekin-RBD (Rho binding domain) was amplified from murine cDNA, which were fused to the GST tag by a linker sequence for prokaryotic expression and purification and subsequent GST-Pulldown.

### VI. Enzyme digestion and electrophoresis separation and recovery of plasmids and PCR products

The corresponding enzyme digestion buffer, vector DNA or PCR products, and restriction enzymes were added to a 10-20µl reaction system, and the reaction system was incubated at 37°C for 2-4h. After adding DNA loading buffer, the reaction products were separated by 1% agarose gel electrophoresis. After the correct PCR band was identified, the PCR product was recovered with DNA Mini Purification Kit (Tiangen), and dissolved in 30 µl deionized water.

### VII. Ligation of DNA fragments and vectors

The ligation buffer, T4 ligase, the recovered DNA fragments and an appropriate amount of vector were added to a 10µl reaction system, and the reaction system was incubated overnight at 16°C.

### VIII. Transformation of E.coli DH5α competent cells with plasmid

(1) 100 µl of competent cells stored at -80°C was placed on ice until it melts;
(2) Add the prepared transformation products;
(3) Mix gently and place on ice for 30 minutes;
(4) 42°C heat shock for 45s;
(5) Hold on ice for 2 minutes;
(6) Add 900µl of liquid LB medium;
(7) Mix gently and hold in a 37°C water bath for 30 minutes.

After the recovery was complete, the EP tube was centrifuged at 5000 rpm for 5 minutes to enrich the bacteria at the bottom of the tube. In an ultra-clean table, excess supernatant was removed, leaving about 100µl, and the pellet was gently pipetted to obtain a suspension. The suspension was spread on a LB (Amp⁺) plate by glass beads and the beads were removed after spreading thoroughly. The plate was placed upside down in a 37°C biochemical incubator and incubated overnight for 12 hours.

### VIIII. Preparation of small amount plasmid DNA

Transfer about 5ml of the overnight bacteria to a 1.5ml EP tube, centrifuge at 10,000 rpm for 1 min, remove the supernatant, and suspend the bacteria with the remaining solution in the tube on a shaker. Add 200µl Sol I and mix well, then add 200µl Sol II, gently invert the tube twice before adding 200µl Sol III, invert and mix well and centrifuge at 12000rpm for 5min. Transfer the supernatant to a new tube, add 300 µl isopropanol, mix well for 15 min at room temperature, and centrifuge at 12000 rpm for 15 min. The supernatant was discarded, the pellet was washed with 70% ethanol, dried and dissolved in 50µl Milli-Q H₂O.

### 1.2.2 Cell culture and transfection

HEK (human embryonic kidney) 293T cells were cultured in DMEM supplemented with 10% fetal bovine serum (Gibco), 50 U/ml penicillin and 50 µg/ml streptomycin at 37°C and 5% CO₂.

### I. Cell recovery and culture (293T cells)

(1) 293T cells stored in liquid nitrogen were melt in a 37°C water bath;
(2) Take 1ml of DMEM culture medium slowly into the cell cryopreservation tube, gently pipette to mix, transfer to a 15ml sterile tube containing 4 ml of fresh DMEM culture medium, centrifuge at 1200rpm for 3min, and remove the supernatant;
(3) Gently resuspend the cells by 1ml of fresh DMEM culture medium, transfer to a 10cm cell culture dish, and add cell culture medium to 10ml;
(4) Incubate the dish at 37°C, 5% CO₂, and saturated humidity.

### II. Cell passaging (293T cells)

(1) After the cells were overgrown in the culture dish, discard the old culture medium;
(2) Add 3ml 1×PBS, wash gently to remove residual culture medium, then discard the PBS;
(3) Add 1ml of pancreatin and allow to rest for 10s, tap gently to dissociate the cells from the culture dish;
(4) Add 3ml of DMEM complete culture medium, stop the digestion, and pipette to form single clones;
(5) Transfer the cells into a 15ml centrifuge tube and centrifuge at 1200rpm for 3min;
(6) Discard the supernatant, add 10ml culture medium and mix well by pipetting. If passaging at 1:10, add 1ml to a petri dish containing 9ml culture medium; if passaging at 1:3, add 3ml;
(7) Incubate the dish at 37°C, 5% CO₂, and saturated humidity.

### III. Transient transfection of 293T cells through calcium phosphate

(1) Divide the cells the day before transfection so that the cells reach a density of 60-70% before transfection;
(2) 1h before transfection, change the medium with a culture medium comprising 25µM chloroquine;
(3) In a 15ml centrifuge tube, add 10µg DNA, dilute to 1095µL with MilliQ-H₂O, and then add 155µL 2M CaCl₂. Add 1250µL of 2×HBS dropwise and mix gently while adding. Then the mixture was directly added dropwise to the cells. Note that the addition should be completed within 1-2min after adding 2×HBS. And the droplets need to be evenly sprinkled on the entire surface of the dish;
(4) Incubate for 7-11h, and very small, dusty particles will be observed. Wash once and change the medium with a medium free of chloroquine;
(5) Cells were collected 48-72h after transfection for later use.

### IV. Infection of T cells by lentivirus

Calcium phosphate transfection of 293T cells was used for lentivirus packaging. The ratio of transfected plasmids was (take 10cm dish as an example): pCDH vector 20µg, psPAX2 15µg and pMD2.G 6µg. 72 hours after transfection, the culture solution comprising the lentiviral supernatant was collected with a 10ml syringe, and the supernatant was filtered with a 0.45µm filter and concentrated by ultracentrifugation to infect the target cells. The ultracentrifugation was performed at 20000rpm, 2h, 4°C. After centrifugation, the precipitate was removed and re-dissolved in 100-200µl serum-free culture medium. During infection, the cells was re-suspended with a 1:1 ratio of cell culture solution: lentivirus culture solution, while polybrene was added at a final concentration of 8µg/ml to the cell supernatant. The mixture was mixed well, incubated at 37°C, and then the medium was replaced with fresh virus culture medium after 24h to continue the infection. Detection by flow cytometry was performed after 72h.

### 1.2.3 Protein expression and purification

### I. Expression and purification of integrin intracellular domain protein

(1) Transform the plasmid, pET-Duet-integrin tail model protein, into *E. coli* BL21 (DE3) pLysS strain, overnight at 37°C;
(2) Select single clones into each 5ml LB culture medium in 2 sterilized tubes, 37°C for 12h;
(3) Transfer the culture solution to 1L of LB, add ampicillin at a final concentration of 100µg/ml, and incubate at 37°C until OD600≈0.4;
(4) Add IPTG at a final concentration of ImM, 37°C for 3h;
(5) Transfer 1L of bacteria liquid to a 500ml large centrifuge cup, 5000rpm for 5min to collect bacteria;
(6) Add 10ml lysis buffer per gram (wet weight of the bacteria) and transfer the mixture to a 50ml centrifuge tube, add 1mg/ml lysozyme and incubate at 37°C for 15min;
(7) Break bacteria by ultrasound: 10 seconds of ultrasound treatment followed by 50 seconds of resting for 49 cycles, which is performed twice. 1 mM PMSF was added before each ultrasound;
(8) Transfer the mixture to a 50ml round bottom centrifuge tube, and centrifuge at 20000g, 4°C for 20min;
(9) Add 12ml binding buffer (8M urea) to the precipitate and mix well, centrifuge at 30000g, 4°C for 20min;
(10) Processing Ni-NTA beads in the column: wash with 5 column volumes of Milli-Q H₂O; wash with 5 column volumes of 6M Gu-HCl; wash with 5 column volumes of Milli-Q H₂O; wash with 5 column volumes of 100mM EDTA; wash with 5 column volumes of Milli-Q H₂O; wash with 5 column volumes of charging buffer; equilibrate with 5 column volumes of binding buffer (8M urea);
(11) The supernatant obtained by centrifugation was added to the column and incubated with Ni-NTA beads at 4°C for 2h;
(12) Flow through the supernatant that does not bind to Ni-NTA beads;
(13) Wash with 1 column volume of binding buffer (8M urea);
(14) Wash with 3 column volume of washing buffer (8M urea);
(15) Washing each tube with 1ml elution buffer (8M urea);
(16) Purity of the target protein was assayed by SDS-PAGE with a gel concentration of 15%;
(17) Add 1 mg of integrin intracellular domain model protein to 500 µl of processed Ni-NTA beads, and incubate at room temperature for 1 hour with rotation;
(18) Remove the supernatant after centrifugation at 2800rpm for 3min, and wash the Ni-NTA beads with 1ml binding buffer (8M urea), binding buffer (4M urea), binding buffer (2M urea), and binding buffer (0M urea), respectively (gradually remove urea), wherein each are incubated for 1h with rotation at room temperature;
(19) Centrifuge at 2800rpm for 3min to remove supernatant, wash the Ni-NTA beads with 1ml XT buffer;
(20) Resuspend the Ni-NTA beads in 500µl XT buffer and store at 4°C.

### II. Expression and purification of GST tag fusion protein

(1) Transform the plasmid, pGEX-6P-1-target protein, into *E. coli* Rosetta-gami (DE3) pLysS strain, overnight at 37°C;
(2) Select single clones into each 5ml LB culture medium in 2 sterilized tubes, 37°C for 12h;
(3) Transfer to 1L LB, add ampicillin at a final concentration of 100µg/ml, and incubate at 37°C until OD600≈0.4;
(4) Add IPTG at a final concentration of 0.1mM, induce at 16°C for 3h;
(5) Transfer 1L of bacteria liquid to a 500ml large centrifuge cup, 5000rpm for 5 min to collect bacteria;
(6) Add 10ml lysis buffer per gram (wet weight of the bacteria) and transfer the mixture to a 50ml centrifuge tube, add 1mg/ml lysozyme and incubate at 37°C for 15min;
(7) Break bacteria by ultrasound: 3 seconds of ultrasound treatment followed by 7 seconds of resting for 99 cycles, which is performed 3 times. 1 mM PMSF was added before each ultrasound;
(8) Transfer the mixture to a 50ml round bottom centrifuge tube, and centrifuge at 20000g, 4°C for 20min;
(9) Equilibrate GST beads: wash 2ml GST beads (50:50 stored in 20% ethanol) in a 15ml centrifuge tube and centrifuge at 1200rpm for 5min. Repeat the washing three times to remove the ethanol in the storage solution;
(10) The supernatant was added to the equilibrated GST beads, add DTT with a final concentration of 5mM, and incubate with rotation at 4°C for 2h;
(11) Centrifuge at 750g for 3min, and remove the supernatant;
(12) Wash with PBS, PBS+0.5% Triton-X100, PBS+0.5% Triton-X100 and PBS, 5ml each; then add the GST beads to the chromatography column with 10ml PBS, and discard the flowed-through effluent;
(13) Elute the column with 10ml elution buffer, 1ml per *Eppendorf* tube;
(14) Purity of the target protein was assayed by SDS-PAGE with a gel concentration of 7-10%.
(15) If the result showed that protein purity was not good, perform purification by molecular sieve ÄKATA system: pre-equilibrating a Superdex 200 (10/300GL) column (GE healthcare) with PBS degassed by ultrasound, concentrating samples to 0.5 ml and loading the samples, and then collecting the eluate of each main peak. The protein purity was identified by SDS-PAGE, and the protein was aliquoted and stored at -80°C.

### III. Expression and purification of antibody or Fc-Tag protein

(1) Collect the culture solution of hybridoma cells or 293T expressing Fc-Tag protein by transfection, centrifuge at 2400rpm for 3min, and remove cell debris and other impurities;
(2) Equal volume mix the culture solution and binding buffer, with stiring while adding the binding buffer, pH 8.0;
(3) Centrifuge at 4°C and 15000rpm for 30min;
(4) Load the supernatant on the Protein A or Protein G column, overnight at 4°C;
(5) Wash the column with 15 column volumes of binding buffer;
(6) Elute with 10 column volumes of elution buffer. The collection tube was added with neutralization buffer at a ratio of 1:10 in advance;
(7) Collect the eluate, concentrate it by centrifugation in a concentration tube, change the buffer to HBS, and freeze at -80°C.

### 1.2.4 Detection of the expression of integrin by Flow cytometry (FACS)

(1) Add 5×10⁵ cells in a 1.5ml Ep tube, centrifuge at 3000 rpm and 4°C for 3 min, and wash with PBS;
(2) a. Dissolve the cell pellet directly in 300µl PBS and place at 4°C (Mock);
   b. Dissolve the cell pellet in 50µl PBS, add 5µg/ml antibody, mix gently, then place at 4°C, and rest for 30min. Add 1ml PBS, mix well, centrifuge at 3000rpm and 4°C for 3min, and discard the supernatant; then repeat once. Suspend the cells in 300µl PBS;
(3) Transfer the cell suspension to a cell collection tube and analyze with a flow cytometer Calibur or LSRII (BD).

### 1.2.5 Detection of cell adhesion mediated by integrins and their ligands by Flow chamber system

### I. Cell processing

(1) Add T cells in a 15ml centrifuge tube, centrifuge at 1200rpm for 3min;
(2) Wash twice with 5ml washing buffer (HBSS, 5mM EDTA, 2% BSA, pH 8.0), centrifuge at 1200rpm for 3min;
(3) Add 5ml of buffer A (HBSS, 2% BSA, pH 8.0), centrifuge at 1200rpm for 3min, then repeat once;
(4) Dissolve the cells in 1ml buffer A and count cell number. The final cell concentration was adjusted to 1×10⁷ cells/ml.

### II. Flow chamber cell adhesion experiment

### Mechanism:

In order to study the integrin-mediated cell adhesion function, the inventers established a flow chamber system *in vitro* to study the interaction of α4 integrin or β2 integrin with its ligands VCAM-1, MAdCAM-1 or ICAM-1, respectively (Figure 2). Cells entered in the inlet pipe, flowed through the flow chamber, and then flowed out from another pipe. A programmable pump was connected to this pipe to control the flow rate of the liquid. The integrin ligand was coated on this plastic petri dish. First, EDTA was used to chelate the metal ions in the solution, and then corresponding metal ions were added to the suspension containing the cells. The cells were sucked into the flow chamber with a wall shear stress of 1 dyn/cm². The experiment process is recorded in video for later data analysis.

### Steps:

(1) Coating ligand: draw a circle with a diameter of about 5mm in the center of the bottom of a clean plastic plate, drop 20 µl of 5 µg/ml VCAM-1, MAdCAM-1 or ICAM-1, and incubate it in a humid box at 37°C for 1h;
(2) Block the contaminant protein: wash the plate with coating buffer three times at the marked place (the surface of the ligand should always be covered with liquid), add 20µl of blocking buffer in the circle, and incubate at 37°C for 1 hour;
(3) Wash twice with HBS, then install the flow cell system and spread a layer of HBS on it, pump air out by a vacuum pump and prevent air leakage;
(4) Add 50µl of the processed cells to 450µl of buffer A, add 2µl of divalent cations, mix well, and detect in the flow chamber system (Figure 2). Start the pump program and the video recording program simultaneously to record the movement of the cells for analysis.

### 1.2.6 Chemokine induced transwell migration

The chemokine-induced transwell migration can simulate the process of integrin-mediated lymphocyte migration across vascular endothelial cells. Transwell chambers (5µm well size, Millipore) were coated with 5µg/ml VCAM-1, MAdCAM-1 or ICAM-1 respectively, incubated overnight at 4°C, and blocked with blocking buffer at 37°C for 1h. T cells were resuspended in serum-free RPMI 1640 culture medium at 2×10⁶ cells/ml, and 150µl of cells were added to the upper layer of Well. 600µl of RPMI 1640 culture medium comprising 500ng/ml CCL21 was added to the bottom layer of Well to ensure that the inside and outside liquid level of Well is even, and the Well was incubated at 37°C for 4h. By counting the cells that have migrated to the underneath of the well, the ability of cells to migrate across the membrane was relatively quantified.

### 1.2.7 Immunofluorescence staining of cell spreading and integrin clustering on the cell surface

(1) T cells were suspended in FBS-free RPMI 1640 culture medium, plated on a glass slide coated with PLL (100µg/ml), and incubated at 37°C for 10 minutes;
(2) Wash once with the incubated PBS, then fix with the incubated 2% paraformaldehyde/PBS (Sigma) for 15 min at room temperature;
(3) Block with PBS+10% FBS for 1 hour at room temperature;
(4) Primary antibody incubation (PS/2): 37°C for 2h or 4°C overnight, wash three times with 1% BSA+PBS, each for 5 minutes;
(5) Secondary antibody incubation (goat anti-rat-Cy3): 37°C for 1 hour, then wash with 1% BSA+PBS three times, each for 5 minutes;
(6) Stain cell nuclei with DAPI: incubate 5min at room temperature, then wash three times with PBS, mount the slide and take pictures.

### 1.2.8 Separation of nuclear and cytoplasmic membrane

(1) Collect T cells (1×10⁷), and wash once with TBS, centrifuge at 5000rpm for 3min;
(2) Resuspend the cells in 600µl buffer (TBS+protease inhibitor), and allow the cells to rest on ice for 10 minutes;
(3) Repeatedly blow and suck 30-40 times by the insulin needle to break the cells by mechanical force, and collect the whole cell lysate (WCL);
(4) Centrifuge at low speed, 2000rpm for 3min to remove nuclear components and incompletely broken cells;
(5) Transfer the supernatant to a new *Eppendorf* tube, 13000rpm, 30min;
(6) The supernatant was the cell cytoplasmic fraction and the precipitate was the cell membrane fraction. Lyse the cells with cell lysate on ice for 30 min;
(7) Centrifuge at 14000rpm for 30min. the supernatant is the soluble fraction of the cell membrane after lysis. Proceed with the following Co-IP or collect Input samples.

### 1.2.9 Western Blotting

Western blotting was used to detect the relative content of a specific protein in a cell lysate or purified sample. The protein content was determined by BCA method. The protein samples with the same amount were separated by 7-9% SDS-PAGE electrophoresis: concentrated gel 80V, 30min; separation gel 120V, 60min. Following the electrophoresis, the protein was transferred to the nitrocellulose membrane (NC) using a wet electroporator with a current of 280mA for 100min. After the transfer, the NC membrane was obtained with the surface comprising the transferred protein facing up. Incubating with blocking solution (TBST comprising 5% skimmed milk powder) at room temperature for 1 hour; incubating with specific antibody (diluted in TBST comprising 2% BSA) for 2 hours at room temperature or overnight at 4°C; washing NC membrane three times with TBST, each 5 minutes. Incubating with HRP-coupled secondary antibody (diluted in TBST) for 1h at room temperature. After incubation, washing the NC membrane twice with TBST for 5 minutes each time. Washing the NC membrane twice with TBS for 5 minutes each time. After fluorescent development with ECL developing solution, holding the NC film in the holder, and displaying the protein bands in darkness with the developing solution and the fixing solution respectively.

### 1.2.10 Co-immunoprecipitation

All steps of co-immunoprecipitation were performed on ice, and the used buffers such as TBS, Cell lysis buffer, and IP buffer needed to be pre-cooled on ice.
(1) Collect the cells in a 15ml centrifuge tube, centrifuge in a horizontal rotor at 1200rpm for 3min;
(2) Discard the supernatant, add 1ml TBS in the tube and transfer the mixture to a 1.5ml Eppendorf tube, centrifuge at 5000rpm for 3min;
(3) Discard the supernatant, add 600µl Cell lysis buffer and lyse on ice for 30 minutes with mixing during the lysis to avoid cell sedimentation;
(4) Centrifuge at 14000rpm for 30min, and the supernatant was used as Input (40µl) for Western blotting;
(5) Add the remaining supernatant in a new 1.5ml Eppendorf tube, add 50µl Protein G beads, and pre-wash at 4°C for 2h;
(6) Centrifuge at 2800rpm for 3min, transfer the supernatant into a new 1.5ml Eppendorf tube and add 1µg primary antibody, then incubate overnight at 4°C on a turnplate;
(7) Add 50µl Protein G beads and incubate at 4°C for 2h;
(8) Centrifuge at 2,800rpm for 3min, wash with IP buffer for three times;
(9) Remove the supernatant using the insulin needle, add 60µl 2×SDS loading buffer, and boil at 100°C for 10 minutes;
(10) Centrifuge at 14000rpm for 2min, transfer to a new 1.5ml Eppendorf tube by the insulin needle, and then detect the target protein by Western blotting.

### 1.2.11 Detection of protein interaction by Pull down

The integrin intracellular domain model protein obtained by *E.coli* prokaryotic expression and purification was mixed with the supernatant of T cell lysate and incubated for 2h at 4°C. Wash away non-specific binding protein with IP buffer, resuspend the beads by 2×protein-loading-buffer, then boil at 100°C for 10 minutes and collect the samples. The bands of the target protein were identified by Western blotting. In addition, the integrin intracellular domain model protein stained with Coomassie Blue was used as a loading control.

### 1.2.12 Detection of protein interaction by BiFC-Split GFP

In addition to traditional Pull down, Co-IP and other biochemical methods, protein-fragment complementation assay (PCA) can detect protein-protein interaction more convenient and intuitive, can be relatively quantitative, and are favored by scientists. PCA detects protein-protein interaction by dividing some enzyme protein (such as dihydrofolate reductase, β-galactosidase, β-lactamase, or luciferase) into two fragments, which are used as tags to fuse to target protein. PCA based on split green fluorescent protein (Split GFP) or its various mutants is called bimolecular fluorescence complementation (BiFC). Because the assembly of GFP elements is irreversible, Split GFP-based BiFC can be used to study transient protein-protein interactions and low-affinity complexes.

This disclosure used the Split GFP system to study the self-dimerization of α4 integrin. The intracellular domains of α4 subunits fused with two parts of GFP respectively, GFP 1-10 OPT and GFP S11 M3, were simultaneously expressed in cells. If the α4 integrin underwent self-dimerization, the two parts of the protein were close to each other, thereby forming a stable GFP fluorescent, which can be conveniently and directly observed by fluorescence microscope or FACS, and relative quantified. The cells expressing α4-Split GFP were subjected to fever-range thermal stress or Hsp90-overexpressing treatment. FACS could conveniently and intuitively detect the change of GFP fluorescence, which indicated the change of the self-dimerization of α4 integrin.

### 1.2.13 Detection of protein polymerization by Native-PAGE

Native-PAGE was used to assay the polymerized form of the target protein in cells, i.e., in its natural state. Native-PAGE mainly comprises:
(1) Collect the cell lysate, add 5× protein loading buffer (SDS free), place on ice without boiling at 100°C;
(2) Load the sample directly after high-speed centrifugation without adding protein Marker;
(3) The electrophoresis buffer was the transfer buffer (methanol free) used in ordinary SDS-PAGE, and the electrophoresis process was performed on ice to prevent degradation of the target protein;
(4) Membrane Transfer and the following Western blotting were the same as ordinary SDS-PAGE.

### 1.2.14 Detection of Rho GTPase activation

The key step of integrin-mediated migration of immune cells across the membrane is the rearrangement of cytoskeletal protein, which in turn changes the cell morphology, and finally the immune cells penetrate vascular endothelial cells into secondary lymphoid organs or inflammation sites. There are three main Rho GTPases that mediate the formation of cytoskeleton, namely Rac1, RhoA and Cdc42.

In order to detect the activity changes of the three GTPases, the inventors expressed and purified their respective GTP-binding effector protein *in vitro* (the effector protein of Rac1 and Cdc42 are both PAK-PBD, and the effector protein of RhoA is Rhotekin-RBD), each connected to agarose beads via GST tags and incubated with cell lysate. Thus, the activated GTPase (in the Rho-GTP form) can specifically bind to its corresponding effector-protein-beads in the lysate. The non-specifically bound protein was washed away by IP buffer, and finally each Rho GTPase antibody was detected by Western blotting. The brightness of the band reflected the degree of activation of the corresponding Rho GTPase.

### 1.2.15 Construction of Itga4^{R985A/R985A} knock-in mice by CRISPR/Cas9

The mice used for integrin α4-R985A knock-in were C57BL/6J mice. The knock-in mice were constructed by Shanghai Model Organisms Center, Inc. Specifically,

### (1) Requirement:

Mouse point mutation site: R1018A (corresponding to human site R985A), located in exon28.
Wild type sequence: AAC AGG AGA (-N--R--R-)
Mutated sequence: AAC AGG AGA (-N--A--R-)

### (2) Cas9 strategy:

Design guide RNAs for exon28 sequence.
Exon28 target sequence: (UTR is in bold, and non-bold sequence is the coding region) 
GCTGGATTCTTAAAAGACAGTACAAATCTATCCTACAAGAAGAAAACAGGA
GAGACAGCTGGAGTTATGTCAACAGCAAAAGCAATGATGACTGAAGACTTCTAC
ACTGAGAGAACTGAAAAACTCAGGTTAGGAAAAAGAAATCCTGTTCAGAAGAC
CCGTCAGAATTTCTTCTTTTT (SEQ ID NO:15)
GuideRNA:
   Guide #1: CTATCCTACAAGAAGAAAACAGG (SEQ ID NO:16)
Donor DNA sequence:

tttcagGCTGGATTCTTTAAAAGACAGTACAAATCTATCCTACAAGAAGAAAACG
CGAGAGACAGCTGGAGTTATGTCAACAGCAAAAGCAATGATGACTGAAGACTTCT
ACACTGAGA (SEQ ID NO:17)

### (3) CRISPR/Cas9 process to establish a genetic mutation mouse

Cas9 protein, guide-RNAs and template DNAs were injected into mouse fertilized eggs through microinjection. After homologous recombination, the target gene in the genomic DNA of the blastocyst cells had been replaced with a mutant gene. Finally, the embryos were transplanted into the surrogate mice to give birth to F0 generation mutant mice.

### (4) Mechanism

Guide-RNAs guide the endonuclease Cas9 to locate on a specific sequence of the genomic DNA in the cell, and then cause a DNA double-strand break in the target fragment. At the same time, the template DNA with sequence homologous (but containing a new gene or gene mutation) to the flanks of the original gene break were added. The mutation was introduced into the genomic DNA sequence through homologous recombination.

### B. Experimental results

### 2.1: Fever-range thermal stress promotes α4 integrin-mediated cell adhesion and transmigration

In order to study the effect of fever-range thermal stress on the adhesion and migration of lymphocytes, the inventor isolated T lymphocytes from the spleen of C57BL/6J mice, and treated them with physiological conditions (37°C) or fever-range thermal stress (40°C) for 12 hours. FACS found that fever-range thermal stress did not affect the expression of all α4 and β2 integrin subunits on the cell membrane surface (Figure 3, A). Then, the effects of fever-range thermal stress on cell adhesion and migration mediated by α4β1, α4β7 and β2 integrins on their respective ligands VCAM-1, MAdCAM-1 and ICAM-1 under the physiological condition of 1 mM Ca²⁺/Mg²⁺ were assayed. When VCAM-1 was used as a substrate protein, T cells were treated with α4β7 blocking antibody DATK32 in advance to block the binding of α4β7-VCAM-1, so that the function of α4β1-VCAM-1 can be studied exclusively. Compared with control cells, T cells pre-treated at 40°C significantly enhanced cell adhesion and migration abilities with VCAM-1 and MAdCAM-1 protein as the ligands; however, when ICAM-1 was used as the ligand, cells treated in advance at 37°C and 40°C showed similar adhesion and migration abilities (Figure 3, B and C). These results indicate that fever-range thermal stress specifically promotes α4 integrin-mediated adhesion and transmigration of lymphocytes.

### 2.2 Hsp90 protein specifically binds to α4 integrin and promotes cell adhesion and transmigration

When studying the related molecular mechanism, T cells were treated with fever-range thermal stress, and the expression of heat shock protein family members in the whole cell lysate was significantly up-regulated (Figure 4, A). Further, through Co-IP experiments, it was found that Hsp90 (including the two subtypes of Hsp90AA1 and Hsp90AB1) specifically bound to α4 integrin, with a significantly increased binding when the cells were pre-treated at 40°C. Meanwhile, Hsp40, Hsp60 and Hsp70 bind to both α4 and β2 integrins. These suggested that Hsp90 may be involved in fever-specific regulation of α4 integrin (Figure 4, A). Then, by overexpressing Hsp90 protein in T cells, their binding to α4 integrin was enhanced (Figure 4, B). The results showed that the overexpression of Hsp90 protein did significantly promote the cell adhesion and migration of T cells on VCAM-1 and MAdCAM-1, but did not affect the adhesion and migration of β2 integrin-mediated adhesion and migration on ICAM-1 (Figure 4, C and D). Therefore, fever-range thermal stress enhances the α4-integrin-mediated cell adhesion and transmigration by up-regulating the expression of Hsp90 protein.

### 2.3 Both the N-terminus and C-terminus of Hsp90 can bind to the cytoplasmic domain of α4 integrin

Because integrin is a α/β heterodimer, Hsp90 protein can bind to α4 integrin by interacting with α or β subunit. The inventors purified the intracellular domain model proteins of α4, β1, and β7 integrins, and then verified that Hsp90 binds to the intracellular segment of α4 integrin by pull down (Figure 5, A). Further, biochemical experiments identified that three amino acids R985, W989 and Y991 in "ENRRDSWSY" motif of the intracellular segment of α4 integrin are mainly responsible for the binding to Hsp90. Mutation of each of the 3 amino acids to A caused significant decreased binding of α4 integrin to Hsp90 (Figure 5, B and C, SEQ ID NO: 18-21). According to the structural characteristics of Hsp90 protein, Hsp90 mainly comprises three domains of N-terminal domain, middle domain and C-terminal domain (Figure 5, D). By expressing each domain of Hsp90 fused with a HA tag in T cells, the results of Co-IP showed that both the N-terminal domain and C-terminal domain of Hsp90 can specifically bind to α4 integrin (Figure 5, E). Further, GST-pull down found that the binding of the two domains of N-terminal and C-terminal of Hsp90 to the intracellular segment of α4 integrin is a direct protein-protein interaction (Figure 5, F).

### 2.4 Construction of integrin Itga4^{R985A/R985A} knock-in mice

In order to identify whether Hsp90 promotes α4-integrin-mediated cell adhesion and transmigration by binding to the intracellular segment of α4 integrin, *Itga4*^{R985A/R985A} knock-in mice (KI) was constructed by CRISPR/Cas9 gene editing which destroyed the Hsp90-α4 interaction *in vivo* (Figure 6, A). The R985A mutation was chosen because that it is the only one, among the three point-mutations that inhibit the binding of Hsp90 to α4 integrin, that does not affect the binding of paxillin (a known α4 integrin intracellular regulatory protein) to the intracellular segment of α4 integrin (Figure 6, B). In knock-in mice, the expression of α4 integrin did not change significantly (Figure 6, C). Co-IP confirmed that the R985A mutation can significantly inhibit the binding of Hsp90 to α4 integrin *in vivo* (Figure 6, D).

### 2.5 Disruption of Hsp90-α4 integrin binding inhibits fever-induced T cell adhesion and transmigration

As expected, R985A mutation of α4 integrin completely inhibited the fever-induced cell adhesion and transmigration of T cells on VCAM-1 and MAdCMA-1 ligands (Figure 7, A and B), which proves that Hsp90-α4-integrin binding is essential for the fever-induced adhesion and migration of T cells. Then, intravital microscopy was used to observe the adhesion process of lymphocytes in the inguinal lymph nodes of mice *in vivo.* T cells from WT and KI mice were pretreated at 37°C or 40°C for 12 hours, then labeled with fluorescein calcein and injected into WT mice through the right femoral artery. The results showed that WT T cells pre-treated at 40°C significantly increased the ratio of stable adhesion in grade IV and V blood vessels (Figure 7, C), and increased the homing of WT T cells to inguinal lymph nodes (Figure 7, D). However, stable adhesion and homing of KI T cells were not affected. Conversely, pre-treatment at 40°C also increased the transient adhesion and rolling adhesion of WT and KI T cells in grade IV and V blood vessels, which may be due to the enhanced function of T cell L-selectin by thermal treatment. Compared with WT T cells, KI T cells showed a lower rolling adhesion ability upon thermal treatment, suggesting that the enhanced function of α4 integrin promoted by fever may partly contribute to the rolling adhesion of T cells on the surface of HEVs.

### 2.6 Hsp90-α4 integrin binding induces activation of α4 integrin

In order to study whether α4 integrin is activated by Hsp90 upon fever-range thermal stress, the binding of T cell α4β1 and α4β7 integrins to soluble VCAM-1 and MAdCAM-1 was first detected. Being pre-treated at 40°C, WT T cells showed a significant increase in the binding to VCAM-1 and MAdCAM-1; on the contrary, the ligand binding of KI T cells did not change significantly. This confirmed that the ligand binding increased by fever depends on the binding of Hsp90-α4 integrin (Figure 8, A). Consistently, the binding to VCAM-1 and MAdCAM-1 was also enhanced by overexpression of Hsp90 in WT T cells (Figure 8, B). Further, FRET was used to study the conformational changes of the extracellular segment of α4 integrin, and the extension of the conformation indicated the activation of α4 integrin. Upon pre-treatment at 40°C, WT T cells showed significantly decreased FRET efficiency (Figure 8, C), indicating that the conformation of the extracellular segment of α4 integrin became more extended after fever treatment. No significant change in FRET efficiency was observed in KI T cells. Similarly, overexpression of Hsp90 also significantly reduced the FRET efficiency of WT T cells (Figure 8, D). The activation of integrins depends on the binding of signal proteins. Talin and kindlin-3 are the two most important adaptor proteins for integrins in cells, which mediate the inside-out activation of integrins by binding to the β subunit. Co-IP results showed that the high fever-range thermal stress and the overexpression of Hsp90 significantly enhanced the binding of talin and kindlin-3 to α4 integrin in WT T cells (Figure 8, E and F).

### 2.7 Knockdown of Talin and Kindlin-3 inhibits the activation of α4 integrin

In order to further confirm the role of talin and kindlin-3 in the fever-induced α4 integrin activation, the expression of talin and kindlin-3 in T cells were knocked down by shRNA-mediated gene silencing. Upon fever-range thermal stress, the binding of talin and kindlin-3 to α4 integrin did not increase significantly (Figure 9, A and B); correspondingly, knockdown of talin and kindlin-3 significantly inhibited the binding to VCAM-1 and MAdCAM-1 inducted by fever (Figure 9, C and D), and the reduction of FRET efficiency between the extracellular segment of α4 integrin and the cell membrane (Figure 9, E and F). These confirmed that the activation of α4 integrin by the fever-induced interaction of Hsp90 and α4 integrin can be achieved by enhancing the binding of talin and kindlin-3 to α4 integrin.

### 2.8 Hsp90-α4 integrin binding induces the dimerization and clustering of α4 integrin on the plasma membrane

Given that both the N-terminal and C-terminal domains of Hsp90 can directly bind to the intracellular segment of α4 integrin, it is conjectured that one Hsp90 molecule may bind to two α4 subunits simultaneously in the cells, which mediates the dimerization of α4 integrin on the cell membrane and further activates the downstream signaling pathways of integrins. To confirm this conjecture, a bimolecular fluorescence complementation system (BiFC) was established, wherein two complementary components of GFP: GFP S1-10 and GFP S11 were fused to the C segment of the intracellular segment of α4 integrin and expressed. The dimerization of α4 integrin can induce the approach of GFP S1-10 and GFP S11, which reconstructed to form a functional GFP protein (Figure 10, A). First, the endogenous expression of the α4 subunit in T cells was knocked down by shRNA, and the shRNA-resistant α4 subunits fused with GFP S1-10 and GFP S11, respectively, were co-expressed in the T cells to construct α4-integrin-Split-GFP cells. Upon the pre-treatment at 40°C, the GFP fluorescence level of cells expressing WT α4-integrin-Split-GFP increased significantly (Figure 10, B), indicating that fever promotes the dimerization of α4 integrin on the cell membrane. Correspondingly, after the pre-treatment at 40°C, cells expressing WT α4-integrin-Split-GFP showed obvious clustering of α4 integrin on the cell membrane surface (Figure 10, D). In contrast, the GFP fluorescence signal and membrane clustering of cells expressing α4 (R985A) integrin-Split-GFP were not induced by fever. Similarly, overexpression of Hsp90 protein in cells expressing WT α4-integrin-Split-GFP also increased the fluorescence level of GFP (Figure 10, C). These results indicate that Hsp90-α4 integrin interaction induces the dimerization and clustering of α4 integrin on the cell membrane surface after fever treatment.

To further study the molecular mechanism of Hsp90-mediated dimerization of α4 integrin, Hsp90 mutants lacking different domains were constructed (Figure 10, E), and separately expressed in WT α4-integrin-Split-GFP cells. Native-PAGE results showed that Hsp90AA1-WT and Hsp90AA1-MC existed as homodimers, while Hsp90AB1-WT and Hsp90AB1-MC existed mainly as monomers. Deletion of the C-terminal domain (Hsp90-NM) significantly inhibited the self-dimerization of Hsp90-AA1 and Hsp90AB1 (Figure 10, F). In cells expressing WT α4-integrin-Split-GFP, overexpression of Hsp90-WT significantly enhanced the GFP signal; however, overexpression of Hsp90-MC only showed a very weak GFP signal; overexpression of Hsp90-NM did not induce GFP signal at all (Figure 10, G). The weak GFP signal induced by Hsp90-MC may be mediated by the two C-terminal domains of the Hsp90-MC dimer. Therefore, the N-terminal and C-terminal domains of Hsp90 protein both play an important role in the effective dimerization of α4 integrin. Then, the Hsp90-NC5 mutant with 49 amino acids in the C-terminal domain deleted was constructed to inhibit the self-dimerization of Hsp90 (Figure 10, H). In cells expressing WT α4-integrin-Split-GFP, overexpression of Hsp90-WT and Hsp90-NC5 induced similar GFP signals (Figure 10, I), indicating that the monomeric form of Hsp90 can adequately mediate dimerization of α4 integrin. Therefore, one Hsp90 molecule can simultaneously bind to two subunits of α4 integrin through the N-terminal and C-terminal domains of Hsp90, and mediate the dimerization of α4 integrin on the cell membrane.

### 2.9 The binding of Hsp90-α4 integrin activates the FAK-RhoA GTPase signaling pathway

The clustering of integrins on the cell membrane surface can trigger the activation of downstream signaling pathways of integrins from the outside to the inside. FAK and Rho family GTPases (RhoA, Rac1 and Cdc42) are important signal proteins activated by integrins, which can promote cell migration by regulating the rearrangement of skeletal proteins. Therefore, the effect of fever-range thermal stress on the activation of FAK and Rho GTPases was tested. According to the results, the 40°C pre-treated WT T cells showed significantly increased phosphorylation of tyrosine at position 397 of FAK (Figure 11, A), and induced activation of RhoA (Figure 11, B), while Rac1 and Cdc42 were not activated. On the contrary, FAK and RhoA in KI T cells were not regulated by fever (Figure 11, C), thus confirming that the activation of FAK and RhoA induced by fever depends on the binding of Hsp90-α4 integrin. Furthermore, overexpression of Hsp90-WT in T cells also promoted the activation of FAK and RhoA; while overexpression of Hsp90-NM (which cannot induce the dimerization of α4 integrin, Figure 10, G) was not able to activate FAK and RhoA (Figure 11, D). In summary, the binding of Hsp90-α4 integrin induces the dimerization and clustering of α4 integrin on the cell membrane, and the following activation of FAK and RhoA GTPase.

### 2.10 Disruption of Hsp90-α4 interaction inhibits fever-induced T cell trafficking in vivo

To study the effect of the Hsp90-α4 integrin signal axis on the migration of lymphocytes *in vivo,* WT and KI mice were treated by normal temperature (core temperature 36.8±0.2°C) or fever-range whole-body hyperthermia (WBH; core temperature 39.5±0.5°C) for 6 hours, and T cells were separated from the mouse spleens. WT and KI mice showed similar expression levels of α4 integrin (Figure 12, A). Compared with the normal temperature group, the T cells of WBH treated WT mouse showed significantly enhanced binding of Hsp90 to α4 integrin; while in KI T cells, the binding of Hsp90 to α4 integrin was not detected (Figure 12, A). Consistently, mutation of α4 (R985A) completely blocked the fever-induced adhesion and transmigration of T cells on VCAM-1 and MAdCAM-1 (Figure 12, B and C). Then, the changes in the distribution of T cells in different lymphatic tissues of mice upon normal temperature or WBH treatment were assayed. In WT and KI mice treated by normal temperature, T cells were found distributed similarly in peripheral blood (peripheral lymph nodes, PLNs), mesenteric lymph nodes (MLNs), Peyer's patches (PPs), spleen and peripheral blood (PB), indicating that the binding of Hsp90-α4 integrin does not affect the homing of T cells to each lymph node under normal temperature. Upon WBH treatment, WT mouse T cells significantly accumulated in PLNs, MLNs and PPs, and correspondingly decreased significantly in PB (Figure 12, D). The distribution of T cells in spleen has hardly changed because spleen lacks the structure of HEVs. On the contrary, the distribution of KI mouse T cells in these lymphoid organs was significantly low (Figure 12, D), which proves that disruption of the binding of Hsp90 to α4 integrin inhibits the fever-induced homing of lymphocytes to these lymph nodes.

### 2.11 LPS-induced mild fever does not affect the homing of T cells in mice

Next, mouse models of LPS-induced fever were established. LPS (10µg/kg) or PBS was injected into the tail vein of WT and KI mice, and the rectal temperature of the mice was monitored every hour. Similar to the previous report, LPS can induce the body temperature of mice to rise to about 38°C for less than 6 hours (Figure 13, A). However, the mild fever in mice induced by LPS did not change the expression of Hsp90 in T cells and the distribution of T cells in various lymphatic organs (Figure 13, B and C). *In vitro* experiments also showed that only when T cells were treated at 38.5°C or higher, the expression of Hsp90 protein was significantly increased (Figure 13, D). Therefore, high temperature and fever are necessary for the expression of Hsp90 and the promotion of T cell migration.

### 2.12 Disruption of Hsp90-α4 interaction impairs the clearance of bacterial infection

In order to further study the role of the Hsp90-α4 integrin signal axis in the pathological process accompanied by high temperature and fever, *Salmonella typhimurium* infected mouse models were established. High-dose *Salmonella typhimurium* (SL1344) was injected by oral gavage to induce gastroenteritis and typhoid fever in mice. After injection of *Salmonella typhimurium,* WT and KI mice developed fever on the second day, and reached a maximum temperature of about 40°C on the 4th day (Figure 14, A). The survival curve clearly showed that *Salmonella typhimurium* infection caused a higher lethality rate in KI mice (Figure 14, B). Compared with WT mice, KI mice showed more serious intestinal tissue damage, a large amount of epithelial tissue structural destruction (Figure 14, C); and on the 5th day, the infection of bacteria in the small intestine were significantly increased (Figure 14, D). According to further detections, upon the fever caused by *Salmonella typhimurium* infection in mice, WT mice showed increased infiltration of T cells into the small intestine, PLNs and spleen, but reduced distribution in MLNs, PPs and PB (Figure 14, D and E). The decrease of T cells in MLNs and PPs, as well as the increase in spleen, is due to *Salmonella typhimurium* infection that destroys the structure of these lymphoid organs which directly affects the distribution of lymphocytes. In KI mice, disrupting the binding of Hsp90-α4 integrin significantly inhibited the distribution of fever-induced T cells in PLNs (Figure 14, E), and reduced the infiltration of T cells into the small intestine (Figure 14, D). B cells, as other important lymphocytes, are activated by bacterial antigens presented by antigen-presenting cells and can differentiate into mature plasma cells that migrate to the infection site and secrete specific antibodies to kill pathogenic bacteria. Similarly, disrupting the binding of Hsp90-α4 integrin also significantly inhibited the migration of fever-induced B cells to the bacterial infection site of small intestine (Figure 15). In summary, these results showed that the Hsp90-α4 integrin signal axis activated by fever promotes immune surveillance during inflammation and fever, and plays an important role in the migration of T cells to inflammatory tissues and the elimination of infections.

### 2.13 Disruption of Hsp90-α4 interaction inhibits monocyte recruitment to draining lymph nodes in mice during S. typhimurium infection

Since α4 integrin is also expressed on the surface of some innate immune cells (such as monocytes), it can be conjectured that monocyte migration is also regulated by the Hsp90-α4 signal axis induced by fever. When mice were injected with *S. typhimurium* by oral gavage for 3 days, the increase of monocytes that migrated to PLNs, MLNs and PPs in KI mice was significantly less than that in WT mice (Figure 16, A). This indicates that the Hsp90-α4 integrin interaction promotes the migration of monocytes to the draining lymph nodes during fever. However, the neutrophils that did not express α4 integrin showed similar migration to PLNs, MLNs and PPs in WT and KI mice (Figure 16, B). Therefore, the Hsp90-α4 integrin signaling pathway can promote the migration of innate immune cells and adaptive immune cells expressing α4 integrin during fever, thereby promoting the elimination of pathogenic bacterial infections.

### 2.14 Fever promotes T cell trafficking via a thermal sensory Hsp90-α4 integrin pathway

During fever, thermal stress up-regulates the expression of heat shock protein Hsp90 in immune cells. Hsp90 can specifically bind to α4 integrin and activate α4 integrin through inside-out signaling pathways. Moreover, the N-terminal and C-terminal domains of one Hsp90 molecule can directly bind to the intracellular regions of two α4 integrins simultaneously, thereby promoting the dimerization and clustering of α4 integrin on the cell surface and activating downstream FAK-RhoA pathway to regulate cytoskeleton rearrangement and promote immune cell migration (Figure 17). In summary, this disclosure identified the Hsp90-α4 integrin signal axis as a sensitive heat-sensitive pathway that can promote immune cell migration, enhance immune surveillance and maintain immune homeostasis during pathogen infection. Based on the disclosure, regulating the directional migration of immune cells by changing the temperature or the expression of Hsp90 in cells will provide new strategies for the treatment of infection, inflammation or tumor.

ÐòÁÐ±í

<110>  ÖÐ¹ú¿ÆÑ§Ôº£ÉÏº£ÉúÃü¿ÆÑ§ÑÐ¾¿Ôº

<120>  µ÷¿ØÃâÒßÏ¸°ûÇ¨ÒÆµÄ×é°ÏÎï°Í•½•¨

<130>  187532

<150>  201811378994.5
<151>  2018-11-19

<160>  21

<170>  SIPOSequenceListing 1.0

<210>  1
<211>  733
<212>  PRT
<213>  Êó£¨Mus musculus£©

<400>  1
Met Pro Glu Glu Thr Gln Thr Gln Asp Gln Pro Met Glu Glu Glu Glu
1               5                   10                  15
Val Glu Thr Phe Ala Phe Gln Ala Glu Ile Ala Gln Leu Met Ser Leu
            20                  25                  30
Ile Ile Asn Thr Phe Tyr Ser Asn Lys Glu Ile Phe Leu Arg Glu Leu
        35                  40                  45
Ile Ser Asn Ser Ser Asp Ala Leu Asp Lys Ile Arg Tyr Glu Ser Leu
    50                  55                  60
Thr Asp Pro Ser Lys Leu Asp Ser Gly Lys Glu Leu His Ile Asn Leu
65                  70                  75                  80
Ile Pro Ser Lys Gln Asp Arg Thr Leu Thr Ile Val Asp Thr Gly Ile
                85                  90                  95
Gly Met Thr Lys Ala Asp Leu Ile Asn Asn Leu Gly Thr Ile Ala Lys
            100                 105                 110
Ser Gly Thr Lys Ala Phe Met Glu Ala Leu Gln Ala Gly Ala Asp Ile
        115                 120                 125
Ser Met Ile Gly Gln Phe Gly Val Gly Phe Tyr Ser Ala Tyr Leu Val
    130                 135                 140
Ala Glu Lys Val Thr Val Ile Thr Lys His Asn Asp Asp Glu Gln Tyr
145                 150                 155                 160
Ala Trp Glu Ser Ser Ala Gly Gly Ser Phe Thr Val Arg Thr Asp Thr
                165                 170                 175
Gly Glu Pro Met Gly Arg Gly Thr Lys Val Ile Leu His Leu Lys Glu
            180                 185                 190
Asp Gln Thr Glu Tyr Leu Glu Glu Arg Arg Ile Lys Glu Ile Val Lys
        195                 200                 205
Lys His Ser Gln Phe Ile Gly Tyr Pro Ile Thr Leu Phe Val Glu Lys
    210                 215                 220
Glu Arg Asp Lys Glu Val Ser Asp Asp Glu Ala Glu Glu Lys Glu Glu
225                 230                 235                 240
Lys Glu Glu Glu Lys Glu Lys Glu Glu Lys Glu Ser Asp Asp Lys Pro
                245                 250                 255
Glu Ile Glu Asp Val Gly Ser Asp Glu Glu Glu Glu Glu Lys Lys Asp
            260                 265                 270
Gly Asp Lys Lys Lys Lys Lys Lys Ile Lys Glu Lys Tyr Ile Asp Gln
        275                 280                 285
Glu Glu Leu Asn Lys Thr Lys Pro Ile Trp Thr Arg Asn Pro Asp Asp
    290                 295                 300
Ile Thr Asn Glu Glu Tyr Gly Glu Phe Tyr Lys Ser Leu Thr Asn Asp
305                 310                 315                 320
Trp Glu Glu His Leu Ala Val Lys His Phe Ser Val Glu Gly Gln Leu
                325                 330                 335

Glu Phe Arg Ala Leu Leu Phe Val Pro Arg Arg Ala Pro Phe Asp Leu
            340                 345                 350
Phe Glu Asn Arg Lys Lys Lys Asn Asn Ile Lys Leu Tyr Val Arg Arg
        355                 360                 365
Val Phe Ile Met Asp Asn Cys Glu Glu Leu Ile Pro Glu Tyr Leu Asn
    370                 375                 380
Phe Ile Arg Gly Val Val Asp Ser Glu Asp Leu Pro Leu Asn Ile Ser
385                 390                 395                 400
Arg Glu Met Leu Gln Gln Ser Lys Ile Leu Lys Val Ile Arg Lys Asn
                405                 410                 415
Leu Val Lys Lys Cys Leu Glu Leu Phe Thr Glu Leu Ala Glu Asp Lys
            420                 425                 430
Glu Asn Tyr Lys Lys Phe Tyr Glu Gln Phe Ser Lys Asn Ile Lys Leu
        435                 440                 445
Gly Ile His Glu Asp Ser Gln Asn Arg Lys Lys Leu Ser Glu Leu Leu
    450                 455                 460
Arg Tyr Tyr Thr Ser Ala Ser Gly Asp Glu Met Val Ser Leu Lys Asp
465                 470                 475                 480
Tyr Cys Thr Arg Met Lys Glu Asn Gln Lys His Ile Tyr Phe Ile Thr
                485                 490                 495
Gly Glu Thr Lys Asp Gln Val Ala Asn Ser Ala Phe Val Glu Arg Leu
            500                 505                 510
Arg Lys His Gly Leu Glu Val Ile Tyr Met Ile Glu Pro Ile Asp Glu
        515                 520                 525
Tyr Cys Val Gln Gln Leu Lys Glu Phe Glu Gly Lys Thr Leu Val Ser
    530                 535                 540
Val Thr Lys Glu Gly Leu Glu Leu Pro Glu Asp Glu Glu Glu Lys Lys
545                 550                 555                 560
Lys Gln Glu Glu Lys Lys Thr Lys Phe Glu Asn Leu Cys Lys Ile Met
                565                 570                 575
Lys Asp Ile Leu Glu Lys Lys Val Glu Lys Val Val Val Ser Asn Arg
            580                 585                 590
Leu Val Thr Ser Pro Cys Cys Ile Val Thr Ser Thr Tyr Gly Trp Thr
        595                 600                 605
Ala Asn Met Glu Arg Ile Met Lys Ala Gln Ala Leu Arg Asp Asn Ser
    610                 615                 620
Thr Met Gly Tyr Met Ala Ala Lys Lys His Leu Glu Ile Asn Pro Asp
625                 630                 635                 640
His Ser Ile Ile Glu Thr Leu Arg Gln Lys Ala Glu Ala Asp Lys Asn
                645                 650                 655
Asp Lys Ser Val Lys Asp Leu Val Ile Leu Leu Tyr Glu Thr Ala Leu
            660                 665                 670
Leu Ser Ser Gly Phe Ser Leu Glu Asp Pro Gln Thr His Ala Asn Arg
        675                 680                 685
Ile Tyr Arg Met Ile Lys Leu Gly Leu Gly Ile Asp Glu Asp Asp Pro
    690                 695                 700
Thr Val Asp Asp Thr Ser Ala Ala Val Thr Glu Glu Met Pro Pro Leu
705                 710                 715                 720
Glu Gly Asp Asp Asp Thr Ser Arg Met Glu Glu Val Asp
                725                 730

<210>  2
<211>  2202
<212>  DNA
<213>  Êó£¨Mus musculus£©

<400>  2
atgcctgagg aaacccagac ccaagaccaa ccaatggagg aggaggaggt cgagaccttt      60
gcctttcagg cagaaattgc ccagttaatg tccttgatca tcaatacctt ctactcgaac     120
aaagagatct ttctgaggga gctcatctcc aattcatcgg acgctctgga taaaatccgt     180
tacgagagcc tgacggaccc cagtaaactg gactcgggga aggagctgca catcaatctc     240
attcccagca aacaggaccg aaccctgacc attgtggata ccgggattgg aatgaccaag     300
gccgacttga tcaataacct tggcaccatt gccaagtcgg gcaccaaagc cttcatggag     360

gctttgcagg ctggtgcaga tatctctatg attggccagt ttggtgttgg tttttactct 420
gcctatttgg ttgctgagaa agtgactgtc atcacgaagc ataacgacga tgagcagtat 480
gcctgggagt cctcagctgg gggatccttc acagtgagga ctgacacagg tgaaccaatg 540
ggtcgtggaa caaaggttat cttgcatctg aaagaagacc aaacagagta tttggaggaa 600
aggagaataa aggagatcgt gaagaagcat tctcagttca ttggctatcc cattactctc 660
tttgtggaga aggaacgaga taaggaagtc agtgatgatg aggctgaaga aaaggaagag 720
aaagaggaag agaaagaaaa agaagaaaag gagtctgatg ataaacctga aatagaagat 780
gttggctctg atgaagaaga ggaggagaag aaggatggtg acaagaagaa aaagaagaag 840
ataaaggaaa agtacattga tcaagaagaa ctcaacaaaa caaagccgat ttggacgaga 900
aatcctgatg acatcactaa tgaggaatat ggagagttct acaagagctt aactaacgat 960
tgggaagaac acttggcagt aaagcatttt tctgttgaag gacaattaga attccgggcc 1020
cttctttttg tcccaagacg cgctcctttt gatctgtttg aaaacagaaa gaaaaagaac 1080
aacatcaagt tgtatgttcg cagagttttt atcatggata actgtgagga attaatccct 1140
gagtatctga atttcattag aggggtagtg gattctgagg atctccctct aaatatttcc 1200
cgtgaaatgc tgcaacaaag taaaattctg aaagttatca gaaagaattt ggtcaagaaa 1260
tgcttagaac tatttactga actagcagaa gataaagaga actacaaaaa gttttatgag 1320
cagttctcaa aaaatataaa gcttggaatt cacgaggact ctcagaatcg gaagaagctt 1380
tcagagctgt tgcggtacta cacatctgct tctggggacg agatggtttc tctgaaggac 1440
tactgtacca gaatgaagga aaaccagaag cacatctatt ttatcacagg tgagaccaag 1500
gaccaggttg ctaactccgc ctttgtggaa cgtctccgaa agcatggctt agaagtaatt 1560
tatatgattg agcccattga tgagtattgt gtgcaacagc tgaaggaatt tgagggcaag 1620
accttggtgt ctgttaccaa agaaggactg gaacttccag aagatgaaga ggaaaagaag 1680
aaacaggaag agaaaaagac aaaatttgag aacctctgca aaattatgaa agatattttg 1740
gagaagaagg ttgaaaaggt ggttgtgtca aaccgactgg tgacatcccc gtgctgtatt 1800
gtcacaagca catatgggtg gacagcaaac atggagagaa tcatgaaagc tcaagccctc 1860
agagacaact caacaatggg ttacatggca gcaaagaaac acctggagat aaatcctgat 1920
cactccatta ttgaaacctt aaggcaaaag gcagaggctg acaagaatga caaatctgtg 1980
aaggatctgg tcatcttgct gtatgaaact gcactcctat cttctggctt cagtctggaa 2040
gatccccaga cccatgctaa caggatctac aggatgatca agcttggtct aggtattgat 2100
gaggatgatc ctactgtgga tgacaccagt gctgctgtaa ctgaagaaat gcctcccctg 2160
gaaggagatg acgacacatc acgcatggaa gaagtagact aa 2202

<210> 3
<211> 724
<212> PRT
<213> Êó£¨Mus musculus£©

<400> 3
Met Pro Glu Glu Val His His Gly Glu Glu Glu Val Glu Thr Phe Ala
1               5                   10                  15
Phe Gln Ala Glu Ile Ala Gln Leu Met Ser Leu Ile Ile Asn Thr Phe
            20                  25                  30
Tyr Ser Asn Lys Glu Ile Phe Leu Arg Glu Leu Ile Ser Asn Ala Ser
        35                  40                  45
Asp Ala Leu Asp Lys Ile Arg Tyr Glu Ser Leu Thr Asp Pro Ser Lys
    50                  55                  60
Leu Asp Ser Gly Lys Glu Leu Lys Ile Asp Ile Ile Pro Asn Pro Gln
65                  70                  75                  80
Glu Arg Thr Leu Thr Leu Val Asp Thr Gly Ile Gly Met Thr Lys Ala
                85                  90                  95
Asp Leu Ile Asn Asn Leu Gly Thr Ile Ala Lys Ser Gly Thr Lys Ala
            100                 105                 110
Phe Met Glu Ala Leu Gln Ala Gly Ala Asp Ile Ser Met Ile Gly Gln
        115                 120                 125
Phe Gly Val Gly Phe Tyr Ser Ala Tyr Leu Val Ala Glu Lys Val Val
    130                 135                 140
Val Ile Thr Lys His Asn Asp Asp Glu Gln Tyr Ala Trp Glu Ser Ser
145                 150                 155                 160
Ala Gly Gly Ser Phe Thr Val Arg Ala Asp His Gly Glu Pro Ile Gly
                165                 170                 175
Arg Gly Thr Lys Val Ile Leu His Leu Lys Glu Asp Gln Thr Glu Tyr
            180                 185                 190
Leu Glu Glu Arg Arg Val Lys Glu Val Val Lys Lys His Ser Gln Phe

195                 200                 205
Ile Gly Tyr Pro Ile Thr Leu Tyr Leu Glu Lys Glu Arg Glu Lys Glu
    210                 215                 220
Ile Ser Asp Asp Glu Ala Glu Glu Glu Lys Gly Glu Lys Glu Glu Glu
225                 230                 235                 240
Asp Lys Glu Asp Glu Glu Lys Pro Lys Ile Glu Asp Val Gly Ser Asp
                245                 250                 255
Glu Glu Asp Asp Ser Gly Lys Asp Lys Lys Lys Lys Thr Lys Lys Ile
            260                 265                 270
Lys Glu Lys Tyr Ile Asp Gln Glu Glu Leu Asn Lys Thr Lys Pro Ile
        275                 280                 285
Trp Thr Arg Asn Pro Asp Asp Ile Thr Gln Glu Glu Tyr Gly Glu Phe
    290                 295                 300
Tyr Lys Ser Leu Thr Asn Asp Trp Glu Asp His Leu Ala Val Lys His
305                 310                 315                 320
Phe Ser Val Glu Gly Gln Leu Glu Phe Arg Ala Leu Leu Phe Ile Pro
                325                 330                 335
Arg Arg Ala Pro Phe Asp Leu Phe Glu Asn Lys Lys Lys Lys Asn Asn
            340                 345                 350
Ile Lys Leu Tyr Val Arg Arg Val Phe Ile Met Asp Ser Cys Asp Glu
        355                 360                 365
Leu Ile Pro Glu Tyr Leu Asn Phe Ile Arg Gly Val Val Asp Ser Glu
    370                 375                 380
Asp Leu Pro Leu Asn Ile Ser Arg Glu Met Leu Gln Gln Ser Lys Ile
385                 390                 395                 400
Leu Lys Val Ile Arg Lys Asn Ile Val Lys Lys Cys Leu Glu Leu Phe
                405                 410                 415
Ser Glu Leu Ala Glu Asp Lys Glu Asn Tyr Lys Lys Phe Tyr Glu Ala
            420                 425                 430
Phe Ser Lys Asn Leu Lys Leu Gly Ile His Glu Asp Ser Thr Asn Arg
        435                 440                 445
Arg Arg Leu Ser Glu Leu Leu Arg Tyr His Thr Ser Gln Ser Gly Asp
    450                 455                 460
Glu Met Thr Ser Leu Ser Glu Tyr Val Ser Arg Met Lys Glu Thr Gln
465                 470                 475                 480
Lys Ser Ile Tyr Tyr Ile Thr Gly Glu Ser Lys Glu Gln Val Ala Asn
                485                 490                 495
Ser Ala Phe Val Glu Arg Val Arg Lys Arg Gly Phe Glu Val Val Tyr
            500                 505                 510
Met Thr Glu Pro Ile Asp Glu Tyr Cys Val Gln Gln Leu Lys Glu Phe
        515                 520                 525
Asp Gly Lys Ser Leu Val Ser Val Thr Lys Glu Gly Leu Glu Leu Pro
    530                 535                 540
Glu Asp Glu Glu Glu Lys Lys Lys Met Glu Glu Ser Lys Ala Lys Phe
545                 550                 555                 560
Glu Asn Leu Cys Lys Leu Met Lys Glu Ile Leu Asp Lys Lys Val Glu
                565                 570                 575
Lys Val Thr Ile Ser Asn Arg Leu Val Ser Ser Pro Cys Cys Ile Val
            580                 585                 590
Thr Ser Thr Tyr Gly Trp Thr Ala Asn Met Glu Arg Ile Met Lys Ala
        595                 600                 605
Gln Ala Leu Arg Asp Asn Ser Thr Met Gly Tyr Met Met Ala Lys Lys
    610                 615                 620
His Leu Glu Ile Asn Pro Asp His Pro Ile Val Glu Thr Leu Arg Gln
625                 630                 635                 640
Lys Ala Glu Ala Asp Lys Asn Asp Lys Ala Val Lys Asp Leu Val Val
                645                 650                 655
Leu Leu Phe Glu Thr Ala Leu Leu Ser Ser Gly Phe Ser Leu Glu Asp
            660                 665                 670
Pro Gln Thr His Ser Asn Arg Ile Tyr Arg Met Ile Lys Leu Gly Leu
        675                 680                 685
Gly Ile Asp Glu Asp Glu Val Thr Ala Glu Glu Pro Ser Ala Ala Val
    690                 695                 700

Pro Asp Glu Ile Pro Pro Leu Glu Gly Asp Glu Asp Ala Ser Arg Met
705                 710                 715                 720
Glu Glu Val Asp

<210>  4
<211>  2175
<212>  DNA
<213>  Êó£¨Mus musculus£©

<400>  4
atgcctgagg aagtgcacca tggagaggag gaggtggaga cctttgcctt tcaggcagaa      60
attgcccagc tcatgtccct catcatcaac actttctatt caaacaagga gattttcctc     120
cgcgagttga tctctaatgc ttcagatgcc ctggacaaga ttcgatatga gagcctgacg     180
gacccttcta agttggacag tgggaaagag ctgaaaattg acatcatccc caaccctcag     240
gagcgcacgc tgactttggt ggacacaggc attggcatga ccaaggctga cctcattaat     300
aacctgggaa ccattgctaa gtctggcacg aaggcgttca tggaggctct ccaggctggt     360
gcagacatct ccatgatcgg gcagtttggt gtcggattct actcggccta tctagttgca     420
gagaaagtgg ttgtgatcac gaagcacaat gatgatgagc agtatgcctg ggagtcgtct     480
gcgggtggct ccttcaccgt ccgggcagac catggtgagc ccattggccg gggtaccaaa     540
gtgatccttc acctcaaaga agaccagaca gagtacttgg aggagaggag ggtcaaggaa     600
gtggtgaaga aacattcgca gttcataggc tatcccatca ccctctattt ggagaaggaa     660
cgggagaagg agatcagtga tgatgaggca gaggaagaga aaggtgagaa agaggaggaa     720
gataaggagg atgaggagaa gcctaagatt gaagatgtgg gatccgatga ggaagatgac     780
agcggcaaag acaagaaaaa gaaaacaaag aagatcaaag agaagtacat tgaccaggag     840
gagctgaaca agacaaagcc tatctggacc agaaacccgg atgacatcac gcaggaggag     900
tatggcgaat tctataagag cctcaccaat gactgggagg accacttggc agtcaagcac     960
ttctctgtag aaggtcagtt ggaattcagg gcattgctct tcattccccg gcgggctccc    1020
ttcgaccttt ttgagaacaa gaagaagaag aacaacatca aattgtatgt ccgccgtgtg    1080
ttcatcatgg acagctgtga cgagctgata cctgagtacc tcaactttat ccgcggtgtg    1140
gttgactccg aggacctgcc cctgaacatc tcccgggaga tgctgcagca gagcaagatc    1200
ctgaaggtca tccgcaagaa catcgtcaag aagtgcctgg agctcttctc cgagctggct    1260
gaggacaagg agaactacaa gaagttctat gaggccttct ccaaaaattt aaagcttgga    1320
attcatgaag attccactaa ccgacgccgc ctctctgagc tccttcgcta tcacacctct    1380
cagtctggag atgagatgac ctccttgtca gagtatgtgt ctcgcatgaa ggagacccag    1440
aagtccatct actatatcac tggtgagagc aaagagcaag tggccaactc tgcctttgtg    1500
gagcgagtgc ggaagcgggg cttcgaggtg gtgtatatga ctgagcctat tgacgagtac    1560
tgcgtgcagc agctcaagga gtttgatggg aagagcctgg tctcagtgac taaggagggc    1620
ctggagctac cagaggacga ggaagagaag aagaaaatgg aggagagcaa ggcaaagttt    1680
gagaatctct gcaagctcat gaaggagatc ttggacaaga aggttgaaaa ggtgacaatc    1740
tccaataggc ttgtgtcttc accctgctgc attgtgacaa gcacctatgg ctggacagcc    1800
aacatggaac ggatcatgaa ggcccaggca ctgcgagaca actctacaat gggctacatg    1860
atggccaaaa aacacctgga gatcaaccct gaccacccca tcgtggagac cctgcggcag    1920
aaggctgagg cagacaaaaa cgacaaagct gtcaaggacc tggtggtgct gctgtttgaa    1980
actgctctgc tctcctctgg tttctcactt gaggatcccc aaacccactc caaccgcatc    2040
taccgcatga ttaaactagg cctgggcatc gatgaagatg aggtcactgc agaggagccc    2100
agtgctgctg ttcctgatga gatcccccct ctggaaggcg atgaggatgc ctcgcgcatg    2160
gaagaggtgg attaa                                                     2175

<210>  5
<211>  732
<212>  PRT
<213>  ÖÇÈË(Homo sapiens)

<400>  5
   Met Pro Glu Glu Thr Gln Thr Gln Asp Gln Pro Met Glu Glu Glu Glu
   1               5                   10                  15
   Val Glu Thr Phe Ala Phe Gln Ala Glu Ile Ala Gln Leu Met Ser Leu
               20                  25                  30
   Ile Ile Asn Thr Phe Tyr Ser Asn Lys Glu Ile Phe Leu Arg Glu Leu
           35                  40                  45
   Ile Ser Asn Ser Ser Asp Ala Leu Asp Lys Ile Arg Tyr Glu Ser Leu
       50                  55                  60
   Thr Asp Pro Ser Lys Leu Asp Ser Gly Lys Glu Leu His Ile Asn Leu
   65                  70                  75                  80
   Ile Pro Asn Lys Gln Asp Arg Thr Leu Thr Ile Val Asp Thr Gly Ile
                   85                  90                  95
   Gly Met Thr Lys Ala Asp Leu Ile Asn Asn Leu Gly Thr Ile Ala Lys
               100                 105                 110
   Ser Gly Thr Lys Ala Phe Met Glu Ala Leu Gln Ala Gly Ala Asp Ile
           115                 120                 125
   Ser Met Ile Gly Gln Phe Gly Val Gly Phe Tyr Ser Ala Tyr Leu Val
       130                 135                 140
   Ala Glu Lys Val Thr Val Ile Thr Lys His Asn Asp Asp Glu Gln Tyr
   145                 150                 155                 160
   Ala Trp Glu Ser Ser Ala Gly Gly Ser Phe Thr Val Arg Thr Asp Thr
                   165                 170                 175
   Gly Glu Pro Met Gly Arg Gly Thr Lys Val Ile Leu His Leu Lys Glu
               180                 185                 190
   Asp Gln Thr Glu Tyr Leu Glu Glu Arg Arg Ile Lys Glu Ile Val Lys
           195                 200                 205
   Lys His Ser Gln Phe Ile Gly Tyr Pro Ile Thr Leu Phe Val Glu Lys
       210                 215                 220
   Glu Arg Asp Lys Glu Val Ser Asp Asp Glu Ala Glu Glu Lys Glu Asp
   225                 230                 235                 240
   Lys Glu Glu Glu Lys Glu Lys Glu Glu Lys Glu Ser Glu Asp Lys Pro
                   245                 250                 255
   Glu Ile Glu Asp Val Gly Ser Asp Glu Glu Glu Glu Lys Lys Asp Gly
               260                 265                 270
   Asp Lys Lys Lys Lys Lys Lys Ile Lys Glu Lys Tyr Ile Asp Gln Glu
           275                 280                 285
   Glu Leu Asn Lys Thr Lys Pro Ile Trp Thr Arg Asn Pro Asp Asp Ile
       290                 295                 300
   Thr Asn Glu Glu Tyr Gly Glu Phe Tyr Lys Ser Leu Thr Asn Asp Trp
   305                 310                 315                 320
   Glu Asp His Leu Ala Val Lys His Phe Ser Val Glu Gly Gln Leu Glu
                   325                 330                 335
   Phe Arg Ala Leu Leu Phe Val Pro Arg Arg Ala Pro Phe Asp Leu Phe
               340                 345                 350
   Glu Asn Arg Lys Lys Lys Asn Asn Ile Lys Leu Tyr Val Arg Arg Val
           355                 360                 365
   Phe Ile Met Asp Asn Cys Glu Glu Leu Ile Pro Glu Tyr Leu Asn Phe
       370                 375                 380
   Ile Arg Gly Val Val Asp Ser Glu Asp Leu Pro Leu Asn Ile Ser Arg
   385                 390                 395                 400
   Glu Met Leu Gln Gln Ser Lys Ile Leu Lys Val Ile Arg Lys Asn Leu
                   405                 410                 415
   Val Lys Lys Cys Leu Glu Leu Phe Thr Glu Leu Ala Glu Asp Lys Glu
               420                 425                 430
   Asn Tyr Lys Lys Phe Tyr Glu Gln Phe Ser Lys Asn Ile Lys Leu Gly
           435                 440                 445
   Ile His Glu Asp Ser Gln Asn Arg Lys Lys Leu Ser Glu Leu Leu Arg
       450                 455                 460
   Tyr Tyr Thr Ser Ala Ser Gly Asp Glu Met Val Ser Leu Lys Asp Tyr
   465                 470                 475                 480
   Cys Thr Arg Met Lys Glu Asn Gln Lys His Ile Tyr Tyr Ile Thr Gly
                   485                 490                 495
   Glu Thr Lys Asp Gln Val Ala Asn Ser Ala Phe Val Glu Arg Leu Arg
               500                 505                 510
   Lys His Gly Leu Glu Val Ile Tyr Met Ile Glu Pro Ile Asp Glu Tyr
           515                 520                 525
   Cys Val Gln Gln Leu Lys Glu Phe Glu Gly Lys Thr Leu Val Ser Val
       530                 535                 540
   Thr Lys Glu Gly Leu Glu Leu Pro Glu Asp Glu Glu Glu Lys Lys Lys
   545                 550                 555                 560
   Gln Glu Glu Lys Lys Thr Lys Phe Glu Asn Leu Cys Lys Ile Met Lys
   565 570 575
   Asp Ile Leu Glu Lys Lys Val Glu Lys Val Val Val Ser Asn Arg Leu
   580 585 590
   Val Thr Ser Pro Cys Cys Ile Val Thr Ser Thr Tyr Gly Trp Thr Ala
   595 600 605
   Asn Met Glu Arg Ile Met Lys Ala Gln Ala Leu Arg Asp Asn Ser Thr
   610 615 620
   Met Gly Tyr Met Ala Ala Lys Lys His Leu Glu Ile Asn Pro Asp His
   625 630 635 640
   Ser Ile Ile Glu Thr Leu Arg Gln Lys Ala Glu Ala Asp Lys Asn Asp
   645 650 655
   Lys Ser Val Lys Asp Leu Val Ile Leu Leu Tyr Glu Thr Ala Leu Leu
   660 665 670
   Ser Ser Gly Phe Ser Leu Glu Asp Pro Gln Thr His Ala Asn Arg Ile
   675 680 685
   Tyr Arg Met Ile Lys Leu Gly Leu Gly Ile Asp Glu Asp Asp Pro Thr
   690 695 700
   Ala Asp Asp Thr Ser Ala Ala Val Thr Glu Glu Met Pro Pro Leu Glu
   705 710 715 720
   Gly Asp Asp Asp Thr Ser Arg Met Glu Glu Val Asp
   725 730

<210> 6
<211> 2199
<212> DNA
<213> ÖÇÈË(Homo sapiens)

<400> 6
atgcctgagg aaacccagac ccaagaccaa ccgatggagg aggaggaggt tgagacgttc 60
gcctttcagg cagaaattgc ccagttgatg tcattgatca tcaatacttt ctactcgaac 120
aaagagatct ttctgagaga gctcatttca aattcatcag atgcattgga caaaatccgg 180
tatgaaagct tgacagatcc cagtaaatta gactctggga aagagctgca tattaacctt 240
ataccgaaca aacaagatcg aactctcact attgtggata ctggaattgg aatgaccaag 300
gctgacttga tcaataacct tggtactatc gccaagtctg ggaccaaagc gttcatggaa 360
gctttgcagg ctggtgcaga tatctctatg attggccagt tcggtgttgg tttttattct 420
gcttatttgg ttgctgagaa agtaactgtg atcaccaaac ataacgatga tgagcagtac 480
gcttgggagt cctcagcagg gggatcattc acagtgagga cagacacagg tgaacctatg 540
ggtcgtggaa caaaagttat cctacacctg aaagaagacc aaactgagta cttggaggaa 600
cgaagaataa aggagattgt gaagaaacat tctcagttta ttggatatcc cattactctt 660
tttgtggaga aggaacgtga taaagaagta agcgatgatg aggctgaaga aaaggaagac 720
aaagaagaag aaaaagaaaa agaagagaaa gagtcggaag acaaacctga aattgaagat 780
gttggttctg atgaggaaga agaaaagaag gatggtgaca agaagaagaa gaagaagatt 840
aaggaaaagt acatcgatca agaagagctc aacaaaacaa agcccatctg gaccagaaat 900
cccgacgata ttactaatga ggagtacgga gaattctata agagcttgac caatgactgg 960
gaagatcact tggcagtgaa gcatttttca gttgaaggac agttggaatt cagagccctt 1020
ctatttgtcc cacgacgtgc tccttttgat ctgtttgaaa acagaaagaa aaagaacaac 1080
atcaaattgt atgtacgcag agttttcatc atggataact gtgaggagct aatccctgaa 1140
tatctgaact tcattagagg ggtggtagac tcggaggatc tccctctaaa catatcccgt 1200
gagatgttgc aacaaagcaa aattttgaaa gttatcagga agaatttggt caaaaaatgc 1260
ttagaactct ttactgaact ggcggaagat aaagagaact acaagaaatt ctatgagcag 1320
ttctctaaaa acataaagct tggaatacac gaagactctc aaaatcggaa gaagctttca 1380
gagctgttaa ggtactacac atctgcctct ggtgatgaga tggtttctct caaggactac 1440
tgcaccagaa tgaaggagaa ccagaaacat atctattata tcacaggtga gaccaaggac 1500
caggtagcta actcagcctt tgtggaacgt cttcggaaac atggcttaga agtgatctat 1560
atgattgagc ccattgatga gtactgtgtc caacagctga aggaatttga ggggaagact 1620
ttagtgtcag tcaccaaaga aggcctggaa cttccagagg atgaagaaga gaaaaagaag 1680
caggaagaga aaaaaacaaa gtttgagaac ctctgcaaaa tcatgaaaga catattggag 1740
aaaaaagttg aaaaggtggt tgtgtcaaac cgattggtga catctccatg ctgtattgtc 1800
acaagcacat atggctggac agcaaacatg gagagaatca tgaaagctca agccctaaga 1860
gacaactcaa caatgggtta catggcagca aagaaacacc tggagataaa ccctgaccat 1920
tccattattg agaccttaag gcaaaaggca gaggctgata agaacgacaa gtctgtgaag 1980
gatctggtca tcttgcttta tgaaactgcg ctcctgtctt ctggcttcag tctggaagat 2040
ccccagacac atgctaacag gatctacagg atgatcaaac ttggtctggg tattgatgaa 2100

gatgacccta ctgctgatga taccagtgct gctgtaactg aagaaatgcc accccttgaa 2160
ggagatgacg acacatcacg catggaagaa gtagactaa 2199

<210> 7
<211> 724
<212> PRT
<213> ÖÇÈË(Homo sapiens)

<400> 7
Met Pro Glu Glu Val His His Gly Glu Glu Glu Val Glu Thr Phe Ala
1               5                   10                  15
Phe Gln Ala Glu Ile Ala Gln Leu Met Ser Leu Ile Ile Asn Thr Phe
            20                  25                  30
Tyr Ser Asn Lys Glu Ile Phe Leu Arg Glu Leu Ile Ser Asn Ala Ser
        35                  40                  45
Asp Ala Leu Asp Lys Ile Arg Tyr Glu Ser Leu Thr Asp Pro Ser Lys
    50                  55                  60
Leu Asp Ser Gly Lys Glu Leu Lys Ile Asp Ile Ile Pro Asn Pro Gln
65                  70                  75                  80
Glu Arg Thr Leu Thr Leu Val Asp Thr Gly Ile Gly Met Thr Lys Ala
                85                  90                  95
Asp Leu Ile Asn Asn Leu Gly Thr Ile Ala Lys Ser Gly Thr Lys Ala
            100                 105                 110
Phe Met Glu Ala Leu Gln Ala Gly Ala Asp Ile Ser Met Ile Gly Gln
        115                 120                 125
Phe Gly Val Gly Phe Tyr Ser Ala Tyr Leu Val Ala Glu Lys Val Val
    130                 135                 140
Val Ile Thr Lys His Asn Asp Asp Glu Gln Tyr Ala Trp Glu Ser Ser
145                 150                 155                 160
Ala Gly Gly Ser Phe Thr Val Arg Ala Asp His Gly Glu Pro Ile Gly
                165                 170                 175
Arg Gly Thr Lys Val Ile Leu His Leu Lys Glu Asp Gln Thr Glu Tyr
            180                 185                 190
Leu Glu Glu Arg Arg Val Lys Glu Val Val Lys Lys His Ser Gln Phe
        195                 200                 205
Ile Gly Tyr Pro Ile Thr Leu Tyr Leu Glu Lys Glu Arg Glu Lys Glu
    210                 215                 220
Ile Ser Asp Asp Glu Ala Glu Glu Glu Lys Gly Glu Lys Glu Glu Glu
225                 230                 235                 240
Asp Lys Asp Asp Glu Glu Lys Pro Lys Ile Glu Asp Val Gly Ser Asp
                245                 250                 255
Glu Glu Asp Asp Ser Gly Lys Asp Lys Lys Lys Lys Thr Lys Lys Ile
            260                 265                 270
Lys Glu Lys Tyr Ile Asp Gln Glu Glu Leu Asn Lys Thr Lys Pro Ile
        275                 280                 285
Trp Thr Arg Asn Pro Asp Asp Ile Thr Gln Glu Glu Tyr Gly Glu Phe
    290                 295                 300
Tyr Lys Ser Leu Thr Asn Asp Trp Glu Asp His Leu Ala Val Lys His
305                 310                 315                 320
Phe Ser Val Glu Gly Gln Leu Glu Phe Arg Ala Leu Leu Phe Ile Pro
                325                 330                 335
Arg Arg Ala Pro Phe Asp Leu Phe Glu Asn Lys Lys Lys Lys Asn Asn
            340                 345                 350
Ile Lys Leu Tyr Val Arg Arg Val Phe Ile Met Asp Ser Cys Asp Glu
        355                 360                 365
Leu Ile Pro Glu Tyr Leu Asn Phe Ile Arg Gly Val Val Asp Ser Glu
    370                 375                 380
Asp Leu Pro Leu Asn Ile Ser Arg Glu Met Leu Gln Gln Ser Lys Ile
385                 390                 395                 400
Leu Lys Val Ile Arg Lys Asn Ile Val Lys Lys Cys Leu Glu Leu Phe
                405                 410                 415
Ser Glu Leu Ala Glu Asp Lys Glu Asn Tyr Lys Lys Phe Tyr Glu Ala
            420                 425                 430

Phe Ser Lys Asn Leu Lys Leu Gly Ile His Glu Asp Ser Thr Asn Arg
        435                 440                 445
Arg Arg Leu Ser Glu Leu Leu Arg Tyr His Thr Ser Gln Ser Gly Asp
    450                 455                 460
Glu Met Thr Ser Leu Ser Glu Tyr Val Ser Arg Met Lys Glu Thr Gln
465                 470                 475                 480
Lys Ser Ile Tyr Tyr Ile Thr Gly Glu Ser Lys Glu Gln Val Ala Asn
                485                 490                 495
Ser Ala Phe Val Glu Arg Val Arg Lys Arg Gly Phe Glu Val Val Tyr
            500                 505                 510
Met Thr Glu Pro Ile Asp Glu Tyr Cys Val Gln Gln Leu Lys Glu Phe
        515                 520                 525
Asp Gly Lys Ser Leu Val Ser Val Thr Lys Glu Gly Leu Glu Leu Pro
    530                 535                 540
Glu Asp Glu Glu Glu Lys Lys Lys Met Glu Glu Ser Lys Ala Lys Phe
545                 550                 555                 560
Glu Asn Leu Cys Lys Leu Met Lys Glu Ile Leu Asp Lys Lys Val Glu
                565                 570                 575
Lys Val Thr Ile Ser Asn Arg Leu Val Ser Ser Pro Cys Cys Ile Val
            580                 585                 590
Thr Ser Thr Tyr Gly Trp Thr Ala Asn Met Glu Arg Ile Met Lys Ala
        595                 600                 605
Gln Ala Leu Arg Asp Asn Ser Thr Met Gly Tyr Met Met Ala Lys Lys
    610                 615                 620
His Leu Glu Ile Asn Pro Asp His Pro Ile Val Glu Thr Leu Arg Gln
625                 630                 635                 640
Lys Ala Glu Ala Asp Lys Asn Asp Lys Ala Val Lys Asp Leu Val Val
                645                 650                 655
Leu Leu Phe Glu Thr Ala Leu Leu Ser Ser Gly Phe Ser Leu Glu Asp
            660                 665                 670
Pro Gln Thr His Ser Asn Arg Ile Tyr Arg Met Ile Lys Leu Gly Leu
        675                 680                 685
Gly Ile Asp Glu Asp Glu Val Ala Ala Glu Glu Pro Asn Ala Ala Val
    690                 695                 700
Pro Asp Glu Ile Pro Pro Leu Glu Gly Asp Glu Asp Ala Ser Arg Met
705                 710                 715                 720
Glu Glu Val Asp


<210>  8
<211>  2175
<212>  DNA
<213>  ÖÇÈË(Homo sapiens)

<400>  8
atgcctgagg aagtgcacca tggagaggag gaggtggaga cttttgcctt tcaggcagaa      60
attgcccaac tcatgtccct catcatcaat accttctatt ccaacaagga gattttcctt     120
cgggagttga tctctaatgc ttctgatgcc ttggacaaga ttcgctatga gagcctgaca     180
gacccttcga agttggacag tggtaaagag ctgaaaattg acatcatccc caaccctcag     240
gaacgtaccc tgactttggt agacacaggc attggcatga ccaaagctga tctcataaat     300
aatttgggaa ccattgccaa gtctggtact aaagcattca tggaggctct tcaggctggt     360
gcagacatct ccatgattgg gcagtttggt gttggctttt attctgccta cttggtggca     420
gagaaagtgg ttgtgatcac aaagcacaac gatgatgaac agtatgcttg ggagtcttct     480
gctggaggtt ccttcactgt gcgtgctgac catggtgagc ccattggcag gggtaccaaa     540
gtgatcctcc atcttaaaga agatcagaca gagtacctag aagagaggcg ggtcaaagaa     600
gtagtgaaga agcattctca gttcataggc tatcccatca ccctttattt ggagaaggaa     660
cgagagaagg aaattagtga tgatgaggca gaggaagaga aaggtgagaa agaagaggaa     720
gataaagatg atgaagaaaa acccaagatc gaagatgtgg gttcagatga ggaggatgac     780
agcggtaagg ataagaagaa gaaaactaag aagatcaaag agaaatacat tgatcaggaa     840
gaactaaaca agaccaagcc tatttggacc agaaaccctg atgacatcac ccaagaggag     900
tatggagaat tctacaagag cctcactaat gactgggaag accacttggc agtcaagcac     960
ttttctgtag aaggtcagtt ggaattcagg gcattgctat ttattcctcg tcggctcccc    1020
tttgaccttt ttgagaacaa gaagaaaaag aacaacatca aactctatgt ccgccgtgtg    1080

ttcatcatgg acagctgtga tgagttgata ccagagtatc tcaattttat ccgtggtgtg 1140
gttgactctg aggatctgcc cctgaacatc tcccgagaaa tgctccagca gagcaaaatc 1200
ttgaaagtca ttcgcaaaaa cattgttaag aagtgccttg agctcttctc tgagctggca 1260
gaagacaagg agaattacaa gaaattctat gaggcattct ctaaaaatct caagcttgga 1320
atccacgaag actccactaa ccgccgccgc ctgtctgagc tgctgcgcta tcatacctcc 1380
cagtctggag atgagatgac atctctgtca gagtatgttt ctcgcatgaa ggagacacag 1440
aagtccatct attacatcac tggtgagagc aaagagcagg tggccaactc agcttttgtg 1500
gagcgagtgc ggaaacgggg cttcgaggtg gtatatatga ccgagcccat tgacgagtac 1560
tgtgtgcagc agctcaagga atttgatggg aagagcctgg tctcagttac caaggagggt 1620
ctggagctgc ctgaggatga ggaggagaag aagaagatgg aagagagcaa ggcaaagttt 1680
gagaacctct gcaagctcat gaaagaaatc ttagataaga aggttgagaa ggtgacaatc 1740
tccaatagac ttgtgtcttc accttgctgc attgtgacca gcacctacgg ctggacagcc 1800
aatatggagc ggatcatgaa agcccaggca cttcgggaca actccaccat gggctatatg 1860
atggccaaaa agcacctgga gatcaaccct gaccacccca ttgtggagac gctgcggcag 1920
aaggctgagg ccgacaagaa tgataaggca gttaaggacc tggtggtgct gctgtttgaa 1980
accgccctgc tatcttctgg cttttccctt gaggatcccc agacccactc caaccgcatc 2040
tatcgcatga tcaagctagg tctaggtatt gatgaagatg aagtggcagc agaggaaccc 2100
aatgctgcag ttcctgatga gatcccccct ctcgagggcg atgaggatgc gtctcgcatg 2160
gaagaagtcg attag 2175

<210> 9
<211> 642
<212> PRT
<213> Êó£¨Mus musculus£©

<400> 9
Met Ala Lys Asn Thr Ala Ile Gly Ile Asp Leu Gly Thr Thr Tyr Ser
1               5                   10                  15
Cys Val Gly Val Phe Gln His Gly Lys Val Glu Ile Ile Ala Asn Asp
            20                  25                  30
Gln Gly Asn Arg Thr Thr Pro Ser Tyr Val Ala Phe Thr Asp Thr Glu
        35                  40                  45
Arg Leu Ile Gly Asp Ala Ala Lys Asn Gln Val Ala Leu Asn Pro Gln
    50                  55                  60
Asn Thr Val Phe Asp Ala Lys Arg Leu Ile Gly Arg Lys Phe Gly Asp
65                  70                  75                  80
Ala Val Val Gln Ser Asp Met Lys His Trp Pro Phe Gln Val Val Asn
                85                  90                  95
Asp Gly Asp Lys Pro Lys Val Gln Val Asn Tyr Lys Gly Glu Ser Arg
            100                 105                 110
Ser Phe Phe Pro Glu Glu Ile Ser Ser Met Val Leu Thr Lys Met Lys
        115                 120                 125
Glu Ile Ala Glu Ala Tyr Leu Gly His Pro Val Thr Asn Ala Val Ile
    130                 135                 140
Thr Val Pro Ala Tyr Phe Asn Asp Ser Gln Arg Gln Ala Thr Lys Asp
145                 150                 155                 160
Ala Gly Val Ile Ala Gly Leu Asn Val Leu Arg Ile Ile Asn Glu Pro
                165                 170                 175
Thr Ala Ala Ala Ile Ala Tyr Gly Leu Asp Arg Thr Gly Lys Gly Glu
            180                 185                 190
Arg Asn Val Leu Ile Phe Asp Leu Gly Gly Gly Thr Phe Asp Val Ser
        195                 200                 205
Ile Leu Thr Ile Asp Asp Gly Ile Phe Glu Val Lys Ala Thr Ala Gly
    210                 215                 220
Asp Thr His Leu Gly Gly Glu Asp Phe Asp Asn Arg Leu Val Ser His
225                 230                 235                 240
Phe Val Glu Glu Phe Lys Arg Lys His Lys Lys Asp Ile Ser Gln Asn
                245                 250                 255
Lys Arg Ala Val Arg Arg Leu Arg Thr Ala Cys Glu Arg Ala Lys Arg
            260                 265                 270
Thr Leu Ser Ser Ser Thr Gln Ala Ser Leu Glu Ile Asp Ser Leu Phe
        275                 280                 285
Glu Gly Ile Asp Phe Tyr Thr Ser Ile Thr Arg Ala Arg Phe Glu Glu

290                 295                 300
Leu Cys Ser Asp Leu Phe Arg Gly Thr Leu Glu Pro Val Glu Lys Ala
305                 310                 315                 320
Leu Arg Asp Ala Lys Met Asp Lys Ala Gln Ile His Asp Leu Val Leu
                325                 330                 335
Val Gly Gly Ser Thr Arg Ile Pro Lys Val Gln Lys Leu Leu Gln Asp
            340                 345                 350
Phe Phe Asn Gly Arg Asp Leu Asn Lys Ser Ile Asn Pro Asp Glu Ala
        355                 360                 365
Val Ala Tyr Gly Ala Ala Val Gln Ala Ala Ile Leu Met Gly Asp Lys
    370                 375                 380
Ser Glu Asn Val Gln Asp Leu Leu Leu Leu Asp Val Ala Pro Leu Ser
385                 390                 395                 400
Leu Gly Leu Glu Thr Ala Gly Gly Val Met Thr Ala Leu Ile Lys Arg
                405                 410                 415
Asn Ser Thr Ile Pro Thr Lys Gln Thr Gln Thr Phe Thr Thr Tyr Ser
            420                 425                 430
Asp Asn Gln Pro Gly Val Leu Ile Gln Val Tyr Glu Gly Glu Arg Ala
        435                 440                 445
Met Thr Arg Asp Asn Asn Leu Leu Gly Arg Phe Glu Leu Ser Gly Ile
    450                 455                 460
Pro Pro Ala Pro Arg Gly Val Pro Gln Ile Glu Val Thr Phe Asp Ile
465                 470                 475                 480
Asp Ala Asn Gly Ile Leu Asn Val Thr Ala Thr Asp Lys Ser Thr Gly
                485                 490                 495
Lys Ala Asn Lys Ile Thr Ile Thr Asn Asp Lys Gly Arg Leu Ser Lys
            500                 505                 510
Glu Glu Ile Glu Arg Met Val Gln Glu Ala Glu Arg Tyr Lys Ala Glu
        515                 520                 525
Asp Glu Val Gln Arg Asp Arg Val Ala Ala Lys Asn Ala Leu Glu Ser
    530                 535                 540
Tyr Ala Phe Asn Met Lys Ser Ala Val Glu Asp Glu Gly Leu Lys Gly
545                 550                 555                 560
Lys Leu Ser Glu Ala Asp Lys Lys Lys Val Leu Asp Lys Cys Gln Glu
                565                 570                 575
Val Ile Ser Trp Leu Asp Ser Asn Thr Leu Ala Asp Lys Glu Glu Phe
            580                 585                 590
Val His Lys Arg Glu Glu Leu Glu Arg Val Cys Ser Pro Ile Ile Ser
        595                 600                 605
Gly Leu Tyr Gln Gly Ala Gly Ala Pro Gly Ala Gly Gly Phe Gly Ala
    610                 615                 620
Gln Ala Pro Pro Lys Gly Ala Ser Gly Ser Gly Pro Thr Ile Glu Glu
625                 630                 635                 640
Val Asp


<210>  10
<211>  1929
<212>  DNA
<213>  Êó£¨Mus musculus£©

<400>  10
atggccaaga acacggcgat cggcatcgac ctgggcacca cctactcgtg cgtgggcgtg      60
ttccagcacg gcaaggtgga gatcatcgcc aacgaccagg gcaaccgcac gacccccagc     120
tacgtggcct tcaccgacac cgagcgcctc atcggggacg ccgccaagaa ccaggtggcg     180
ctgaacccgc agaacaccgt gttcgacgcg aagcggctga tcggccgcaa gttcggcgat     240
gcggtggtgc agtccgacat gaagcactgg cccttccagg tggtgaacga cggcgacaag     300
cccaaggtgc aggtgaacta caagggcgag agccggtcgt tcttcccgga ggagatctcg     360
tccatggtgc tgacgaagat gaaggagatc gctgaggcgt acctgggcca cccggtgacc     420
aacgcggtga tcacggtgcc cgcctacttc aacgactctc agcggcaggc caccaaggac     480
gcgggcgtga tcgccggtct aaacgtgctg cggatcatca acgagcccac ggcggccgcc     540
atcgcctacg ggctggaccg gaccggcaag ggcgagcgca acgtgctcat cttcgacctg     600
gggggcggca cgttcgacgt gtccatcctg acgatcgacg acggcatctt cgaggtgaag     660

gccacggcgg gcgacacgca cctgggaggg gaggacttcg acaaccggct ggtgagccac 720
ttcgtggagg agttcaagag gaagcacaag aaggacatca gccagaacaa gcgcgcggtg 780
cggcggctgc gcacggcgtg tgagagggcc aagaggacgc tgtcgtccag cacccaggcc 840
agcctggaga tcgactctct gttcgagggc atcgacttct acacatccat cacgcgggcg 900
cggttcgaag agctgtgctc ggacctgttc cgcggcacgc tggagcccgt ggagaaggcc 960
ctgcgcgacg ccaagatgga caaggcgcag atccacgacc tggtgctggt gggcggctcg 1020
acgcgcatcc ccaaggtgca gaagctgctg caggacttct tcaacggcgc cgacctgaac 1080
aagagcatca acccggacga ggcggtggcc tacggggcgg cggtgcaggc ggccatcctg 1140
atgggggaca agtcggagaa cgtgcaggac ctgctgctgc tggacgtggc gccgctgtcg 1200
ctgggcctgg agactgcggg cggcgtgatg acggcgctca tcaagcgcaa ctccaccatc 1260
cccaccaagc agacgcagac cttcaccacc tactcggaca accagcccgg ggtgctgatc 1320
caggtgtacg agggcgagag ggccatgacg cgcgacaaca acctgctggg gcgcttcgag 1380
ctgagcggca tcccgccggc gcccaggggc gtgccgcaga tcgaggtgac cttcgacatc 1440
gacgccaacg gcatcctgaa cgtcacggcc accgacaaga gcaccggcaa ggccaacaag 1500
atcaccatca ccaacgacaa gggccgcctg agcaaggagg agatcgagcg catggtgcag 1560
gaggccgagc gctacaaggc cgaggacgag gtgcagcgcg acagggtggc cgccaagaac 1620
gcgctcgagt cctatgcctt caacatgaag agcgccgtgg aggacgaggg tctcaagggc 1680
aagctcagcg aggctgacaa gaagaaggtg ctggacaagt gccaggaggt catctcctgg 1740
ctggactcca acacgctggc cgacaaggag gagttcgtgc acaagcggga ggagctggag 1800
cgggtgtgca gccccatcat cagtggcctg taccagggtg cgggtgctcc tggggctggg 1860
ggcttcgggg cccaggcgcc gccgaaagga gcctctggct caggacccac catcgaggag 1920
gtggattag 1929

<210> 11
<211> 573
<212> PRT
<213> Êó£¨Mus musculus£©

<400> 11
Met Leu Arg Leu Pro Thr Val Leu Arg Gln Met Arg Pro Val Ser Arg
1               5                   10                  15
Ala Leu Ala Pro His Leu Thr Arg Ala Tyr Ala Lys Asp Val Lys Phe
            20                  25                  30
Gly Ala Asp Ala Arg Ala Leu Met Leu Gln Gly Val Asp Leu Leu Ala
        35                  40                  45
Asp Ala Val Ala Val Thr Met Gly Pro Lys Gly Arg Thr Val Ile Ile
    50                  55                  60
Glu Gln Ser Trp Gly Ser Pro Lys Val Thr Lys Asp Gly Val Thr Val
65                  70                  75                  80
Ala Lys Ser Ile Asp Leu Lys Asp Lys Tyr Lys Asn Ile Gly Ala Lys
                85                  90                  95
Leu Val Gln Asp Val Ala Asn Asn Thr Asn Glu Glu Ala Gly Asp Gly
            100                 105                 110
Thr Thr Thr Ala Thr Val Leu Ala Arg Ser Ile Ala Lys Glu Gly Phe
        115                 120                 125
Glu Lys Ile Ser Lys Gly Ala Asn Pro Val Glu Ile Arg Arg Gly Val
    130                 135                 140
Met Leu Ala Val Asp Ala Val Ile Ala Glu Leu Lys Lys Gln Ser Lys
145                 150                 155                 160
Pro Val Thr Thr Pro Glu Glu Ile Ala Gln Val Ala Thr Ile Ser Ala
                165                 170                 175
Asn Gly Asp Lys Asp Ile Gly Asn Ile Ile Ser Asp Ala Met Lys Lys
            180                 185                 190
Val Gly Arg Lys Gly Val Ile Thr Val Lys Asp Gly Lys Thr Leu Asn
        195                 200                 205
Asp Glu Leu Glu Ile Ile Glu Gly Met Lys Phe Asp Arg Gly Tyr Ile
    210                 215                 220
Ser Pro Tyr Phe Ile Asn Thr Ser Lys Gly Gln Lys Cys Glu Phe Gln
225                 230                 235                 240
Asp Ala Tyr Val Leu Leu Ser Glu Lys Lys Ile Ser Ser Val Gln Ser
                245                 250                 255
Ile Val Pro Ala Leu Glu Ile Ala Asn Ala His Arg Lys Pro Leu Val
            260                 265                 270

Ile Ile Ala Glu Asp Val Asp Gly Glu Ala Leu Ser Thr Leu Val Leu
        275                 280                 285
Asn Arg Leu Lys Val Gly Leu Gln Val Val Ala Val Lys Ala Pro Gly
    290                 295                 300
Phe Gly Asp Asn Arg Lys Asn Gln Leu Lys Asp Met Ala Ile Ala Thr
305                 310                 315                 320
Gly Gly Ala Val Phe Gly Glu Glu Gly Leu Asn Leu Asn Leu Glu Asp
                325                 330                 335
Val Gln Ala His Asp Leu Gly Lys Val Gly Glu Val Ile Val Thr Lys
            340                 345                 350
Asp Asp Ala Met Leu Leu Lys Gly Lys Gly Asp Lys Ala His Ile Glu
        355                 360                 365
Lys Arg Ile Gln Glu Ile Thr Glu Gln Leu Asp Ile Thr Thr Ser Glu
    370                 375                 380
Tyr Glu Lys Glu Lys Leu Asn Glu Arg Leu Ala Lys Leu Ser Asp Gly
385                 390                 395                 400
Val Ala Val Leu Lys Val Gly Gly Thr Ser Asp Val Glu Val Asn Glu
                405                 410                 415
Lys Lys Asp Arg Val Thr Asp Ala Leu Asn Ala Thr Arg Ala Ala Val
            420                 425                 430
Glu Glu Gly Ile Val Leu Gly Gly Gly Cys Ala Leu Leu Arg Cys Ile
        435                 440                 445
Pro Ala Leu Asp Ser Leu Lys Pro Ala Asn Glu Asp Gln Lys Ile Gly
    450                 455                 460
Ile Glu Ile Ile Lys Arg Ala Leu Lys Ile Pro Ala Met Thr Ile Ala
465                 470                 475                 480
Lys Asn Ala Gly Val Glu Gly Ser Leu Ile Val Glu Lys Ile Leu Gln
                485                 490                 495
Ser Ser Ser Glu Val Gly Tyr Asp Ala Met Leu Gly Asp Phe Val Asn
            500                 505                 510
Met Val Glu Lys Gly Ile Ile Asp Pro Thr Lys Val Val Arg Thr Ala
        515                 520                 525
Leu Leu Asp Ala Ala Gly Val Ala Ser Leu Leu Thr Thr Ala Glu Ala
    530                 535                 540
Val Val Thr Glu Ile Pro Lys Glu Glu Lys Asp Pro Gly Met Gly Ala
545                 550                 555                 560
Met Gly Gly Met Gly Gly Gly Met Gly Gly Gly Met Phe
                565                 570

<210>  12
<211>  1722
<212>  DNA
<213>  Êó£¨Mus musculus£©

<400>  12
atgcttcgac tacccacagt ccttcgccag atgagaccag tgtcccgggc actggctcct      60
catctcactc gggcctatgc caaagatgta aaatttggtg cggacgctcg agccttaatg     120
cttcaaggtg tagacctttt agcagatgct gtagctgtta caatggggcc aaagggaaga     180
actgtgatta ttgaacagag ttggggaagt cccaaagtaa caaaagatgg ggtcactgtt     240
gcaaagtcaa ttgatttaaa ggataaatac aaaaatattg gagctaaact tgttcaggac     300
gttgccaata acacaaacga agaggctggg gatggcacca ccactgccac tgttctggca     360
cgatctattg ccaaggaggg ctttgagaag atcagcaaag gggctaatcc agtggaaatc     420
cggagaggtg tgatgttggc tgtggatgct gtaattgctg aacttaagaa acagtctaaa     480
cctgtgacaa cccctgaaga aattgctcag gttgctacaa tttctgcaaa tggagacaaa     540
gacattggga acatcatttc tgatgcaatg aaaaaggttg gaagaaaggg tgtcatcaca     600
gtgaaggatg gaaaaaccct gaatgatgag ctagaaatta ttgaaggcat gaagtttgat     660
agaggatata tttccccgta ttttattaac acatcaaaag gtcaaaagtg tgaattccaa     720
gatgcctatg tcttgttgag tgaaaagaaa atttccagtg ttcagtccat tgtccctgct     780
cttgaaattg ctaatgctca tcggaagcca ttggtcataa tcgccgaaga cgttgacgga     840
gaagctctaa gcacgctggt tttgaacagg ctaaaagttg gtcttcaggt tgtggcagtc     900
aaagctccag gatttgggga caataggaag aaccagctta aagatatggc tattgctact     960
ggtggtgcag tgtttggaga agaggggttg aatctaaatc ttgaagatgt tcaagctcat    1020
gacttaggaa aagttgggga ggtcattgtc accaaagatg atgccatgct tttgaaagga    1080

aaaggtgaca aagctcacat tgaaaaacgt attcaagaaa tcactgagca gctagacatc 1140
acaactagtg aatatgaaaa agaaaagctg aacgagcgac ttgctaaact ttcagatgga 1200
gtagctgtgt tgaaggttgg aggaacaagt gatgttgaag tgaatgagaa aaaagacaga 1260
gttactgatg ctctcaatgc tacaagagca gctgttgaag aaggcattgt tctaggaggg 1320
ggctgcgctc tgcttcggtg catcccagcc ttggattcat taaagcctgc taatgaagac 1380
cagaaaatag gtatagaaat tattaaaaga gcacttaaaa ttcctgcaat gacgattgct 1440
aagaatgcag gtgttgaagg atctttgata gttgagaaaa ttctgcagag ttcctcagaa 1500
gttggttatg acgccatgct tggagatttt gtgaacatgg tggaaaaagg gatcattgat 1560
ccaacaaagg ttgtgagaac tgccttactg gatgctgctg gggtggcctc cttgctaact 1620
acagccgaag ctgtagtgac agaaattcct aaagaagaga aggaccctgg aatgggtgca 1680
atgggtggca tgggaggggg tatgggaggc ggcatgttct aa 1722

<210> 13
<211> 340
<212> PRT
<213> Êó£¨Mus musculus£©

<400> 13
Met Gly Lys Asp Tyr Tyr Gln Thr Leu Gly Leu Ala Arg Gly Ala Ser
1               5                   10                  15
Asp Asp Glu Ile Lys Arg Ala Tyr Arg Arg Gln Ala Leu Arg Tyr His
            20                  25                  30
Pro Asp Lys Asn Lys Glu Pro Gly Ala Glu Glu Lys Phe Lys Glu Ile
        35                  40                  45
Ala Glu Ala Tyr Asp Val Leu Ser Asp Pro Arg Lys Arg Glu Ile Phe
    50                  55                  60
Asp Arg Tyr Gly Glu Glu Gly Leu Lys Gly Gly Ser Pro Ser Gly Gly
65                  70                  75                  80
Ser Ser Gly Gly Ala Asn Gly Thr Ser Phe Ser Tyr Thr Phe His Gly
                85                  90                  95
Asp Pro His Ala Met Phe Ala Glu Phe Phe Gly Gly Arg Asn Pro Phe
            100                 105                 110
Asp Thr Phe Phe Gly Gln Arg Asn Gly Glu Glu Gly Met Asp Ile Asp
        115                 120                 125
Asp Thr Phe Ser Ser Phe Pro Met Gly Met Gly Gly Phe Thr Asn Met
    130                 135                 140
Asn Phe Gly Arg Ser Arg Pro Ser Gln Glu Pro Thr Arg Lys Lys Gln
145                 150                 155                 160
Asp Pro Pro Val Thr His Asp Leu Arg Val Ser Leu Glu Glu Ile Tyr
                165                 170                 175
Ser Gly Cys Thr Lys Lys Met Lys Ile Ser His Lys Arg Leu Asn Pro
            180                 185                 190
Asp Gly Lys Ser Ile Arg Asn Glu Asp Lys Ile Leu Thr Ile Glu Val
        195                 200                 205
Lys Arg Gly Trp Lys Glu Gly Thr Lys Ile Thr Phe Pro Lys Glu Gly
    210                 215                 220
Asp Gln Thr Ser Asn Asn Ile Pro Ala Asp Ile Val Phe Val Leu Lys
225                 230                 235                 240
Asp Lys Pro His Asn Ile Phe Lys Arg Asp Gly Ser Asp Val Ile Tyr
                245                 250                 255
Pro Ala Arg Ile Ser Leu Arg Glu Ala Leu Cys Gly Cys Thr Val Asn
            260                 265                 270
Val Pro Thr Leu Asp Gly Arg Thr Ile Pro Val Val Phe Lys Asp Val
        275                 280                 285
Ile Arg Pro Gly Met Arg Arg Lys Val Pro Gly Glu Gly Leu Pro Leu
    290                 295                 300
Pro Lys Thr Pro Glu Lys Arg Gly Asp Leu Val Ile Glu Phe Glu Val
305                 310                 315                 320
Ile Phe Pro Glu Arg Ile Pro Val Ser Ser Arg Thr Ile Leu Glu Gln
                325                 330                 335
Val Leu Pro Ile
            340

<210> 14
<211> 1023
<212> DNA
<213> Êó£¨Mus musculus£©

<400> 14
atgggcaagg actactatca gacgctgggc ctggcccgcg gcgcgtccga cgacgagatc 60
aagcgagcct accgccgcca ggcgctgcgc taccacccgg acaagaacaa ggagcccggc 120
gctgaggaga agttcaagga gatcgccgag gcctacgacg tgctcagcga cccgcgcaag 180
cgcgagattt tcgaccgcta tggagaggaa ggcctgaagg gtggtagccc cagtggagga 240
agcagtggtg gtgctaatgg tacctctttc agctacacat tccacggaga cccccatgcc 300
atgtttgctg agttcttcgg tggcagaaac ccctttgata ccttttttgg gcagcgcaac 360
ggggaagaag gcatggacat tgatgacaca ttctctagct ttccaatggg tatgggtggc 420
ttcaccaaca tgaactttgg acgttcccgc ccttctcaag agcccacccg gaagaagcaa 480
gatcctccag tcacccatga ccttcgggtc tcccttgaag agatctacag cggctgtacc 540
aagaagatga aaatctccca caagcggctg aaccctgatg gaaagagcat tcgaaatgaa 600
gataagatcc tgaccatcga agtgaagagg ggctggaaag aagggaccaa aatcaccttt 660
cccaaggaag gggaccagac ctcgaacaac attccagcag acatcgtctt tgttttaaag 720
gacaagccac acaatatctt caagagagat ggttctgatg tcatctatcc agccaggatt 780
agccttcggg aggctctctg tggttgcact gtgaatgtcc ctactctgga cggcaggacc 840
atccctgttg tattcaaaga tgtcatcagg cctggtatgc ggcggaaagt ccctggagaa 900
ggcctccctc tccccaaaac acctgagaaa cgtggagacc ttgttatcga gtttgaagtg 960
atcttcccgg aaaggattcc cgtctcatcc agaaccatcc tggagcaggt tcttcccata 1020
tag 1023

<210> 15
<211> 180
<212> DNA
<213> ÈË¹¤ÐòÁÐ(Artificial Sequence)

<220>
<223> Exon28°ÐÐòÁÐ

<400> 15
gctggattct ttaaaagaca gtacaaatct atcctacaag aagaaaacag gagagacagc 60
tggagttatg tcaacagcaa aagcaatgat gactgaagac ttctacactg agagaactga 120
aaaactcagg ttaggaaaaa gaaatcctgt tcagaagacc cgtcagaatt tcttcttttt 180

<210> 16
<211> 23
<212> DNA
<213> ÈË¹¤ÐòÁÐ(Artificial Sequence)

<220>
<223> GuideRNA

<400> 16
ctatcctaca agaagaaaac agg 23

<210> 17
<211> 119
<212> DNA
<213> ÈË¹¤ÐòÁÐ(Artificial Sequence)

<220>
<223> Donor DNAÐòÁÐ

<400> 17
tttcaggctg gattctttaa aagacagtac aaatctatcc tacaagaaga aaacgcgaga 60
gacagctgga gttatgtcaa cagcaaaagc aatgatgact gaagacttct acactgaga 119

<210> 18
<211> 32

<212>  PRT
<213>  ÈË¹¤ÐòÁÐ(Artificial Sequence)

<220>
<223>  ¦Á4Õû°ÏËØ°ûÄÚ¶Î

<400>  18
Lys Ala Gly Phe Phe Lys Arg Gln Tyr Lys Ser Ile Leu Gln Glu Glu
1               5                   10                  15
Asn Arg Arg Asp Ser Trp Ser Tyr Val Asn Ser Lys Ser Asn Asp Asp
            20                  25                  30

<210>  19
<211>  24
<212>  PRT
<213>  ÈË¹¤ÐòÁÐ(Artificial Sequence)

<220>
<223>  ½Ø¶ÌµÄ¦Á4Õû°ÏËØ°ûÄÚ¶Î

<400>  19
Lys Ala Gly Phe Phe Lys Arg Gln Tyr Lys Ser Ile Leu Gln Glu Glu
1               5                   10                  15
Asn Arg Arg Asp Ser Trp Ser Tyr
            20

<210>  20
<211>  15
<212>  PRT
<213>  ÈË¹¤ÐòÁÐ(Artificial Sequence)

<220>
<223>  ½Ø¶ÌµÄ¦Á4Õû°ÏËØ°ûÄÚ¶Î

<400>  20
Lys Ala Gly Phe Phe Lys Arg Gln Tyr Lys Ser Ile Leu Gln Glu
1               5                   10                  15

<210>  21
<211>  7
<212>  PRT
<213>  ÈË¹¤ÐòÁÐ(Artificial Sequence)

<220>
<223>  ½Ø¶ÌµÄ¦Á4Õû°ÏËØ°ûÄÚ¶Î

<400>  21
Lys Ala Gly Phe Phe Lys Arg
1               5

## Claims

1. A method of regulating the migration of immune cells, comprising the step of enhancing or weakening the interaction between Hsp90 and α4 integrin in the immune cells.

2. The method according to claim 1, wherein,
the method includes enhancing the interaction between Hsp90 and α4 integrin in immune cells by any one or more of the following means: (1) overexpressing Hsp90 protein and/or Hsp90 mutant in the immune cells, wherein, compared with the wild-type Hsp90 protein, the Hsp90 mutant is only mutated in its middle domain, or the Hsp90 mutant has a mutation causing its inability to self-dimerize; (2) overexpressing α4 integrin in the immune cells; or
the method includes weakening the interaction between Hsp90 and α4 integrin in immune cells by any one or more of the following means: (1) knocking out Hsp90 protein or knocking down its expression in immune cells; (2) knocking out α4 integrin or knocking down its expression in immune cells; (3) expressing a Hsp90 mutant that has weakened or no interaction with α4 integrin in wild-type immune cells or immune cells with Hsp90 protein knocked out; (4) expressing a α4 integrin mutant that has weakened or no interaction with Hsp90 protein in wild-type immune cells or immune cells with α4 integrin knocked out.

3. The method according to claim 2, wherein,
treating immune cells with an expression vector and/or an integration vector of Hsp90 protein and/or Hsp90 mutant, and/or treating immune cells with an expression vector and/or an integration vector of α4 integrin, and/or treating immune cells with reagents that increase the transcription levels of Hsp90 and/or α4 integrin naturally occurred in immune cells, and/or placing immune cells at a fever-range hyperthermia, thereby enhancing the interaction between Hsp90 and α4 integrin in immune cells; or
knocking out Hsp90 protein or α4 integrin from immune cells by transferring a gene-knockout vector into the immune cells, and/or knocking out Hsp90 protein or α4 integrin from immune cells by ZFN, TALEN or CRISPR/Cas9 and the like, and/or knocking down the expression of Hsp90 protein and/or α4 integrin by interfering-RNA-mediated gene silencing, and/or integrating into the genome of immune cells an expression cassette expressing the Hsp90 mutant that has weakened or no interaction with α4 integrin and/or an expression cassette expressing the α4 integrin mutant that has weakened or no interaction with Hsp90 protein by transferring a gene-insertion vector into immune cells while knocking out the coding sequence of wild-type Hsp90 and/or α4 integrin, thereby weakening or destroying the interaction between Hsp90 and α4 integrin in immune cells.

4. A genetically engineered immune cell, having an enhanced or weakened interaction between Hsp90 and α4 integrin as compared with a wild-type immune cell.

5. The genetically engineered immune cell according to claim 4, wherein,
the genetically engineered immune cell: (1) overexpresses Hsp90 protein and/or Hsp90 mutant, wherein, compared with wild-type Hsp90 protein, the Hsp90 mutant is only mutated in its middle domain, or the Hsp90 mutant has a mutation causing its inability to self-dimerize; (2) overexpresses α4 integrin; or
the genetically engineered immune cell: (1) does not express Hsp90 or has reduced expression level of Hsp90, or expresses Hsp90 with reduced activity, or expresses Hsp90 mutants that has weakened or no interaction with α4 integrin, as compared with wild-type immune cells; and/or (2) has reduced expression level of α4 integrin, or expresses α4 integrin mutants that has weakened or no interaction with Hsp90, as compared with wild-type immune cells.

6. A pharmaceutical composition comprising the genetically engineered immune cell according to claim 4 or 5.

7. A mutant Hsp90 protein selected from the group consisting of:
(1) a mutant Hsp90 protein, lacking the N-terminal domain and/or the C-terminal domain of the wild-type Hsp90 protein;
(2) a mutant Hsp90 protein, only having a mutation in the middle domain as compared with the wild-type Hsp90 protein while maintaining the ability to bind to α4 integrin as compared with the wild-type; and
(3) a mutant Hsp90 protein, having a mutation causing its inability to self-dimerize as compared with the wild-type Hsp90 protein; preferably, the mutation occurs in the C-terminal domain of the Hsp90 protein; more preferably, the mutation is the deletion of the last 49 amino acids of C-terminus.

8. A mutant α4 integrin, wherein the mutant α4 integrin has one or more amino acid residues mutated in its intracellular segment other than amino acid residues 968-974, causing its interaction with Hsp90 protein weakened or eliminated; preferably, there is a mutation at at least one of R985, W989 and Y991; preferably, the mutation is a substitution mutation; more preferably, the substituted amino acid residue is alanine.

9. A coding sequence of the mutant Hsp90 protein according to claim 7 and the mutant α4 integrin according to claim 8, or a complementary sequence thereof, a nucleic acid construct containing the coding sequence or complementary sequence, and a host cell containing the nucleic acid construct.

10. Use selected from the group consisting of:
(1) use of a reagent that enhances the interaction between Hsp90 and α4 integrin in immune cells in the manufacture of immune cells for immunotherapy; preferably, the reagent is selected from the group consisting of: an expression vector or an integration vector for the wild-type Hsp90 protein, or the mutant Hsp90 protein being only mutated in its middle domain, or the mutant Hsp90 protein having a mutation causing its inability to self-dimerize; and an expression vector and/or integration vector for α4 integrin;
(2) use of immune cells with enhanced interaction between Hsp90 protein and α4 integrin in the manufacture of a medicament for the treatment of pathogen infections or tumors;
(3) use of a reagent that weakens the interaction between Hsp90 and α4 integrin in immune cells in the manufacture of immune cells for the treatment of sepsis, hematological tumors, chronic inflammations or autoimmune diseases; preferably, the reagent is selected from the group consisting of: a Hsp90 mutant lacking the N-terminal and/or C-terminal domain or having a mutation in the N-terminal and/or C-terminal domain causing its interaction with α4 integrin weakened or eliminated as compared with the wide-type Hsp90, and/or a targeting vector of the Hsp90 mutant; a α4 integrin mutant having a mutation in its intracellular segment which causes its interaction with Hsp90 weakened or eliminated, and/or a targeting vector of the α4 integrin mutant; and ZFN, TALEN and/or CRISPR/Cas9 reagents and/or small interfering RNA used to knock out Hsp90 and/or α4 integrin or to knock down its/their expression; and
(4) use of immune cells with weakened interaction between Hsp90 and α4 integrin in the manufacture of a medicament for the treatment of sepsis, hematological tumors, chronic inflammations or autoimmune diseases.
